# EUROPEAN PATENT SPECIFICATION

(11) **EP 1 474 144 B1**
(45) Date of publication and mention of the grant of the patent: **06.08.2008**
(21) Application number: 03700414.0
(22) Date of filing: 20.01.2003
(51) Int. Cl.: A61K 31/4706, A61K 9/22

(54) **CONTROLLED RELEASE PHARMACEUTICAL DOSAGE FORMS OF A CHOLESTERYL ESTER TRANSFER PROTEIN INHIBITOR**
PHARMAZEUTISCHE DARREICHUNGSFORM MIT GESTEUERTER FREIGABE EINES CHOLESTERYLESTER-TRANSFERPROTEININHIBITORS
FORME POSOLOGIQUE A LIBERATION CONTROLEE D'UNE PROTEINE INHIBANT LE TRANSFERT DU CHOLESTERYL ESTER

(30) Priority: 01.02.2002 US 353719 P
(43) Date of publication of application: 10.11.2004
(73) Proprietor: Pfizer Products Inc., Groton, CT 06340 (US)
(72) Inventor: CURATOLO, William John Pfizer Global Rrs. & De., Groton, CT 06340 (US); SUTTON, Steven Charles Pfizer Global Rrs. & Dev., Groton, CT 06340 (US); APPEL, Leah Elizabeth Bend Research Inc,, Bend, OR 97701 (US)
(74) Representative: Vanhee-Brossollet, Christine
(86) International application number: PCT/IB2003/000209
(87) International publication number: WO 2003/063868

(56) References cited:
- EP-A- 1 027 887
- EP-A- 1 269 994
- WO-A-01/47500
- WO-A-02/11710

## Description

This application claims the benefit of priority of United States provisional patent application serial number 60/353,719 filed February 1, 2002.

### Field of the Invention

This invention relates to controlled release pharmaceutical dosage forms of a cholesteryl ester transfer protein inhibitor (CETPI), methods of using and methods of making the same.

### Background of the Invention

CETP inhibitors, particularly those that have high binding activity, are generally hydrophobic, have extremely low aqueous solubility and have low oral bioavailability when dosed conventionally. Such compounds have generally proven to be difficult to formulate for oral administration such that high bioavailabilities are achieved.

Atherosclerosis and its associated coronary artery disease is the leading cause of death in the industrialized world. Despite attempts to modify secondary risk factors (smoking, obesity, lack of exercise) and treatment of dyslipidemia with dietary modification and drug therapy, coronary heart disease (CHD) remains the most common cause of death in the U.S., where cardiovascular disease accounts for 44% of all deaths, with 53% of these associated with atherosclerotic coronary heart disease.

Risk for development of this condition has been shown to be strongly correlated with certain plasma lipid levels. While elevated LDL-cholesterol may be the most recognized form of dyslipidemia, it is by no means the only significant lipid associated contributor to CHD. Low HDL-cholesterol is also a known risk factor for CHD (Gordon, D.J., et al.,:"High-density Lipoprotein Cholesterol and Cardiovascular Disease", Circulation, (1989), 79: 8-15).

High LDL-cholesterol and triglyceride levels are positively correlated, while high levels of HDL-cholesterol are negatively correlated with the risk for developing cardiovascular diseases. Thus, dyslipidemia is not a unitary risk profile for CHD but may be comprised of one or more lipid aberrations.

Among the many factors controlling plasma levels of these disease dependent principles, cholesteryl ester transfer protein activity affects all three. The role of this 70,000 dalton plasma glycoprotein found in a number of animal species, including humans, is to transfer cholesteryl ester and triglyceride between lipoprotein particles, including HDL, LDL, very low density lipoproteins (VLDL), and chylomicrons. The net result of CETP activity is a lowering of HDL cholesterol and an increase in LDL cholesterol. This effect on lipoprotein profile is believed to be pro-atherogenic, especially in subjects whose lipid profile constitutes an increased risk for CHD.

No wholly satisfactory HDL-elevating therapies exist. Niacin can significantly increase HDL, but has serious toleration issues, which reduce compliance. Fibrates and the HMG CoA reductase inhibitors raise HDL-cholesterol only modestly (±10-12%). As a result, there is a significant unmet medical need for a well-tolerated agent, which can significantly elevate plasma HDL levels, thereby reversing or slowing the progression of atherosclerosis.

CETP inhibitors have been developed which inhibit CETP activity, and thus, if present in the blood, will result in higher HDL cholesterol levels and lower LDL cholesterol levels. To be effective, such CETP inhibitors must be absorbed into the blood. Oral dosing of CETP inhibitors is preferred because to be effective such CETP inhibitors must be taken on a regular basis, such as daily. Therefore, it is preferred that patients be able to take CETP inhibitors by oral dosing rather than by injection.

However, it has proven to be difficult to formulate CETP inhibitors for oral administration such that therapeutic blood levels are achieved. CETP inhibitors in general possess a number of characteristics, which render them poorly bioavailable when dosed orally in a conventional manner. CETP inhibitors tend to be quite hydrophobic and extremely water insoluble, with solubility in aqueous solution of usually less than about 10 microgm/ml and typically less than 1 microgm/ml. Often, the aqueous solubility of CETP inhibitors is less than 0.1 microgm/ml. Indeed, the solubility of some CETP inhibitors is so low that it is in fact difficult to measure. Accordingly, when CETP inhibitors are dosed orally, concentrations of CETP inhibitor in the aqueous environment of the gastrointestinal tract tend to be extremely low, resulting in poor absorption from the GI tract to blood. The hydrophobicity of CETP inhibitors not only leads to low equilibrium aqueous solubility but also tends to make the drugs poorly wetting and slow to dissolve, further reducing their tendency to dissolve and be absorbed from the gastrointestinal tract. This combination of characteristics has resulted in the bioavailability for orally dosed conventional crystalline or amorphous forms of CETP inhibitors generally to be quite low, often having absolute bioavailabilities of less than 1%.

Various attempts have been made to improve the aqueous concentration of CETP inhibitors, but generally have met with limited success. At the outset, most methods aimed at enhancing aqueous concentration and bioavailability of low-solubility drugs only offer moderate improvements. Such improvements generally lead to enhancements in aqueous concentration on the order of from one to seven fold. In addition, the enhancement may be short-lived, with the drug concentration returning to the equilibrium concentration within 10 to 40 minutes. Such small, short-lived concentration enhancements have led to even lower levels of bioavailability enhancement when tested *in vivo* via oral administration. Thus, when conventional dosage forms of low-solubility drugs are tested *in vivo* via oral administration, bioavailability enhancements are typically on the order of 2-fold to 4-fold or less. For CETP inhibitors having low absolute bioavailabilities, such small improvements are insufficient to allow convenient oral dosing of CETP inhibitors; that is, dosage forms having a convenient size and frequency of dosing.

Convenient oral administration of CETPIs, to a patient is particularly difficult. Because CETPIs have such a low aqueous solubility, dosage forms of CETPIs advantageously contain a means to aid dissolution or solubility of the CETPI in the gastrointestinal (GI) tract and to also inhibit precipitation of the CETPI in the GI tract. If the CETPI precipitates In the GI tract, then it is not available for absorption across the intestinal wall, and will not elicit its therapeutic effect. The inclusion of the dissolution-enhancing means increases the size of the dosage form, e.g. tablet or capsule. It is important that this oral dosage form be of a size, which is easily swallowed, particularly for elderly patients. It is also preferable that the number of dosage forms taken per dose be low, preferably one unit, because many patients take multiple drugs. Furthermore, it is important that dosing be convenient, i.e. once-per-day or twice-per-day, because patients who take multiple drugs may have a difficult time keeping track of which drugs to take at which time of day. Furthermore, some drugs such as CETPIs are advantageously taken with a meal, and it is preferable to minimize the number of times per day that the drug is taken, to simplify the logistics related to taking the drug with a meal.

Several pharmaceutical compositions comprising a cholesteryl ester transfer protein inhibitor are already known. As a matter of example, WO 02/117710 relates to controlled release dosage forms comprising a solid amorphous dispersion of a cholesteryl ester transfer protein inhibitor and a concentration-enhancing polymer. According to another example, EP-A-1 269 994 relates to a combination of a solubility-improved drug form with a concentration-enhancing polymer in a sufficient amount so that the combination provides substantially enhanced drug concentration in a use environment relative to a control comprising the same amount of the same drug form without the concentration-enhancing polymer.

### Summary of the Invention

Thus it is apparent that it is desirable to have a dosage form of a CETPI which:
(a) contains a dissolution- or solubility-enhancing means;
(b) comprises one or at most two units per dose;
(c) can be easily swallowed; and
(d) can be dosed once or twice per day.

Furthermore, it would be desirable to have a method of lowering the maximum CETPI concentration in the plasma (Cmax) after dosing while still providing good bioavailability, in order to decrease undesirable side effects, relative to an immediate release dosage form containing an equivalent amount of CETPI. This invention relates to pharmaceutical dosage forms that provide a controlled release of a low solubility cholesteryl ester transfer protein inhibitor (CETPI) to an environment of use.
In one embodiment, the environment of use is the gastrointestinal tract of a human or other mammal.

This invention relates to controlled release pharmaceutical compositions containing a CETPI and the use of such compositions to elevate certain plasma lipid levels, including high density lipoprotein (HDL)-cholesterol and to lower certain other plasma lipid levels, such as low density lipoprotein (LDL)-cholesterol and triglycerides and accordingly to treat diseases which are affected by low levels of HDL cholesterol and/or high levels of LDL-cholesterol and triglycerides, such as atherosclerosis and cardiovascular diseases in certain mammals (i.e., those which have CETP in their plasma), including humans.

In one aspect, the invention relates to a dosage form comprising:
(a) A CETPI in a solubility-enhanced form;
(b) An optional means to prevent precipitation of the CETPI in the use environment after delivery to this environment; and
(c) A controlled release (CR) means for delivering the CETPI slowly to the use environment.

One preferred solubility-enhanced form of a CETPI is a solid amorphous dispersion, preferably a molecular dispersion, of the CETPI in a polymer termed a Concentration-Enhancing Polymer (CEP). This solid amorphous dispersion is a solid, which is further contained within a dosage form. The dosage form optionally additionally contains a precipitation-inhibiting polymer (PIP), which delays or prevents precipitation of the CETPI in the use environment for a period of time. When the solid amorphous dispersion is delivered to an environment of use, e.g. the human gastrointestinal tract, the CETPI supersaturates the GI lumenal fluid and this supersaturation is maintained for a period of time long enough for the drug to be absorbed across the GI wall into the blood stream. A more preferred solubility-enhanced form of the CETPI is a dispersion in which the CEP also serves the function of a PIP, thus decreasing the quantity of excipients in the dosage form because a PIP is not added to the CEP/ CETPI dispersion.

Another preferred solubility-enhanced form of a CETPI is an amorphous CETPI powder, i.e. amorphous CETPI not in a solid amorphous dispersion.

The CR means comprises any solid dosage form which may be swallowed, and which slowly releases the solubility-enhanced form of the CETPI and the optional PIP. The CETPI and the optional PIP may be delivered into the use environment as a solution or as a suspension, that is as small particles, which then dissolve in the use environment. A preferred CR means is an osmotic tablet which is coated with a semipermeable membrane, and has one or more exit ports through this membrane through which the CETPI and optionally the PIP are released into the use environment. In one embodiment of an osmotic tablet, a "monolayer osmotic tablet", the CETPI and various excipients are contained in a single layer, which possesses the ability to pump itself through the exit port(s) when hydrated. In another embodiment of an osmotic tablet, a "bilayer osmotic tablet", the CETPI and optionally the PIP, are contained in one layer of a bilayer tablet which is accessible to the exit port(s), and another swelling layer contains a polymer which swells when hydrated thus aiding in delivery of the CETPI, and the optional PIP through the exit port(s).

Another preferred CR means is a matrix tablet, which releases the CETPI, and optionally the PIP, by diffusion or more preferably by erosion. For example, an eroding matrix tablet slowly erodes in the use environment, releasing particles of CETPI, and the optional PIP.

While it is certainly desirable to administer the entire dose in one dosage form unit, e.g. in one tablet, this may be difficult to achieve, and more than one dosage form unit may be dosed to achieve the therapeutic effect. For example, two 60 mg QD (once daily) CR dosage forms may be dosed to achieve a total dose of 120 mg. Likewise, two 120 mg QD CR dosage forms may be dosed to achieve a total dose of 240 mg. Likewise, Two 120 mg BID (twice daily) CR dosage forms may be dosed to achieve a total dose of 240 mg.

The above doses are exemplary, and any dose in the range 5 mg to 500 mg may be used. Preferably, the dose of CETPI is from about 5 mg to about 240 mg.

In another aspect, the invention relates to a CR dosage form which releases the CETPI in the Gl tract of a human at a rate which results in about 50% or more, preferably about 70% or more, more preferably about 80% or more, even more preferably about 90% or more inhibition of plasma CETP, for about 12 hours or more, preferably for about 16 hours or more. Said CR dosage form is dosed at most BID, preferably QD. The achievement of this aspect depends upon the CETPI dose and the CETPI release rate from the CR dosage form. Operational doses and release rates may be determined by pharmacokinetic modeling, as exemplified in the Examples of this application. Preferred doses and release rates are set out in the Detailed Description of the Invention below.

In another aspect, the invention relates to a CETPI CR dosage form which, when dosed to a human, results in about 50% or more, preferably about 70% or more, more preferably about 80% or more, even more preferably about 90% or more inhibition of plasma CETP, for a period of time which is greater than 30 minutes longer, preferably greater than 60 minutes longer, more preferably greater than 120 minutes longer, than the time period for inhibition elicited by dosing an immediate release dosage form at the same dose. A preferred CETPI CR dosage form will exhibit this effect at a lower dose than an immediate release dosage form. The achievement of this aspect depends upon the CETPI dose and the CETPI release rate from the CR dosage form. Operational doses and release rates may be determined by pharmacokinetic modeling, as exemplified in the Examples of this application.

In another aspect, the invention relates to a CR dosage form which releases CETPI in the GI tract of a human at a rate which results In a mean increase In HDL cholesterol level of about 20% or greater, after dosing for 8 weeks. Preferred CR dosage forms of this invention also result in a mean decrease in LDL cholesterol levels of about 10% or greater.

In another aspect, the invention relates to a CR CETPI dosage form which is dosed twice-per-day (BID), which dosage form provides efficacious CETP inhibition at a total daily dose which is less than that of an equivalently efficacious BID immediate release (IR) dosage form or once-per-day (QD) IR dosage form.

In another aspect, the invention relates to a controlled release dosage form of a CETPI which, after oral dosing, results in a 20% or greater decrease in plasma Cmax, relative to dosing an immediate release CETPI dosage form at the same dose.

Description of the CETPI release rate of a CETPI CR dosage form is complicated by the fact that such dosage forms may have initial delay periods during which little or no release occurs, and may release CETPI according to zero-order, first-order, mixed-order or other kinetics. To avoid confusion, we describe CR dosage form release rates in terms of the time duration between dosing the dosage form to an environment of use and the time at which 80% of the CETPI has left the dosage form. This description applies to all CR dosage forms, which release CETPI, regardless of the shape of the % released vs. time curve.

Reference to a "use environment" can either mean *in vivo* fluids, such as the fluid in the lumen of the GI tract of an animal, such as a mammal and particularly a human, or the *in vitro* environment of a test solution, such as phosphate buffered saline (PBS) or a Model Fasted Duodenal (MFD) solution. An appropriate PBS solution is an aqueous solution comprising 20 mM sodium phosphate (Na₂HPO₄), 47 mM potassium phosphate (KH₂PO₄), 87 mM NaCl, and 0:2 mM KCl, adjusted to pH 6.5 with NaOH. An appropriate MFD solution is the same PBS solution wherein additionally is present 7.3 mM sodium taurocholic acid and 1.4 mM of 1-palmitoyl-2-oleyl-sn-glycero-3-phosphocholine.

"Administration" to a use environment means, where the use environment is *in vivo,* delivery by ingestion or swallowing or other means to deliver the drugs. Where the use environment is *in vitro,* "administration" refers to placement or delivery of the dosage form to the *in vitro* test medium. Where release of drug into the stomach is not desired but release of the drug in the duodenum or small intestine is desired, the use environment may also be the duodenum or small intestine. In such cases, "introduction" to a use environment is that point in time when the dosage form leaves the stomach and enters the duodenum.

### Detailed Description of the Invention

The current invention comprises CR dosage forms of CETPIs which, after oral dosing, elicit one or more of the following effects: (a) about 50% or more, preferably about 70% or more, more preferably about 80% or more, even more preferably about 90% or more inhibition of plasma CETP, for about 12 hours or more, preferably about 16 hours or more; more preferably about 24 hours or more; (b) a decrease of 20% or more in mean plasma Cmax; (c) a mean increase in HDL cholesterol level of about 20% or greater, after dosing for 8 weeks; (d) a mean decrease in LDL cholesterol levels of about 10% or greater, after dosing for 8 weeks. In other words, the dosage form, following administration to an *in vivo* use environment, provides at least one of: (i) at least 50% inhibition of plasma cholesteryl ester transfer protein for at least 12 hours; (ii) a maximum drug concentration in the blood that is less than or equal to 80% of the maximum drug concentration in the blood provided by an immediate release dosage form consisting of the same amount of the solubility-enhanced form of said CETPI; (iii) a mean HDL cholesterol level after dosing for 8 weeks that is at least about 1.2-fold that obtained prior to dosing; and (iv) a mean LDL cholesterol level after dosing for 8 weeks that is less than or equal to about 90% that obtained prior to dosing. CR CETPI dosage forms of this invention are dosed at most BID, preferably QD.

Preferred embodiments exhibit two of the above effects. More preferred embodiments exhibit three or four of the above effects.

Controlled release dosage forms of CETPIs may be dosed to a human subject in the fasted or fed state. It is preferred that they be dosed in the fed state.

Preferred CETPI doses and CETPI release rates from the controlled release dosage forms of this invention may be determined by pharmacokinetic (PK) modeling for individual CETPIs, or by clinical experimentation (i.e. in human subjects or patients) as familiar to those experienced in the art. The use of PK modeling for the purpose of optimizing plasma CETPI concentrations and %CETP inhibition is exemplified in the Examples below. PK modeling may also be used to predict Cmax for various CETPI doses and release rates, in order to identify those doses and release rates which will decrease Cmax by 20% or more, relative to an immediate release dosage form at the same dose.

CR CETPI dosage forms of this invention comprise:
(a) A CETPI in a solubility-enhanced form;
(b) An optional means to prevent precipitation of the CETPI in the use environment after delivery to the use environment; and
(c) A controlled release (CR) means for delivering the CETPI slowly to the use environment.

The solubility-enhanced form of the CETPI is any form which is capable of supersaturating, at least temporarily, an aqueous use environment by a factor of about 2-fold or more, preferably 10-fold or more, relative to the solubility of crystalline CETPI. That is, the solubility-enhanced form provides a maximum dissolved drug concentration of the CETPI that is at least 2-fold, more preferably at least 10-fold, the equilibrium drug concentration provided by the crystalline form of the CETPI alone. The determination of concentration-enhancement provided by the solubility-enhanced form is performed with the solubility-enhanced form alone, rather than in the CR dosage form. Alternatively, the solubility-enhanced form provides an area under the drug concentration versus time curve (AUC) that is at least 1.25-fold, preferably at least 5-fold and more preferably at least 25-fold that provided by the control composition. (Determination of AUCs is described in more detail below.) The control composition is conventionally the lowest-energy crystalline form of the drug alone without any solubilizing additives or any CEP or PIP.

Alternatively the solubility-enhanced form may consist of amorphous CETPI. The solubility-enhanced form may comprise a solid amorphous dispersion of the CETPI in a Concentration-Enhancing Polymer (CEP) or low molecular weight water-soluble material. Solid amorphous dispersions of CETPIs and Concentration-Enhancing Polymers are disclosed more fully in commonly assigned U.S. patent application serial number 09/918, 127, filed July 30, 2001, and U.S. patent application serial number 10/066,091, filed February 1, 2002. The solubility-enhanced form may comprise nanoparticles, i.e. solid drug particles of diameter less than approximately 900 nm, optionally stabilized by small quantities of surfactants or polymers, as described in US Patent 5,145,884. The solubility-enhanced form may comprise adsorbates of the drug in a crosslinked polymer, as described in US Patent 5.225,192. The solubility-enhanced form may comprise a nanosuspension, the nanosuspension being a disperse system of solid-in-liquid or solid-in-semisolid, the dispersed phase comprising pure drug or a drug mixture, as described in U.S. Patent No. 5,858,410. The solubility-enhanced form may comprise drug that is in a supercooled form, as described in U.S. Patent No. 6.197,349. The solubility-improved drug form may comprise a drug/cyclodextrin drug form, including those described in U.S. Patent Nos. 5,134,127. 6,046,177, 5,874,418, and 5,376,646. The solubility-improved drug form may comprise a softgel form, such as a drug mixed with a lipid or colloidal protein (e.g., gelatin), including those described in U.S. Patent Nos. 5,851,275, 5,834,022 and 5,686,133. The solubility-enhanced drug form may comprise a self-emulsifying form, including those described in U.S. Patent Nos. 6,054,136 and 5,993,858. The solubility-enhanced drug form may comprise a three-phase drug form, including those described in U.S. Patent No. 6,042,847. The above solubility-enhanced drug forms may also be mixed with a concentration-enhancing polymer to provide improved solubility enhancements, as disclosed in commonly assigned copending U.S. Provisional Patent Application Serial No. 60/300,314, filed June 22, 2001. The solubility-enhanced form may also comprise (1) a crystalline highly soluble form of the drug such as a salt; (2) a high-energy crystalline form of the drug; (3) a hydrate or solvate crystalline form of a drug; (4) an amorphous form of a drug (for a drug that may exist as either amorphous or crystalline); (5) a mixture of the drug (amorphous or crystalline) and a solubilizing agent; or (6) a solution of the drug dissolved in an aqueous or organic liquid. The above drug forms may also be mixed with a concentration-enhancing polymer to provide improved solubility enhancements, as disclosed in commonly assigned copending U.S. Patent Application Serial No. 09/742,785 filed December 20, 2000. The solubility-enhanced drug form may also comprise (a) a solid dispersion comprising drug and a matrix, wherein at least a major portion of the drug in the dispersion is amorphous; and (b) a concentration-enhancing polymer, as disclosed in commonly assigned copending U.S. Provisional Patent Application Serial No. 60/300,261, filed June 22, 2001. The solubility-enhanced drug form may also comprise a solid adsorbate comprising a low-solubility drug adsorbed onto a substrate, the substrate having a surface area of at least 20 m²/g, and wherein at least a major portion of the drug in the solid adsorbate is amorphous. The solid adsorbate may optionally comprise a concentration-enhancing polymer. The solid adsorbate may also be mixed with a concentration-enhancing polymer. Such solid adsorbates are disclosed in commonly assigned copending U.S. Provisional Patent Application Serial No. 60/300,260, filed June 22, 2001.

The optional "means to prevent precipitation" comprises any excipient which maintains the supersaturation of a CETPI, e.g. Drug A, in an aqueous environment of use, e.g. the human GI tract. Preferable excipients for this purpose are polymers which are soluble at some pH in the pH range of the GI tract, i.e. pH 1-8, as described in detail below. For the purpose of description of this invention, these polymers are called "Concentration-Enhancing Polymers" (CEP) when they are incorporated into a CETPI dispersion, and may also be called "Precipitation-Inhibiting Polymers" (PIP) when they are physically blended with amorphous CETPI or with a CETPI/CEP dispersion. As used herein, "QD" means once daily and "BID" means twice daily.

### THE DRUG

The CR dosage forms of this invention deliver CETP inhibitors in an optimal manner. Exemplary CETP inhibitors are described in the following.

In the following, by "pharmaceutically acceptable forms" thereof is meant any pharmaceutically acceptable derivative or variation, including stereoisomers, stereoisomer mixtures, enantiomers, solvates, hydrates, isomorphs, polymorphs, salt forms and prodrugs.

A class of CETP inhibitors that finds utility with the present invention consists of (R)-chiral
halogenated 1-substituted amino-(n+I)-alkanols having the Formula XVI 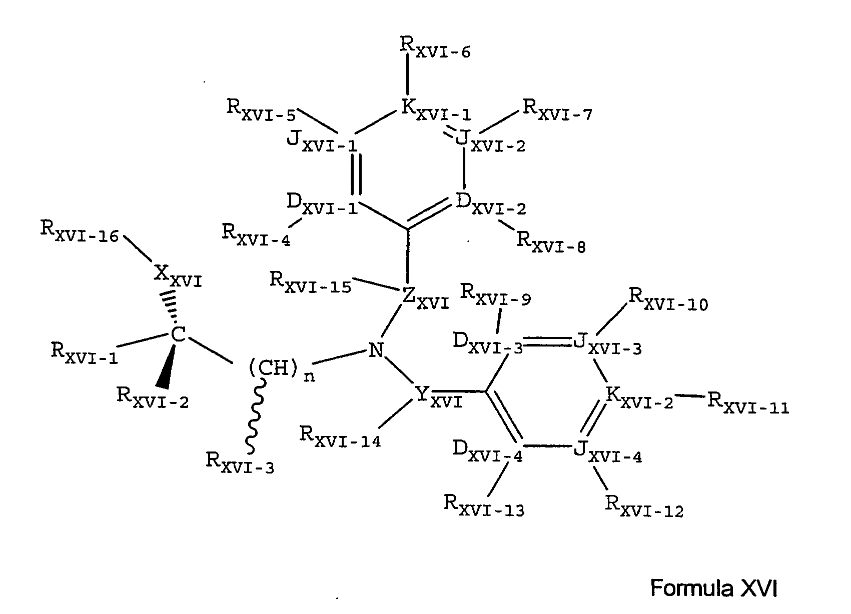 and pharmaceutically acceptable forms thereof, wherein:
n_{XVI} is an integer selected from 1 through 4;
X_{XVI} is oxy;
R_{XVI-1} is selected from the group consisting of haloalkyl, haloalkenyl, haloalkoxymethyl, and haloalkenyloxymethyl with the proviso that R_{XVI-1} has a higher Cahn-Ingold-Prelog stereochemical system ranking than both R_{XVI-2} and (CHR_{XVI-3})ₙ-N(A_{XVI})Q_{XVI} wherein A_{XVI} is Formula XVI-(II) and Q is Formula XVI-(III);

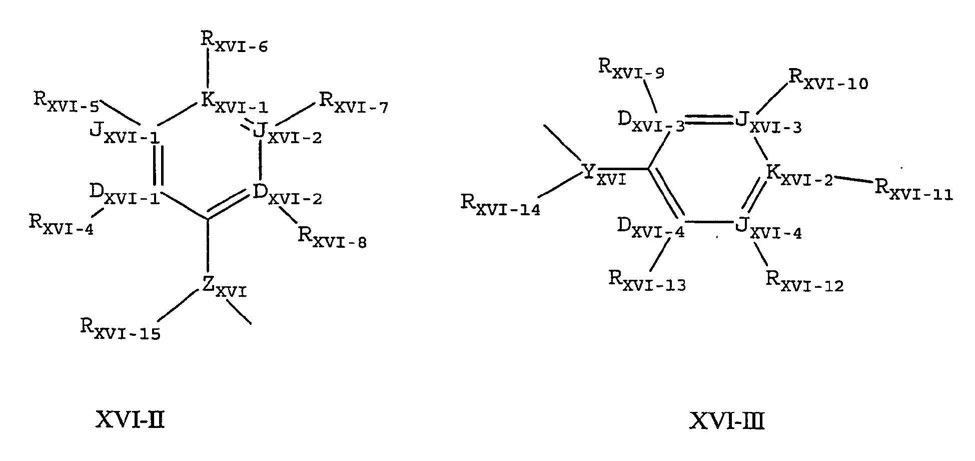

R_{XVI-16} is selected from the group consisting of hydrido, alkyl, acyl, aroyl, heteroaroyl, trialkylsilyl, and a spacer selected from the group consisting of a covalent single bond and a linear spacer moiety having a chain length of 1 to 4 atoms linked to the point of bonding of any aromatic substituent selected from the group consisting of R_{XVI-4}, R_{XVI-8}, R_{XVI-9}, and R_{XVI-13} to form a heterocyclyl ring having from 5 through 10 contiguous members;

D_{XVI-1}, D_{XVI-2}, J_{XVI-1}, J_{XVI-2} and K_{XVI-1} are independently selected from the group consisting of C, N, O, S and covalent bond with the provisos that no more than one of D_{XVI-1}, D_{XVI-2}, J_{XVI-1}, J_{XVI-2} and K_{XVI-1} is a covalent bond, no more than one D_{XVI-1}, D_{XVI-2}, J_{XVI-1}, J_{XVI-2} and K_{XVI-1} is be O, no more than one of D_{XVI-1}, D_{XVI-2}, J_{XVI-1}, J_{XVI-2} and K_{XVI-1} is S, one of D_{XVI-1}, D_{XVI-2}, J_{XVI-1}, J_{XVI-2} and K_{XVI-1} must be a covalent bond when two of D_{XVI-1}, D_{XVI-2}, J_{XVI-1}, J_{XVI-2} and K_{XVI-1} are O and S, and no more than four of D_{XVI-1}, D_{XVI-2}, J_{XVI-1}, J_{XVI-2} and K_{XVI-1} is N;

D_{XVI-3}, D_{XVI-4}, J_{XVI-3}, J_{XVI-4} and K_{XVI-2} are independently selected from the group consisting of C, N, O, S and covalent bond with the provisos that no more than one is a covalent bond, no more than one of D_{XVI-3}, D_{XVI-4}, J_{XVI-3}, J_{XVI-4} and K_{XVI-2} is O, no more than one of D_{XVI-3}, D_{XVI-4}, J_{XVI-3}, J_{XVI-4} and K_{XVI-2} is S, no more than two of D_{XVI-3}, D_{XVI-4}, J_{XVI-3}, J_{XVI-4} and K_{XVI-2} is 0 and S, one of D_{XVI-3}, D_{XVI-4}, J_{XVI-3}, J_{XVI-4} and K_{XVI-2} must be a covalent bond when two of D_{XVI-3}, D_{XVI-4}, J_{XVI-3}, J_{XVI-4} and K_{XVI-2} are O and S, and no more than four of D_{XVI-3}, D_{XVI-4}, J_{XVI-3}, J_{XVI-4} and K_{XVI-2} are N;

R_{XVI-2} is selected from the group consisting of hydrido, aryl, aralkyl, alkyl, alkenyl, alkenyloxyalkyl, haloalkyl, haloalkenyl, halocycloalkyl, haloalkoxy, haloalkoxyalkyl, haloalkenyloxyalkyl, halocycloalkoxy, halocycloalkoxyalkyl, perhaloaryl, perhaloaralkyl, perhaloaryloxyalkyl, heteroaryl, dicyanoalkyl, and carboalkoxycyanoalkyl, with the proviso that R_{XVI-2} has a lower Cahn-Ingold-Prelog system ranking than both R_{XVI-1} and (CHR_{XVI-3})ₙ-N(A_{XVI})Q_{XVI};

R_{XVI-3} is selected from the group consisting of hydrido, hydroxy, cyano, aryl, aralkyl, acyl, alkoxy, alkyl, alkenyl, alkoxyalkyl, heteroaryl, alkenyloxyalkyl, haloalkyl, haloalkenyl, haloalkoxy, haloalkoxyalkyl, haloalkenyloxyalkyl, monocyanoalkyl, dicyanoalkyl, carboxamide, and carboxamidoalkyl, with the provisos that (CHR_{XVI-3})ₙ-N(A_{XVI})Q_{XVI} has a lower Cahn-Ingold-Prelog stereochemical system ranking than R_{XVI-1} and a higher Cahn-Ingold-Prelog stereochemical system ranking than R_{XVI-2};

Y_{XVI} is selected from a group consisting of a covalent single bond, (C(R_{XVI-14})₂)_{q} wherein q is an integer selected from 1 and 2 and (CH(R_{XVI-14}))_{g}-W_{XVI}-(CH(R_{XVI-14}))ₚ wherein g and p are integers independently selected from 0 and 1;

R_{XVI-14} is selected from the group consisting of hydrido, hydroxy, cyano, hydroxyalkyl, acyl, alkoxy, alkyl, alkenyl, alkynyl, alkoxyalkyl, haloalkyl, haloalkenyl, haloalkoxy, haloalkoxyalkyl, haloalkenyloxyalkyl, monocarboalkoxyalkyl, monocyanoalkyl, dicyanoalkyl, carboalkoxycyanoalkyl, carboalkoxy, carboxamide, and carboxamidoalkyl;

Z_{XVI} is selected from a group consisting of a covalent single bond, (C(R_{XVI-15})₂)_{q}, wherein q is an integer selected from 1 and 2, and (CH(R_{XVI-15}))ⱼ-W_{XVI}-(CH(R_{XVI-15}))ₖ wherein j and k are integers independently selected from 0 and 1;

W_{XVI} is selected from the group consisting of O, C(O), C(S),C(O)N(R_{XVI-14}), C(S)N(R_{XVI-14}),(R_{XVI-14})NC(O), (R_{XVI-14})NC(S), S, S(O), S(O)₂, S(O)₂N(R_{XVI-14}), (R_{XVI-14})NS(O)₂, and N(R_{XVI-14}) with the proviso that R_{XVI-14} is other than cyano;

R_{XVI-15} is selected, from the group consisting of hydrido, cyano, hydroxyalkyl, acyl, alkoxy, alkyl, alkenyl, alkynyl, alkoxyalkyl, haloalkyl, haloalkenyl, haloalkoxy, haloalkoxyalkyl, haloalkenyloxyalkyl, monocarboalkoxyalkyl, monocyanoalkyl, dicyanoalkyl, carboalkoxycyanoalkyl, carboalkoxy, carboxamide, and carboxamidoalkyl;

R_{XVI-4}, R_{XVI-5}, R_{XVI-6}, R_{XVI-7}, R_{XVI-8}, R_{XVI-9}, R_{XVI-10}, R_{XVI-11}, R_{XVI-12}, and R_{XVI-13} are independently selected from the group consisting of hydrido, carboxy, heteroaralkylthio, heteroaralkoxy, cycloalkylamino, acylalkyl, acylalkoxy, aroylalkoxy, heterocyclyloxy, aralkylaryl, aralkyl, aralkenyl, aralkynyl, heterocyclyl, perhaloaralkyl, aralkylsulfonyl, aralkylsulfonylalkyl, aralkylsulfinyl, aralkylsulfinylalkyl, halocycloalkyl, halocycloalkenyl, cycloalkylsulfinyl, cycloalkylsulfinylalkyl, cycloalkylsulfonyl, cycloalkylsulfonylalkyl, heteroarylamino, N-heteroarylamino-N-alkylamino, heteroaralkyl, heteroarylaminoalkyl, haloalkylthio, alkanoyloxy, alkoxy, alkoxyalkyl, haloalkoxylalkyl, heteroaralkoxy, cycloalkoxy, cycloalkenyloxy, cycloalkoxyalkyl, cycloalkylalkoxy, cycloalkenyloxyalkyl, cycloalkylenedioxy, halocycloalkoxy, halocycloalkoxyalkyl, halocycloalkenyloxy, halocycloalkenyloxyalkyl, hydroxy, amino, thio, nitro, lower alkylamino, alkylthio, alkylthioalkyl, arylamino, aralkylamino, arylthio, arylthioalkyl, heteroaralkoxyalkyl, alkylsulfinyl, alkylsulfinylalkyl, arylsulfinylalkyl, arylsulfonylalkyl, heteroarylsulfinylalkyl, heteroarylsulfonylalkyl, alkylsulfonyl, alkylsulfonylalkyl, haloalkylsulfinylalkyl, haloalkylsulfonylalkyl, alkylsulfonamido, alkylaminosulfonyl, amidosulfonyl, monoalkyl amidosulfonyl, dialkyl, amidosulfonyl, monoarylamidosulfonyl, arylsulfonamido, diarylamidosulfonyl, monoalkyl monoaryl amidosulfonyl, arylsulfinyl, arylsulfonyl, heteroarylthio, heteroarylsulfinyl, heteroarylsulfonyl, heterocyclylsulfonyl, heterocyclylthio, alkanoyl, alkenoyl, aroyl, heteroaroyl, aralkanoyl, heteroaralkanoyl, haloalkanoyl, alkyl, alkenyl, alkynyl, alkenyloxy, alkenyloxyalky, alkylenedioxy, haloalkylenedioxy, cycloalkyl, cycloalkylalkanoyl, cycloalkenyl, lower cycloalkylalkyl, lower cycloalkenylalkyl, halo, haloalkyl, haloalkenyl, haloalkoxy, hydroxyhaloalkyl, hydroxyaralkyl, hydroxyalkyl, hydoxyheteroaralkyl, haloalkoxyalkyl, aryl, heteroaralkynyl, aryloxy, aralkoxy, aryloxyalkyl, saturated heterocyclyl, partially saturated heterocyclyl, heteroaryl, heteroaryloxy, heteroaryloxyalkyl, arylalkenyl, heteroarylalkenyl, carboxyalkyl, carboalkoxy, alkoxycarboxamido, alkylamidocarbonylamido, arylamidocarbonylamido, carboalkoxyalkyl, carboalkoxyalkenyl, carboaralkoxy, carboxamido, carboxamidoalkyl, cyano, carbohaloalkoxy, phosphono, phosphonoalkyl, diaralkoxyphosphono, and diaralkoxyphosphonoalkyl with the proviso that R_{XVI-4}, R_{XVI-5}, R_{XVI-6}, R_{XVI-7}, R_{XVI-8}, R_{XVI-9}, R_{XVI-10}, R_{XVI-11}, R_{XVI-12}, and R_{XVI-13} are each independently selected to maintain the tetravalent nature of carbon, trivalent nature of nitrogen, the divalent nature of sulfur, and the divalent nature of oxygen;

R_{XVI-4} and R_{XVI-5}, R_{XVI-5} and R_{XVI-6}, R_{XVI-6} and R_{XVI-7}, R_{XVI-7} and R_{XVI-8}, R_{XVI-9} and R_{XVI-10}, R_{XVI-10} and R_{XVI-11}, R_{XVI-11} and R_{XVI-12}, and R_{XVI-12} and R_{XVI-13} are independently selected to form spacer pairs wherein a spacer pair is taken together to form a linear moiety having from 3 through 6 contiguous atoms connecting the points of bonding of said spacer pair members to form a ring selected from the group consisting of a cycloalkenyl ring having 5 through 8 contiguous members, a partially saturated heterocyclyl ring having 5 through 8 contiguous members, a heteroaryl ring having 5 through 6 contiguous members, and an aryl with the provisos that no more than one of the group consisting of spacer pairs R_{XVI-4} and R_{XVI-5}, R_{XVI-5} and R_{XVI-6}, R_{XVI-6} and R_{XVI-7}, and R_{XVI-7} and R_{XVI-8} is used at the same time and that no more than one of the group consisting of spacer pairs R_{XIV-9} and R_{XVI-10}, R_{XVI-10} and R_{XVI-11}, R_{XVI-11} and R_{XVI-12}, and R_{XVI-12} and R_{XVI-13} can be used at the same time;

R_{XVI-4} and R_{XVI-9}, R_{XVI-4} and R_{XVI-13}, R_{XVI-8} and R_{XVI-9}, and R_{XVI-8} and R_{XVI-13} is independently selected to form a spacer pair wherein said spacer pair is taken together to form a linear moiety wherein said linear moiety forms a ring selected from the group consisting of a partially saturated heterocyclyl ring having from 5 through 8 contiguous members and a heteroaryl ring having from 5 through 6 contiguous members with the proviso that no more than one of the group consisting of spacer pairs R_{XVI-4} and R_{XVI-9}, R_{XVI-4} and R_{XVI-13}, R_{XVI-8} and R_{XVI-9}, and R_{XVI-8} and R_{XVI-13} is used at the same time.

Compounds of Formula XVI are disclosed in WO 00/18724.

In a preferred embodiment, the CETP inhibitor is selected from the following compounds of Formula XVI:
(2R)-3-[[3-(3-trifluoromethoxyphenoxy)phenyl][[3-(1,1,2,2-tetrafluoroethoxy)phenyl]methyl]amino]-1,1,1-trifluoro-2-propanol;
(2R)-3-[[3-(3-isopropylphenoxy)phenyl][[3-(1,1,2,2-tetrafluoroethoxy)phenyl]-methyl]amino]-1,1,1-trifluoro-2-propanol;
(2R)-3-[[3-(3-cyclopropylphenoxy)phenyl][[3-(1,1,2,2-tetrafluoroethoxy)phenyl]-methyl]amino]-1,1,1-trifluoro-2-propanol;
(2R)-3-[[3-(3-(2-furyl)phenoxy)phenyl][[3-(1,1,2,2-tetrafluoroethoxy)phenyl]-methyl]amino]-1,1,1-trifluoro-2-propanol;
(2R)-3-[[3-(2,3-dichlorophenoxy)phenyl][[3-(1,1,2,2-tetrafluoroethoxy)phenyl]-methyl]amino]-1,1,1-trifluoro-2-propanol;
(2R)-3-[[3-(4-fluorophenoxy)phenyl][[3-(1,1,2,2-tetrafluoroethoxy)phenyl]-methyl]amino]-1,1,1-trifluoro-2-propanol;
(2R)-3-[[3-(4-methylphenoxy)phenyl][[3-(1,1,2,2-tetrafluoroethoxy)phenyl]-methyl]amino]-1,1,1-trifluoro-2-propanol;
(2R)-3-[[3-(2-fluoro-5-bromophenoxy)phenyl][[3-(1,1,2,2-tetrafluoroethoxy)phenyl]-methyl]amino]-1,1,1-trifluoro-2-propanol;
(2R)-3-[[3-(4-chloro-3-ethylphenoxy)phenyl][[3-(1,1,2,2-tetrafluoroethoxy)phenyl]-methyl]amino]-1,1,1-trifluoro-2-propanol;
(2R)-3-[[3-[3-(1,1,2,2-tetrafluoroethoxy)phenoxy]phenyl] [[3-(1,1,2,2-tetrafluoro-ethoxy)phenyl]methyl]amino]-1,1,1-trifluoro-2-propanol;
(2R)-3-[[3-[3-(pentafluoroethyl)phenoxy]phenyl][[3-(1,1,2,2-tetrafluoroethoxy)-phenyl]methyl]amino]-1,1,1-trifluoro-2-propanol;
(2R)-3-[[3-(3,5-dimethylphenoxy)phenyl][[3-(1,1,2,2-tetrafluoroethoxy)phenyl]-methyl]amino]-1,1,1-trifluoro-2-propanol;
(2R)-3-[[3-(3-ethylphenoxy)phenyl][[3-(1,1,2,2-tetrafluoroethoxy)phenyl]-methyl]amino]-1,1,1-trifluoro-2-propanol;
(2R)-3-[[3-(3-t-butylphenoxy)phenyl][[3-(1,1,2,2-tetrafluoroethoxy)phenyl]-methyl]amino]-1,1,1-trifluoro-2-propanol:
(2R)-3-[[3-(3-methylphenoxy)phenyl][[3-(1,1,2,2-tetrafluoroethoxy)phenyl]-methyl]amino]-1,1,1-trifluoro-2-propanol;
(2R)-3-[[3-(5,6,7,8-tetrahydro-2-naphthoxy)phenyl][[3-(1,1,2,2-tetrafluoroethoxy)phenyl]methyl]amino]-1,1,1-trifluoro-2-propanol;
(2R)-3-[[3-(phenoxy)phenyl][[3-(1,1,2,2-tetrafluoroethoxy)phenyl]methyl]amino]-1,1,1-trifluoro-2-propanol;
(2R)-3-[[3-[3-(N,N-dimethylamino)phenoxy]phenyl][[3-(1,1,2,2-tetrafluoroethoxy)phenyl]methyl]amino]-1,1,1-trifluoro-2-propanol;
(2R)-3-[[[3-(1,1,2,2,-tetrafluoroethoxy)phenyl]methyl][3-[[3-(trifluoromethoxy)-phenyl]methoxy]phenyl]amino]-1,1,1 -trifluoro-2-propanol;
(2R)-3-[[[3-(1,1,2,2-tetrafluoroethoxy)phenyl]methyl][3-[[3-(trifluoromethyl)phenyl]methoxy]phenyl]amino]-1,1,1-trifluoro-2-propanol;
(2R)-3-[[[3-(1,1,2,2-tetrafluoroethoxy)phenyl]methyl][3-[[3,5-dimethylphenyl]-methoxy]phenyl]amino]-1,1,1-trifluoro-2-propanol;
(2R)-3-[[[3-(1,1,2,2-tetrafluoroethoxy)phenyl]methyl][3-[[3-(trifluoromethylthio)-phenyl]methoxy]phenyl]amino]-1,1,1-trifluoro-2-propanol;
(2R)-3-[[[3-(1,1,2,2-tetrafluoroethoxy)phenyl]methyl][3-[[3,5-difluorophenyl]-methoxy]phenyl]amino]-1,1,1-trifluoro-2-propanol;
(2R)-3-[[[3-(1,1,2,2-tetrafluoroethoxy)phenyl]methyl][3-[cyclohexylmethoxy]-phenyl]amino]-1,1,1-trifluoro-2-propanol;
(2R)-3-[[3-(2-difluoromethoxy-4-pyridyloxy)phenyl][[3-(1,1,2,2-tetrafluoroethoxy)-phenyl]methyl]amino]-1,1,1-trifluoro-2-propanol;
(2R)-3-[[3-(2-trifluoromethyl-4-pyridyloxy)phenyl][[3-(1,1,2,2-tetrafluoroethoxy)-phenyl]methyl]amino]-1,1,1-trifluoro-2-propanol;
(2R)-3-[[3-(3-difluoromethoxyphenoxy)phenyl][[3-(1,1,2,2-tetrafluoroethoxy)-phenyl]methyl]amino]-1,1,1-trifluoro-2-propanol;
(2R)-3-[[[3-(3-trifuoromethylthio)phenoxy]phenyl][[3-(1,1,2,2-tetrafluoroethoxy)-phenyl]methyl]amino]-1,1,1-trifluoro-2-propanol;
(2R)-3-[[3-(4-chloro-3-trifluoromethylphenoxy)phenyl][[3-(1,1,2,2-tetrafluoroethoxy)-phenyl]methyl]amino]-1,1,1-trifluoro-2-propanol;
(2R)-3-[[3-(3-trifluoromethoxyphenoxy)phenyl][[3-(pentafluoroethyl)phenyl]-methyl]amino]-1,1,1-trifluoro-2-propanol;
(2R)-3-[[3-(3-isopropylphenoxy)phenyl][[3-(pentafluoroethyl)phenyl]methyl]-amino]-1,1,1-trifluoro-2-propanol;
(2R)-3-[[3-(3-cyclopropylphenoxy)phenyl][[3-(pentafluoroethyl)phenyl]methyl]-amino]-1,1,1-trifluoro-2-propanol;
(2R)-3-[[3-(3-(2-furyl)phenoxy)phenyl][[3-(pentafluoroethyl)phenyl]methyl]-amino]-1,1,1-trifluoro-2-propanol;
(2R)-3-[[3-(2,3-dichlorophenoxy)phenyl][[3-(pentafluoroethyl)phenyl]methyl]-amino]-1,1,1-trifluoro-2-propanol;
(2R)-3-[[3-(4-fluorophenoxy)phenyl][[3-(pentafluoroethyl)phenyl]methyl]amino]-1,1,1-trifluoro-2-propanol;
(2R)-3-[[3-(4-methylphenoxy)phenyl][[3-(pentafluoroethyl)phenyl]methyl]amino]-1,1,1-trifluoro-2-propanol;
(2R)-3-[[3-(2-fluoro-5-bromophenoxy)phenyl][[3-(pentafluoroethyl)phenyl]methyl]-amino]-1,1,1-trifluoro-2-propanol;
(2R)-3-[[3-(4-chloro-3-ethylphenoxy)phenyl][[3-(pentafluoroethyl)phenyl]methyl]-amino]-1,1,1-trifluoro-2-propanol;
(2R)-3-[[3-[3-(1,1,2,2-tetrafluoroethoxy)phenoxy]phenyl][[3-(pentafluoroethyl)-phenyl]methyl]amino]-1,1,1-trifluoro-2-propanol;
(2R)-3-[[3-[3-(pentafluoroethyl)phenoxy]phenyl][[3-(pentafluoroethyl)phenyl]-methyl]amino]-1,1,1-trifluoro-2-propanol;
(2R)-3-[[3-(3,5-dimethylphenoxy)phenyl][[3-(pentafluoroethyl) phenyl]methyl]-amino]-1,1,1-trifluoro-2-propanol;
(2R)-3-[[3-(3-ethylphenoxy)phenyl][[3-(pentafluoroethyl) phenyl]methyl]amino]-1,1,1-trifluoro-2-propanol;
(2R)-3-[[3-(3-t-butylphenoxy)phenyl][[3-(pentafluoroethyl) phenyl]methyl]amino]-1,1,1-trifluoro-2-propanol;
(2R)-3-[[3-(3-methylphenoxy)phenyl][[3-(pentafluoroethyl) phenyl]methyl]amino]-1,1,1-trifluoro-2-propanol;
(2R)-3-[[3-(5,6,7,8-tetrahydro-2-naphthoxy)phenyl][[3-(pentafluoroethyl)phenyl]-methyl]amino]-1,1,1-trifluoro-2-propanol;
(2R)-3-[[3-(phenoxy)phenyl][[3(pentafluoroethyl) phenyl]methyl]amino]-1,1,1-trifluoro-2-propanol;
(2R)-3-[[3-[3-(N,N-dimethylamino)phenoxy]phenyl][[3-(pentafluoroethyl)phenyl]-methyl]amino]-1,1,1-trifluoro-2-propanol;
(2R)-3-[[[3-(pentafluoroethyl)phenyl]methyl][3-[[3-(trifluoromethoxy)phenyl]-methoxy]phenyl]amino]-1,1,1-trifluoro-2-propanol;
(2R)-3-[[[3-(pentafluoroethyl)phenyl]methyl][3-[[3-(trifluoromethyl)-phenyl]-methoxy]phenyl]amino]-1,1,1-trifluoro-2-propanol;
(2R)-3-[[[3-(pentafluoroethyl)phenyl]methyl][3-[[3,5-dimethylphenyl]methoxy]-phenyl]amino]-1,1,1-trifluoro-2-propanol;
(2R)-3-[[[3-(pentafluoroethyl)phenyl]methyl][3-[[3-(trifluoromethylthio)phenyl]-methoxy]phenyl]amino]-1,1,1-trifluoro-2-propanol;
(2R)-3-[[[3-(pentafluoroethyl)phenyl]methyl][3-[[3,5-difluorophenyl]methoxy]-phenyl]amino]-1,1,1-trifluoro-2-propanol;
(2R)-3-[[[3-(pentafluoroethyl)phenyl]methyl][3-[cyclohexylmethoxy]phenyl]-amino]-1,1,1-trifluoro-2-propanol;
(2R)-3-[[3-(2-difluoromethoxy-4-pyridyloxy)phenyl][[3-(pentafluoroethyl)phenyl]-methyl]amino]-1,1,1-trifluoro-2-propanol;
(2R)-3-[[3-(2-trifluoromethyl-4-pyridyloxy)phenyl][[3-(pentafluoroethyl)phenyl]-methyl]amino]-1,1,1-trifluoro-2-propanol;
(2R)-3-[[3-(3-difluoromethoxyphenoxy)phenyl][[3-(pentafluoroethyl)phenyl]-methyl]amino]-1,1,1-trifluoro-2-propanol;
(2R)-3-[[[3-(3-trifluoromethylthio)phenoxy]phenyl][[3-(pentafluoroethyl)phenyl]-methyl]amino]-1,1,1-trifluoro-2-propanol;
(2R)-3-[[3-(4-chloro-3-trifluoromethylphenoxy)phenyl][[3-(pentafluoroethyl)-phenyl]methyl]amino]-1,1,1-trifluoro-2-propanol;
(2R)-3-[[3-(3-trifluoromethoxyphenoxy)phenyl][[3-(heptafluoropropyl)phenyl]-methyl]amino]-1,1,1-trifluoro-2-propanol;
(2R)-3-[[3-(3-isopropylphenoxy)phenyl][[3-(heptafluoropropyl)phenyl]methyl]-amino]-1,1,1-trifluoro-2-propanol;
(2R)-3-[[3-(3-cyclopropylphenoxy)phenyl][[3-(heptafluoropropyl)phenyl]methyl]-amino]-1,1,1-trifluoro-2-propanol;
(2R)-3-[[3-(3-(2-furyl)phenoxy)phenyl][[3-(heptafluoropropyl) phenyl]methyl]-amino]-1,1,1-trifluoro-2-propanol;
(2R)-3-[[3-(2,3-dichlorophenoxy)phenyl][[3-(heptafluoropropyl) phenyl]methyl]-amino]-1,1,1-trifluoro-2-propanol;
(2R)-3-[[3-(4-fluorophenoxy)phenyl][[3-(heptafluoropropyl) phenyl]methyl]amino]-1,1,1-trifluoro-2-propanol;
(2R)-3-[[3-(4-methylphenoxy)phenyl][[3-(heptafluoropropyl) phenyl]methyl]amino]-1,1,1,-trifluoro-2-propanol;
(2R)-3-[[3-(2-fluoro-5-bromophenoxy)phenyl][[3-(heptafluoropropyl)phenyl]-methyl]amino]-1,1,1-trifluoro-2-propanol;
(2R)-3-[[3-(4-chloro-3-ethylphenoxy)phenyl][[3-(heptafluoropropyl)phenyl]methyl]-amino]-1,1,1-trifluoro-2-propanol;
(2R)-3-[[3-[3-(1,1,2,2-tetrafluoroethoxy)phenoxy]phenyl][[3-(heptafluoropropyl)-phenyl]methyl]amino]-1,1,1-trifluoro-2-propanol;
(2R)-3-[[3-[3-(pentafluoroethyl)phenoxy]phenyl][[3-(heptafluoropropyl)phenyl]-methyl]amino]-1,1,1-trifluoro-2-propanol;
(2R)-3-[[3-(3,5-dimethylphenoxy)phenyl][[3-(heptafluoropropyl) phenyl]methyl]-amino]-1,1,1-trifluoro-2-propanol;
(2R)-3-[[3-(3-ethylphenoxy)phenyl][[3-(heptafluoropropyl) phenyl]methyl]amino]-1,1,1-trifluoro-2-propanol;
(2R)-3-[[3-(3-t-butylphenoxy)phenyl][[3-(heptafluoropropyl) phenyl]methyl]amino]-1,1,1-trifluoro-2-propanol;
(2R)-3-[[3-(3-methylphenoxy)phenyl][[3-(heptafluoropropyl) phenyl]methyl]amino]-1,1,1-trifluoro-2-propanol;
(2R)-3-[[3-(5,6,7,8-tetrahydro-2-naphthoxy)phenyl][[3-(heptafluoropropyl)phenyl]-methyl]amino]-1,1,1-trifluoro-2-propanol;
(2R)-3-[[3-(phenoxy)phenyl][[3-(heptafluoropropyl) phenyl]methyl]amino]-1,1,1-trifluoro-2-propanol;
(2R)-3-[[3-[3-(N,N-dimethylamino)phenoxy]phenyl][[3-(heptafluoropropyl)phenyl]-methyl]amino]-1,1,1-trifluoro-2-propanol;
(2R)-3-[[[3-(heptafluoropropyl)phenyl]methyl][3-[[3-(trifluoromethoxy)phenyl]-methoxy]phenyl]amino]-1,1,1-trifluoro-2-propanol;
(2R)-3-[[[3-(heptafluoropropyl)phenyl]methyl][3-[[3-(trifluoromethyl)phenyl]-methoxy]phenyl]amino]-1,1,1-trifluoro-2-propanol;
(2R)-3-[[[3-(heptafluoropropyl)phenyl]methyl][3-[[3,5-dimethylphenyl]methoxy]-phenyl]amino]-1,1,1-trifluoro-2-propanol;
(2R)-3-[[[3-(heptafluoropropyl)phenyl]methyl][3-[[3-(trifluoromethylthio)phenyl]-methoxy]phenyl]amino]-1,1,1-trifluoro-2-propanol;
(2R)-3-[[[3-(heptafluoropropyl)phenyl]methyl][3-[[3,5-difluorophenyl]methoxy]-phenyl]amino]-1,1,1-trifluoro-2-propanol;
(2R)-3-[[[3-(heptafluoropropyl)phenyl]methyl][3-[cyclohexylmethoxy]phenyl]-amino]-1,1,1-trifluoro-2-propanol;
(2R)-3-[[3-(2-difluoromethoxy-4-pyridyloxy)phenyl][[3-(heptafluoropropyl)phenyl]-methyl]amino]-1,1,1-trifluoro-2-propanol;
(2R)-3-[[3-(2-trifluoromethyl-4-pyridyloxy)phenyl][[3-(heptafluoropropyl)phenyl]-methyl]amino]-1,1,1-trifluoro-2-propanol;
(2R)-3-[[3-(3-difluoromethoxyphenoxy)phenyl][[3-(heptafluoropropyl)phenyl]-methyl]amino]-1,1,1-trifluoro-2-propanol;
(2R)-3-[[[3-(3-trifluoromethylthio)phenoxy]phenyl][[3-(heptafluoropropyl)phenyl]-methyl]amino]-1,1,1-trifluoro-2-propanol;
(2R)-3-[[3-(4-chloro-3-trifluoromethylphenoxy)phenyl][[3-(heptafluoropropyl)-phenyl]methyl]amino]-1,1,1-trifluoro-2-propanol;
(2R)-3-[[3-(3-trifluoromethoxyphenoxy)phenyl][[2-fluoro-5-(trifluoromethyl)-phenyl]methyl]amino]-1,1,1-trifluoro-2-propanol;
(2R)-3-[[3-(3-isopropylphenoxy)phenyl][[2-fluoro-5-(trifluoromethyl)phenyl]-methyl]amino]-1,1,1-trifluoro-2-propanol;
(2R)-3-[[3-(3-cyclopropylphenoxy)phenyl][[2-fluoro-5-(trifluoromethyl)phenyl]-methyl]amino]-1,1,1-trifluoro-2-propanol;
(2R)-3-[[3-(3-(2-furyl)phenoxy)phenyl][[2-fluoro-5-(trifluoromethyl)phenyl]-methyl]amino]-1,1,1-trifluoro-2-propanol;
(2R)-3-[[3-(2,3-dichlorophenoxy)phenyl][[2-fluoro-5-(trifluoromethyl)phenyl]-methyl]amino]-1,1,1-trifluoro-2-propanol;
(2R)-3-[[3-(4-fluorophenoxy)phenyl][[2-fluoro-5-(trifluoromethyl)phenyl]-methyl]amino]-1,1,1-trifluoro-3-propanol;
(2R)-3-[[3-(4-methylphenoxy)phenyl][[2-fluoro-5-(trifluoromethyl)phenyl]-methyl]amino]-1,1,1-trifluoro-2-propanol;
(2R)-3-[[3-(2-fluoro-5-bromophenoxy)phenyl][[2-fluoro-5-(trifluoromethyl)-phenyl]methyl]amino]-1,1,1-trifluoro-2-propanol;
(2R)-3[[3-(4-chloro-3-ethylphenoxy)phenyl][[2-fluoro-5-(trifluoromethyl)-phenyl]methyl]amino]-1,1,1-trifluoro-2-propanol;
(2R)-3-[[3-[3-(1,1,2,2-tetrafluoroethoxy)phenoxy]phenyl] [[2-fluoro-5-(trifluoro-methyl)phenyl]methyl]amino]-1,1,1-trifluoro-2-propanol;
(2R)-3-[[3-[3-(pentafluoroethyl)phenoxy]phenyl][[2-fluoro-5-(trifluoromethyl)-phenyl]methyl]amino]-1,1,1-trifluoro-2-propanol;
(2R)-3-[[3-(3,5-dimethylphenoxy)phenyl][[2-fluoro-5-(trifluoromethyl)phenyl]-methyl]amino]-1,1,1-trifluoro-2-propanol;
(2R)-3-[[3-(3-ethylphenoxy)phenyl][[2-fluoro-5-(trifluoromethyl)phenyl]methyl]-amino]-1,1,1-trifluoro-2-propanol;
(2R)-3-[[3-(3-t-butylphenoxy)phenyl][[2-fluoro-5-(trifluoromethyl)phenyl]methyl]-amino]-1,1,1-trifluoro-2-propanol;
(2R)-3-[[3-(3-methylphenoxy)phenyl][[2-fluoro-5-(trifluoromethyl)phenyl]methyl]-amino]-1,1,1-trifluoro-2-propanol;
(2R)-3-[[3-(5,6,7,8-tetrahydro-2-naphthoxy)phenyl][[2-fluoro-5-(trifluoromethyl)-phenyl]methyl]amino]-1,1,1-trifluoro-2-propanol;
(2R)-3-[[3-(phenoxy)phenyl][[2-fluoro-5-(trifluoromethyl) phenyl]methyl]amino]-1,1,1-trifluoro-2-propanol;
(2R)-3-[[3-[3-(N,N-dimethylamino,phenoxy]phenyl][[2-fluoro-5-(trifluoromethyl)-phenyl]methyl]amino]-1,1,1-trifluoro-2-propanol;
(2R)-3-[[[2-fluoro-5-(trifluoromethyl)phenyl]methyl][3-[[3-(trifluoromethoxy)-phenyl]methoxy]phenyl]amino]-1,1,1-trifluoro-3-propanol;
(2R)-3-[[[2-fluoro-5-(trifluoromethyl)phenyl]methyl][3-[[3-(trifluoromethyl)-phenyl]methoxy]phenyl]amino]-1,1,1-trifluoro-2-propanol;
(2R)-3-[[[2-fluoro-5-(trifluoromethyl)phenyl]methyl][3-[[3,5-dimethylphenyl]-methoxy]phenyl]amino]-1,1,1-trifluoro-2-propanol;
(2R)-3-[[[2-fluoro-5-(trifluoromethyl)phenyl]methyl][3-[[3-(trifluoromethylthio)-phenyl]methoxy]phenyl]amino]-1,1,1-trifluoro-2-propanol;
(2R)-3-[[[2-fluoro-5-(trifluoromethyl)phenyl]methyl][3-[[3,5-difluorophenyl]-methoxy]phenyl]amino]-1,1,1-trifluoro-2-propanol;
(2R)-3-[[[2-fluoro-5-(trifluoromethyl)phenyl]methyl][3-[cyclohexylmethoxyl-phenyl]amino]-1,1,1-trifluoro-2-propanol;
(2R)-3-[[3-(2-difluoromethoxy-4-pyridyloxy)phenyl][[2-fluoro-5-(trifluoromethyl)-phenyl]methyl]amino]-1,1,1-trifluoro-2-propanol;
(2R)-3-[[3-(2-trifluoromethyl-4-pyridyloxy)phenyl][[2-fluoro-5-(trifluoromethyl)-phenyl]methyl]amino]-1,1,1-trifluoro-2-propanol;
(2R)-3-[[3-(3-difluoromethoxyphenoxy)phenyl][[2-fluoro-5-(trifluoromethyl)-phenyl]methyl]amino]-1,1,1-trifluoro-2-propanol;
(2R)-3-[[[3-(3-trifluoromethylthio)phenoxy]phenyl][[2-fluoro-5-(trifluoromethyl)-phenyl]methyl]amino]-1,1,1-trifluoro-2-propanol;
(2R)-3-[[3-(4-chloro-3-trifluoromethylphenoxy)phenyl][[2-fluoro-5-(trifluoromethyl)phenyl]methyl]amino]-1,1,1-trifluoro-2-propanol;
(2R)-3-[[3-(3-trifluoromethoxyphenoxy)phenyl][[2-fluoro-4-(trifluoromethyl)-phenyl]methyl]amino]-1,1,1-trifluoro-2-propanol;
(2R)-3-[[3-(3-isopropylphenoxy)phenyl][[2-fluoro-4-(trifluoromethyl)phenyl]-methyl]amino]1-1,1,1-trifluoro-2-propanol;
(2R)-3-[[3-(3-cyclopropylphenoxy)phenyl][[2-flouro-4-(trifluoromethyl)phenyl]-methyl]amino]-1,1,1-trifluoro-2-propanol;
(2R)3-[[3-(3-(2-furyl)phenoxy)phenyl][[2-fluoro-4-(trifluoromethyl)phenyl]-methyl]amino]-1,1,1-trifluoro-2-propanol;
(2R)-3-[[3-(2,3-dichlorophenoxy)phenyl][[2-fluoro-4-(trifluoromethyl)phenyl]-methyl]amino]-1,1,1-trifluoro-2-propanol;
(2R)-3-[[3-(4-fluorophenoxy)phenyl][[2-fluoro-4-(trifluoromethyl)phenyl]-methyl]amino]-1,1,1-trifluoro-2-propanol;
(2R)-3-[[3-(4-methylphenoxy)phenyl][[2-fluoro-4-(trifluoromethyl)phenyl]-methyl]amino]-1,1,1-trifluoro-2-propanol;
(2R)-3-[[3-(2-fluoro-5-bromophenoxy)phenyl][[2-fluoro-4-(trifluoromethyl)-phenyl]methyl]amino]-1,1,1-trifluoro-2-propanol;
(2R)-3-[[3-(4-chloro-3-ethylphenoxy)phenyl][[2-fluoro-4-(trifluoromethyl)-phenyl]methyl]amino]-1,1,1-trifluoro-2-propanol;
(2R)-3-[[3-[3-(1,1,2,2-tetrafluoroethoxy)phenoxy]phenyl] [[2-fluoro-4-(trifluoromethyl)phenyl]methyl]amino]-1,1,1-trifluoro-2-propanol;
(2R)-3-[[3-[3-(pentafluoroethyl)phenoxy]phenyl][[2-fluoro-4-(trifluoromethyl)-phenyl]methyl]amino]-1,1,1-trifluoro-2-propanol;
(2R)-3-[[3-(3,5-dimethylphenoxy)phenyl][[2-fluoro-4-(trifluoromethyl)phenyl]-methyl]aminol-1,1,1-trifluoro-2-propanol;
(2R)-3-[[3-(3-ethylphenoxy)phenyl][[2-fluoro-4-(trifluoromethyl)phenyl]methyl]-amino]-1,1,1-trifluoro-2-propanol;
(2R)-3-[[3-(3-t-butylphenoxy)phenyl][[2-fluoro-4-(trifluoromethyl)phenyl]methyl]-amino]-1,1,1-trifluoro-2-propanol;
(2R)-3-[[3-(3-methylphenoxy)phenyl][[2-fluoro-4-(trifluoromethyl)phenyl]methyl]-amino]-1,1,1-trifluoro-2-propanol;
(2R)-3-[[3-(5,6,7,8-tetrahydro-2-naphthoxy)phenyl][[2-fluoro-4-(trifluoromethyl)-phenyl]methyl]amino]-1,1,1-trifluoro-2-propanol;
(2R)-3-[[3-(phenoxy)phenyl][[2-fluoro-4-(trifluoromethyl)phenyl]methyl]amino]-1,1,1-trifluoro-2-propanol;
(2R)-3-[[3-[3-(N,N-dimethylamino)phenoxy]phenyl][[2-fluoro-4-(trifluoromethyl)-phenyl]methyl]amino]-1,1,1-trifluoro-2-propanol;
(2R)-3-[[[2-fluoro-4-(trifluoromethyl)phenyl]methyl][3-[[3-(trifluoromethoxy)phenyl]methoxy]phenyl]amino]-1,1,1-trifluoro-2-propanol;
(3R)-3-[[[2-fluoro-4-(trifluoromethyl)phenyl]methyl][3-[[3-(trifluoromethyl)phenyl]methoxy]phenyl]amino]-1,1,1-trifluoro-2-propanol;
(2R)-3-[[[2-fluoro-4-(trifluoromethyl)phenyl]methyl][3-[[3,5-dimethylphenyl]-methoxy]phenyl]amino]-1,1,1-trifluoro-2-propanol;
(2R)-3-[[[2-fluoro-4-(trifluoromethyl)phenyl]methyl][3-[[3-(trifluoromethylthio)-phenyl]methoxy]phenyl]amino]-1,1,1-trifluoro-2-propenol;
(2R)-3-[[[2-fluoro-4-(trifluoromethyl)phenyl]methyl][3-[[3,5-difluorophenyl]-methoxy]phenyl]amino]-1,1,1-trifluoro-2-propanol;
(2R)-3-[[[2-fluoro-4-(trifluoromethy)phenyl]methyl][3-[cyclohexylmethoxy]-phenyl]amino]-1,1,1-trifluoro-2-propanol;
(2R)-3-[[3-(2-difluoromethoxy-4-pyridyloxy)phenyl][[2-fluoro-4-(trifluoromethyl)-phenyl]methyl]amino]-1,1,1-trifluoro-2-propanol;
(2R)-3-[[3-(2-trifluoromethyl-4-pyridyloxy)phenyl][[2-fluoro-4-(trifluoromethyl)-phenyl]methyl]amino]-1,1,1-trifluoro-2-propanol;
(2R)-3-[[3-(3-difluoromethoxyphenoxy)phenyl][[2-fluoro-4-(trifluoromethyl)-phenyl]methyl]amino]-1,1,1-trifluoro-2-propanol;
(2R)-3-[[[3-(3-trifluoromethylthio)phenoxy]phenyl][[2-fluoro-4-(trifluoromethyl)-phenyl]methyl]amino]-1.1,1-trifluoro-2-propanol; and
(2R)-3-[[3-(4-chloro-3-trifluoromethylphenoxy)phenyl][[2-fluoro-4-(trifluoromethyl)phenyl]methyl]amino]-1,1,1-trifluoro-2-propanol.

### SOLUBILITY-ENHANCED FORM OF THE DRUG

As used herein, a "solubility-enhanced" form of a drug is a form, which, when dissolved in an aqueous environment, provides a drug concentration in excess of the equilibrium concentration of the non-solubility-enhanced (e.g., crystalline) form of the drug. One type of solubility-enhanced form of CETPI is a solid amorphous dispersion of CETPI in a concentration-enhancing polymer. The composition, characteristics, and performance of said solid amorphous dispersions are described in commonly assigned US Provisional Patent Application No. 60/223,279, filed August 3, 2000, now US patent application 09/918,127 filed July 30, 2001, and US Patent application 10/066,091 filed February 1, 2002.

More than one concentration-enhancing polymer may be incorporated in the formulations of this invention.

Preferably, at least a major portion of the CETPI in the solid amorphous dispersion is amorphous. By "amorphous" is meant simply that the CETPI is in a non-crystalline state. As used herein, the term "a major portion" of the CETPI means that at least 60% of the CETPI in the solid amorphous dispersion is in the amorphous form, rather than the crystalline form. Preferably, the CETPI in the dispersion is substantially amorphous. As used herein, "substantially amorphous" means that the amount of the CETPI in crystalline form does not exceed about 25%. More preferably, the CETPI in the dispersion is "almost completely amorphous" meaning that the amount of CETPI in the crystalline form does not exceed about 10%. Amounts of crystalline CETPI may be measured by powder X-ray diffraction, Scanning Electron Microscope (SEM) analysis, differential scanning calorimetry (DSC), or any other standard quantitative measurement.

The solid amorphous dispersion may contain from about 1 to about 80 wt% CETPI, depending on the dose of the CETPI and the effectiveness of the concentration-enhancing polymer. Enhancement of aqueous CETPI concentrations and relative bioavailability are typically best at low CETPI levels, typically less than about 25 to 40 wt%. However, due to the practical limit of the dosage form size, higher CETPI levels are often preferred and in many cases perform well.

The amorphous CETPI can exist within the solid amorphous dispersion as a pure phase, as a solid solution of CETPI homogeneously distributed throughout the polymer or any combination of these states or those states that lie intermediate between them. The dispersion is preferably substantially homogeneous so that the amorphous CETPI is dispersed as homogeneously as possible throughout the polymer. As used herein, "substantially homogeneous" means that the fraction of CETPI that is present in relatively pure amorphous domains within the solid dispersion is relatively small, on the order of less than 20%, and preferably less than 10% of the total amount of CETPI.

While the dispersion may have some CETPI-rich domains, it is preferred that the dispersion itself have a single glass transition temperature (T_{g}) which demonstrates that the dispersion is substantially homogeneous. This contrasts with a simple physical mixture of pure amorphous CETPI particles and pure amorphous polymer particles which generally display two distinct T_{g}s, one that of the CETPI and one that of the polymer. T_{g} as used herein is the characteristic temperature where a glassy material, upon gradual heating, undergoes a relatively rapid (e.g., 10 to 100 seconds) physical change from a glass state to a rubber state. The T_{g} of an amorphous material such as a polymer, drug or dispersion can be measured by several techniques, including by a dynamic mechanical analyzer (DMA), a dilatometer, dielectric analyzer, and by a differential scanning calorimeter (DSC). The exact values measured by each technique can vary somewhat but usually fall within 10° to 30°C of each other. Regardless of the technique used, when an amorphous dispersion exhibits a single T_{g}, this indicates that the dispersion is substantially homogeneous. Dispersions of the present invention that are substantially homogeneous generally are more physically stable and have improved concentration-enhancing properties and, in turn improved bioavailability, relative to nonhomogeneous dispersions.

The solid amorphous dispersions comprising the CETPI and concentration-enhancing polymer provide enhanced concentration of the dissolved CETPI in *in vitro* dissolution tests. It has been determined that enhanced drug concentration in *in vitro* dissolution tests in Model Fasted Duodenal (MFD) solution or Phosphate Buffered Saline (PBS) is a good indicator of *in vivo* performance and bioavailability. An appropriate PBS solution is an aqueous solution comprising 20 mM sodium phosphate (Na₂HPO₄), 47 mM potassium phosphate (KH₂PO₄), 87 mM NaCl, and 0.2 mM KCI, adjusted to pH 6.5 with NaOH. An appropriate MFD solution is the same PBS solution wherein additionally is present 7.3 mM sodium taurocholic acid and 1.4 mM of 1-palmitoyl-2-oleyl-sn-glycero-3-phosphocholine. In particular, a dispersion of the present invention can be dissolution-tested by adding it to MFD or PBS solution and agitating to promote dissolution. Generally, the amount of dispersion added to the solution in such a test is an amount that, if all the drug in the dispersion dissolved, would produce a CETPI concentration that is at least about 10-fold and preferably at least 100-fold the equilibrium solubility of the CETPI alone in the test solution. To demonstrate even higher levels of dissolved CETPI concentration, addition of even larger amounts of the dispersion is desirable.

In one aspect, the solid amorphous dispersions of the present invention provide a Maximum Drug Concentration (MDC) that is at least about 10-fold the equilibrium concentration of a control composition consisting essentially of an equivalent quantity of CETPI but free from the polymer. In other words, if the equilibrium concentration provided by the control composition is 1 microgm/mL, then a dispersion of the present invention provides an MDC of at least about 10 microgm/mL. The control composition is conventionally the undispersed CETPI alone (e.g., typically, the crystalline CETPI alone in its most thermodynamically stable crystalline form). Preferably, the MDC of CETPI achieved with the dispersions of the present invention is at least about 1.25 fold-relative to a control composition. Often greater enhancement is observed, such as 10-fold, preferably at least 50-fold, more preferably at least about 200-fold and even more preferably at least about 500-fold, the equilibrium concentration of the control composition.

Alternatively, the solid amorphous dispersions of the present invention provide an MDC that is greater than the MDC of the control composition.

Alternatively, the solid amorphous dispersions of the present invention provide in an aqueous use environment a concentration versus time AUC, for any period of at least 90 minutes between the time of introduction into the use environment and about 270 minutes following introduction to the use environment, that is at least about 1.25-fold relative to a control composition. The AUC may be at least 5-fold, preferably at least about 25-fold, more preferably at least about 100-fold and even more preferably at least about 250-fold that of a control composition as described above. Such large enhancements in aqueous concentration versus time AUC values are surprising given the extremely low aqueous solubility and hydrophobicity of CETPIs.

A typical *in vitro* test to evaluate enhanced drug concentration in aqueous solution can be conducted by (1) adding with agitation a sufficient quantity of control composition, typically the CETPI alone, to the *in vitro* test medium, typically MFD or PBS solution, to achieve equilibrium concentration of the CETPI; (2) adding with agitation a sufficient quantity of test dispersion (e.g., the CETPI and polymer) in an equivalent test medium, such that if all the CETPI dissolved, the theoretical concentration of CETPI would exceed the equilibrium concentration of the CETPI by a factor of at least 10, and preferably a factor of at least 100; and (3) comparing the measured MDC and/or aqueous concentration versus time AUC of the test dispersion in the test medium with the equilibrium concentration, and/or the aqueous concentration versus time AUC of the control composition: In conducting such a dissolution test, the amount of test dispersion or control composition used is an amount such that if all of the CETPI dissolved the CETPI concentration would be at least 10-fold and preferably at least 100-fold that of the equilibrium concentration. If the CETPI concentration is greater than the equilibrium concentration in either PBS or MFD, then the dispersion is a solubility-enhanced form of CETPI.

The concentration of dissolved CETPI is typically measured as a function of time by sampling the test medium and plotting CETPI concentration in the test medium vs. time so that the MDC can be ascertained. The MDC is taken to be the maximum value of dissolved CETPI measured over the duration of the test. The aqueous concentration of the CETPI versus time AUC is calculated by integrating the concentration versus time curve over any 90-minute time period between the time of introduction of the dispersion into the aqueous use environment (time equals zero) and 270 minutes following introduction to the use environment (time equals 270 minutes). Typically, when the dispersion reaches its MDC rapidly, less than about 30 minutes, the time interval used to calculate AUC is from time equals zero to time equals 90 minutes. However, if the AUC over any 90-minute time period described above of a dispersion meets the criterion of this invention, then the dispersion is a part of this invention.

To avoid large CETPI particulates which would give an erroneous determination, the test solution is either filtered or centrifuged. "Dissolved CETPI" is typically taken as that material that either passes a 0.45 µm syringe filter or, alternatively, the material that remains in the supernatant following centrifugation. Filtration can be conducted using a 13 mm, 0.45 µm polyvinylidine difluoride syringe filter sold by Scientific Resources under the trademark TITAN®. Centrifugation is typically carried out in a polypropylene microcentrifuge tube by centrifuging at 13,000 G for 60 seconds. Other similar filtration or centrifugation methods can be employed and useful results obtained. For example, using other types of microfilters may yield values somewhat higher or lower (±10-40%) than that obtained with the filter specified above but will still allow identification of preferred dispersions. It is recognized that this definition of "dissolved CETPI" encompasses not only monomeric solvated CETPI molecules but also a wide range of species such as polymer/CETPI assemblies that have submicron dimensions such as CETPI aggregates, aggregates of mixtures of polymer and CETPI, micelles, polymeric micelles, colloidal particles or nanocrystals, polymer/CETPI complexes, and other such CETPI-containing species that are present in the filtrate or supernatant in the specified dissolution test.

Another type of solubility-enhanced form of CETPI is amorphous CETPI, which has a higher initial solubility in an aqueous environment of use than does crystalline CETPI. In this case, amorphous CETPI is not in a dispersion with a polymer. Amorphous CETPI may be prepared by a variety of methods known in the art, such as precipitation from an organic solvent. A preferred method is spray-drying from a solution of the crystalline form of CETPI an organic solvent, e.g. from acetone.

### CONCENTRATION-ENHANCING POLYMERS

Concentration-enhancing polymers suitable for use in the compositions of the present invention should be inert, in the sense that they do not chemically react with the CETP inhibitor in an adverse manner, are pharmaceutically acceptable, and have at least some solubility in aqueous solution at physiologically relevant pHs (e.g. 1-8). The polymer can be neutral or ionizable, and should have an aqueous-solubility of at least 0.1 mg/mL over at least a portion of the pH range of 1-8.

The polymer is a "concentration-enhancing polymer," meaning that it meets at least one, and more preferably both, of the following conditions. The first condition is that the concentration-enhancing polymer, when incorporated into a dispersion with a CETPI, increases the MDC of the CETPI in the environment of use relative to a control composition consisting of an equivalent amount of the CETPI but no polymer. That is, once the composition is introduced into an environment of use, the polymer increases the aqueous concentration of CETPI relative to the control composition. Preferably, the polymer increases the MDC of the CETPI in aqueous solution by at least 1.25-fold. Often greater enhancement is observed, such as 10-fold relative to a control composition, preferably by at least 50-fold, and more preferably by at least 200-fold. Even more preferably, the polymer increases the MDC of the CETPI in aqueous solution by at least 500-fold, and most preferably by at least 1000-fold. Such large enhancements may be necessary in order for some extremely water insoluble CETP inhibitors such as Drug A to achieve effective blood levels through oral dosing. The second condition is that the concentration-enhancing polymer increases the AUC of the CETPI in the environment of use relative to a control composition consisting of a CETPI but no polymer as described above. That is, in the environment of use, the composition comprising the CETPI and the concentration-enhancing polymer provides an area under the concentration versus time curve (AUC) for any period of 90 minutes between the time of introduction into the use environment and about 270 minutes following introduction to the use environment that is at least 1.25-fold that of a control composition comprising an equivalent quantity of CETPI but no polymer. The AUC provided by the composition may be at least 5-fold, preferably at least 25-fold , more preferably at least 100-fold, and even more preferably at least 250-fold that of the control composition.

Concentration-enhancing polymers suitable for use with the present invention may be cellulosic or non-cellulosic. The polymers may be neutral or ionizable in aqueous solution. Of these, ionizable and cellulosic polymers are preferred, with ionizable cellulosic polymers being more preferred.

A preferred class of polymers comprises polymers that are "amphiphilic" in nature, meaning that the polymer has hydrophobic and hydrophilic portions. The hydrophobic portion may comprise groups such as aliphatic or aromatic hydrocarbon groups. The hydrophilic portion may comprise either ionizable or non-ionizable groups that are capable of hydrogen bonding such as hydroxyls, carboxylic acids, esters, amines or amides.

Amphiphilic and/or ionizable polymers are preferred because it is believed that such polymers may tend to have relatively strong interactions with the CETP inhibitor and may promote the formation of the various types of polymer/drug assemblies in the use environment as described previously. In addition, the repulsion of the like charges of the ionized groups of such polymers may serve to limit the size of the polymer/drug assemblies to the nanometer or submicron scale. For example, while not wishing to be bound by a particular theory, such polymer/drug assemblies may comprise hydrophobic CETP inhibitor clusters surrounded by the polymer with the polymer's hydrophobic regions turned inward towards the CETP inhibitor and the hydrophilic regions of the polymer turned outward toward the aqueous environment. Alternatively, depending on the specific chemical nature of the CETP inhibitor, the ionized functional groups of the polymer may associate, for example, via ion pairing or hydrogen bonds, with ionic or polar groups of the CETP inhibitor. In the case of ionizable polymers, the hydrophilic regions of the polymer would include the ionized functional groups. Such polymer/drug assemblies in solution may well resemble charged polymeric micellar-like structures. In any case, regardless of the mechanism of action, such amphiphilic polymers, particularly ionizable cellulosic polymers, have been shown to improve the MDC and/or AUC of CETP inhibitor in aqueous solution relative to control compositions free from such polymers (described in commonly assigned US Provisional Patent Application No. 60/223,279, filed August 3, 2000, which is incorporated herein by reference).

Surprisingly, such amphiphilic polymers can greatly enhance the maximum concentration of CETP inhibitor obtained when CETP inhibitor is dosed to a use environment. In addition, such amphiphilic polymers interact with the CETP inhibitor to prevent the precipitation or crystallization of the CETP inhibitor from solution despite its concentration being substantially above its equilibrium concentration. In particular, when the preferred compositions are solid amorphous dispersions of the CETP inhibitor and the concentration-enhancing polymer, the compositions provide a greatly enhanced drug concentration, particularly when the dispersions are substantially homogeneous. The maximum drug concentration may be 10-fold and often more than 50-fold the equilibrium concentration of the crystalline CETP inhibitor. Such enhanced CETP inhibitor concentrations in turn lead to substantially enhanced relative bioavailability for the CETP inhibitor.

One class of polymers suitable for use with the present invention comprises neutral non-cellulosic polymers. Exemplary polymers include: vinyl polymers and copolymers having substituents of hydroxyl, alkylacyloxy, or cyclicamido; polyvinyl alcohols that have at least a portion of their repeat units in the unhydrolyzed (vinyl acetate) form; polyvinyl alcohol polyvinyl acetate copolymers; polyvinyl pyrrolidone; polyoxyethylene-polyoxypropylene copolymers, also known as poloxamers; and polyethylene polyvinyl alcohol copolymers.

Another class of polymers suitable for use with the present invention comprises ionizable non-cellulosic polymers. Exemplary polymers include: carboxylic acid-functionalized vinyl polymers, such as the carboxylic acid functionalized polymethacrylates and carboxylic acid functionalized polyacrylates such as the EUDRAGITS® manufactured by Rohm Tech Inc., of Malden, Massachusetts; amine-functionalized polyacrylates and polymethacrylates; proteins; and carboxylic acid functionalized starches such as starch glycolate.

Non-cellulosic polymers that are amphiphilic are copolymers of a relatively hydrophilic and a relatively hydrophobic monomer. Examples include acrylate and methacrylate copolymers, and polyoxyethylene-polyoxypropylene copolymers. Exemplary commercial grades of such copolymers include the EUDRAGITS, which are copolymers of methacrylates and acrylates, and the PLURONICS supplied by BASF, which are polyoxyethylene-polyoxypropylene copolymers.

A preferred class of polymers comprises ionizable and neutral cellulosic polymers with at least one ester- and/or ether-linked substituent in which the polymer has a degree of substitution of at least 0.1 for each substituent.

It should be noted that in the polymer nomenclature used herein, ether-linked substituents are recited prior to "cellulose" as the moiety attached to the ether group; for example, "ethylbenzoic acid cellulose" has ethoxybenzoic acid substituents. Analogously, ester-linked substituents are recited after "cellulose" as the carboxylate; for example, "cellulose phthalate" has one carboxylic acid of each phthalate moiety ester-linked to the polymer and the other carboxylic acid unreacted.

It should also be noted that a polymer name such as "cellulose acetate phthalate" (CAP) refers to any of the family of cellulosic polymers that have acetate and phthalate groups attached via ester linkages to a significant fraction of the cellulosic polymer's hydroxyl groups. Generally, the degree of substitution of each substituent group can range from 0.1 to 2.9 as long as the other criteria of the polymer are met. "Degree of substitution" refers to the average number of the three hydroxyls per saccharide repeat unit on the cellulose chain that have been substituted. For example, if all of the hydroxyls on the cellulose chain have been phthalate substituted, the phthalate degree of substitution is 3. Also included within each polymer family type are cellulosic polymers that have additional substituents added in relatively small amounts that do not substantially alter the performance of the polymer.

Amphiphilic cellulosics comprise polymers in which the parent cellulosic polymer has been substituted at any or all of the 3 hydroxyl groups present on each saccharide repeat unit with at least one relatively hydrophobic substituent. Hydrophobic substituents may be essentially any substituent that, if substituted to a high enough level or degree of substitution, can render the cellulosic polymer essentially aqueous insoluble. Examples of hydrophobic substituents include ether-linked alkyl groups such as methyl, ethyl, propyl, butyl, etc.; or ester-linked alkyl groups such as acetate, propionate, butyrate, etc.; and ether- and/or ester-linked aryl groups such as phenyl, benzoate, or phenylate. Hydrophilic regions of the polymer can be either those portions that are relatively unsubstituted, since the unsubstituted hydroxyls are themselves relatively hydrophilic, or those regions that are substituted with hydrophilic substituents. Hydrophilic substituents include ether- or ester-linked nonionizable groups such as the hydroxy alkyl substituents hydroxyethyl, hydroxypropyl, and the alkyl ether groups such as ethoxyethoxy or methoxyethoxy. Particularly preferred hydrophilic substituents are those that are ether- or ester-linked ionizable groups such as carboxylic acids, thiocarboxylic acids, substituted phenoxy groups, amines, phosphates or sulfonates.

One class of cellulosic polymers comprises neutral polymers, meaning that the polymers are substantially non-ionizable in aqueous solution. Such polymers contain non-ionizable substituents, which may be either ether-linked or ester-linked. Exemplary ether-linked non-ionizable substituents include: alkyl groups, such as methyl, ethyl, propyl, butyl, etc.; hydroxy alkyl groups such as hydroxymethyl, hydroxyethyl, hydroxypropyl, etc.; and aryl groups such as phenyl. Exemplary ester-linked non-ionizable substituents include: alkyl groups, such as acetate, propionate, butyrate, etc.; and aryl groups such as phenylate. However, when aryl groups are included, the polymer may need to include a sufficient amount of a hydrophilic substituent so that the polymer has at least some water solubility at any physiologically relevant pH of from 1 to 8.

Exemplary non-ionizable polymers that may be used as the polymer include: hydroxypropyl methyl cellulose acetate, hydroxypropyl methyl cellulose, hydroxypropyl cellulose, methyl cellulose, hydroxyethyl methyl cellulose, hydroxyethyl cellulose acetate, and hydroxyethyl ethyl cellulose.

A preferred set of neutral cellulosic polymers are those that are amphiphilic. Exemplary polymers include hydroxypropyl methyl cellulose and hydroxypropyl cellulose acetate, where cellulosic repeat units that have relatively high numbers of methyl or acetate substituents relative to the unsubstituted hydroxyl or hydroxypropyl substituents constitute hydrophobic regions relative to other repeat units on the polymer.

A preferred class of cellulosic polymers comprises polymers that are at least partially ionizable at physiologically relevant pH and include at least one ionizable substituent, which may be either ether-linked or ester-linked. Exemplary ether-linked ionizable substituents include: carboxylic acids, such as acetic acid, propionic acid, benzoic acid, salicylic acid, alkoxybenzoic acids such as ethoxybenzoic acid or propoxybenzoic acid, the various isomers of alkoxyphthalic acid such as ethoxyphthalic acid and ethoxyisophthalic acid, the various isomers of alkoxynicotinic acid such as ethoxynicotinic acid, and the various isomers of picolinic acid such as ethoxypicolinic acid, etc.; thiocarboxylic acids, such as thioacetic acid; substituted phenoxy groups, such as hydroxyphenoxy, etc.; amines, such as aminoethoxy, diethylaminoethoxy, trimethylaminoethoxy, etc.; phosphates, such as phosphate ethoxy; and sulfonates, such as sulphonate ethoxy. Exemplary ester linked ionizable substituents include: carboxylic acids, such as succinate, citrate, phthalate, terephthalate, isophthalate, trimellitate, and the various isomers of pyridinedicarboxylic acid, etc.; thiocarboxylic acids, such as thiosuccinate; substituted phenoxy groups, such as amino salicylic acid; amines, such as natural or synthetic amino acids, such as alanine or phenylalanine; phosphates, such as acetyl phosphate; and sulfonates, such as acetyl sulfonate. For aromatic-substituted polymers to also have the requisite aqueous solubility, it is also desirable that sufficient hydrophilic groups such as hydroxypropyl or carboxylic acid functional groups be attached to the polymer to render the polymer aqueous soluble at least at pH values where any ionizable groups are ionized. In some cases, the aromatic group may itself be ionizable, such as phthalate or trimellitate substituents.

Exemplary cellulosic polymers that are at least partially ionized at physiologically relevant pHs include: hydroxypropyl methyl cellulose acetate succinate, hydroxypropyl methyl cellulose succinate, hydroxypropyl cellulose acetate succinate, hydroxyethyl methyl cellulose succinate, hydroxyethyl cellulose acetate succinate, hydroxypropyl methyl cellulose phthalate, hydroxyethyl methyl cellulose acetate succinate, hydroxyethyl methyl cellulose acetate phthalate, carboxyethyl cellulose, carboxymethyl cellulose, carboxymethyl ethyl cellulose, cellulose acetate phthalate, methyl cellulose acetate phthalate, ethyl cellulose acetate phthalate, hydroxypropyl cellulose acetate phthalate, hydroxypropyl methyl cellulose acetate phthalate, hydroxypropyl cellulose acetate phthalate succinate, hydroxypropyl methyl cellulose acetate succinate phthalate, hydroxypropyl methyl cellulose succinate phthalate, cellulose propionate phthalate, hydroxypropyl cellulose butyrate phthalate, cellulose acetate trimellitate, methyl cellulose acetate trimellitate, ethyl cellulose acetate trimellitate, hydroxypropyl cellulose acetate trimellitate, hydroxypropyl methyl cellulose acetate trimellitate, hydroxypropyl cellulose acetate trimellitate succinate, cellulose propionate trimellitate, cellulose butyrate trimellitate, cellulose acetate terephthalate, cellulose acetate isophthalate, cellulose acetate pyridinedicarboxylate, salicylic acid cellulose acetate, hydroxypropyl salicylic acid cellulose acetate, ethylbenzoic acid cellulose acetate, hydroxypropyl ethylbenzoic acid cellulose acetate, ethyl phthalic acid cellulose acetate, ethyl nicotinic acid cellulose acetate, and ethyl picolinic acid cellulose acetate.

Exemplary cellulosic polymers that meet the definition of amphiphilic, having hydrophilic and hydrophobic regions include polymers such as cellulose acetate phthalate and cellulose acetate trimellitate where the cellulosic repeat units that have one or more acetate substituents are hydrophobic relative to those that have no acetate substituents or have one or more ionized phthalate or trimellitate substituents.

A particularly desirable subset of cellulosic ionizable polymers are those that possess both a carboxylic acid functional aromatic substituent and an alkylate substituent and thus are amphiphilic. Exemplary polymers include cellulose acetate phthalate, methyl cellulose acetate phthalate, ethyl cellulose acetate phthalate, hydroxypropyl cellulose acetate phthalate, hydroxylpropyl methyl cellulose phthalate, hydroxypropyl methyl cellulose acetate phthalate, hydroxypropyl cellulose acetate phthalate succinate, cellulose propionate phthalate, hydroxypropyl cellulose butyrate phthalate, cellulose acetate trimellitate, methyl cellulose acetate trimellitate, ethyl cellulose acetate trimellitate, hydroxypropyl cellulose acetate trimellitate, hydroxypropyl methyl cellulose acetate trimellitate, hydroxypropyl cellulose acetate trimellitate succinate, cellulose propionate trimellitate, cellulose butyrate trimellitate, cellulose acetate terephthalate, cellulose acetate isophthalate, cellulose acetate pyridinedicarboxylate, salicylic acid cellulose acetate, hydroxypropyl salicylic acid cellulose acetate, ethylbenzoic acid cellulose acetate, hydroxypropyl ethylbenzoic acid cellulose acetate, ethyl phthalic acid cellulose acetate, ethyl nicotinic acid cellulose acetate, and ethyl picolinic acid cellulose acetate.

Another particularly desirable subset of cellulosic ionizable polymers are those that possess a non-aromatic carboxylate substituent. Exemplary polymers include hydroxypropyl methyl cellulose acetate succinate, hydroxypropyl methyl cellulose succinate, hydroxypropyl cellulose acetate succinate, hydroxyethyl methyl cellulose acetate succinate, hydroxyethyl methyl cellulose succinate, hydroxyethyl cellulose acetate succinate, and carboxymethyl ethyl cellulose.

While, as listed above, a wide range of polymers may be used to form dispersions of CETP inhibitors, the inventors have found that relatively hydrophobic polymers have shown the best performance as demonstrated by high MDC and AUC values. In particular, cellulosic polymers that are aqueous insoluble in their nonionized state but are aqueous soluble in their ionized state perform particularly well. A particular subclass of such polymers are the so-called "enteric" polymers which include, for example, certain grades of hydroxypropyl methyl cellulose phthalate and cellulose acetate trimellitate. Dispersions formed from such polymers generally show very large enhancements, on the order of 50-fold to over 1000-fold, in the maximum drug concentration achieved in dissolution tests relative to that for a crystalline drug control. In addition, non-enteric grades of such polymers as well as closely related cellulosic polymers are expected to perform well due to the similarities in physical properties within the CETP inhibitor class.

Thus, especially preferred polymers are hydroxypropyl methyl cellulose acetate succinate (HPMCAS), hydroxypropyl methyl cellulose phthalate (HPMCP), cellulose acetate phthalate (CAP), cellulose acetate trimellitate (CAT), methyl cellulose acetate phthalate, hydroxypropyl cellulose acetate phthalate, cellulose acetate terephthalate, cellulose acetate isophthalate, and carboxymethyl ethyl cellulose. The most preferred ionizable cellulosic polymers are hydroxypropyl methyl cellulose acetate succinate, hydroxypropyl methyl cellulose phthalate, cellulose acetate phthalate, cellulose acetate trimellitate, and carboxymethyl ethyl cellulose.

One particularly effective polymer for forming dispersions of the present invention is carboxymethyl ethyl cellulose (CMEC). Dispersions made from CETP inhibitors and CMEC typically have high glass-transition temperatures at high relative humidities, due to the high glass-transition temperature of CMEC. As discussed below, such high T_{g}s result in solid amorphous dispersions with excellent physical stability. In addition, because all of the substituents on CMEC are attached to the cellulose backbone through ether linkages, CMEC has excellent chemical stability. Additionally, commercial grades of CMEC, such as that provided by Freund Industrial Company, Limited (Tokyo, Japan), are amphiphilic, leading to high degrees of concentration enhancement. Finally, hydrophobic CETP inhibitors often have a high solubility in CMEC allowing for formation of physically stable dispersions with high drug loadings.

A particularly effective CEP for use with CETPI is HPMCAS.

While specific polymers have been discussed as being suitable for use in the compositions of the present invention, blends of such polymers may also be suitable. Thus the term "polymer" is intended to include blends of polymers in addition to a single species of polymer.

To obtain the best performance, particularly upon storage for long times prior to use, it is preferred that the CETP inhibitor remain, to the extent possible, in the amorphous state. This is best achieved when the glass-transition temperature, T_{g}, of the amorphous CETPI material is substantially above the storage temperature of the composition. In particular, it is preferable that the T_{g} of the amorphous state of the CETPI be at least 40°C and preferably at least 60°C. However, this is not always the case. For example, the T_{g} of amorphous Drug A is about 30 °C . For those aspects of the invention in which the composition is a solid, substantially amorphous dispersion of a CETPI in the concentration-enhancing polymer, it is preferred that the concentration-enhancing polymer have a T_{g} of at least 40°C, preferably at least 70°C and more preferably greater than 100°C. Exemplary high T_{g} polymers include HPMCAS, HPMCP, CAP, CAT, CMEC and other cellulosics that have alkylate or aromatic substituents or both alkylate and aromatic substituents.

In addition, the preferred polymers listed above, that is amphiphilic cellulosic polymers, tend to have greater concentration-enhancing properties relative to the other polymers of the present invention. Generally those concentration-enhancing polymers that have ionizable substituents tend to perform best. *In vitro* tests of compositions with such polymers tend to have higher MDC and AUC values than compositions with other polymers of the invention.

### PREPARATION OF DISPERSIONS

Dispersions of a CETPI and concentration-enhancing polymer may be made according to any known process which results in at least a major portion (at least 60%) of the CETP inhibitor being in the amorphous state. Exemplary mechanical processes include milling and extrusion; melt processes include high temperature fusion, solvent modified fusion and melt-congeal processes; and solvent processes include non-solvent precipitation, spray coating and spray-drying. See, for example, U.S. Patent No. 5,456,923, U.S. Patent No. 5,939,099 and U.S. Patent No. 4,801,460 which describe formation of dispersions via extrusion processes; U.S. Patent No. 5,340,591 and U.S. Patent No. 4,673,564 which describe forming dispersions by milling processes; and U.S. Patent No. 5,684,040, U.S. Patent No. 4,894,235 and U.S. Patent No. 5,707,646 which describe the formation of dispersions via melt/congeal processes. Although the dispersions of the present invention may be made by any of these processes, the dispersions generally have their maximum bioavailability and stability when the CETP inhibitor is dispersed in the polymer such that it is substantially amorphous and substantially homogeneously distributed throughout the polymer.

In general, as the degree of homogeneity of the dispersion increases, the enhancement in the aqueous concentration of the CETP inhibitor and rotative bioavailability increases as well. Given the extremely low aqueous solubility and bioavailability of CETPIs in general it is highly preferred for the dispersions to be as homogeneous as possible to achieve therapeutically effective levels of the CETP inhibitor. Thus, most preferred are dispersions having a single glass transition temperature, which indicates a high degree of homogeneity.

In one embodiment, the solid amorphous dispersion of CETP inhibitor and concentration-enhancing polymer may be formed via a melt-congeal or extrusion process. Such processes are preferred when the CETP inhibitor has a relatively low melting point, typically less than about 200°C and preferably less than about 150°C. In such processes, a molten mixture comprising the CETP inhibitor and concentration-enhancing polymer is rapidly solidified to form a solid amorphous dispersion. By "rapidly solidify", it is meant solidify before the phases separate, in order to form a solid amorphous dispersion. By "molten mixture" is meant that the mixture comprising the CETP inhibitor and concentration-enhancing polymer is about 10°C or more above the melting point of the lowest melting point excipient in the composition. The CETP inhibitor can exist in the molten mixture as a pure phase, as a solution of CETP inhibitor homogeneously distributed throughout the molten mixture, or any combination of these states or those states that lie intermediate between them. The molten mixture is preferably substantially homogeneous so that the CETP inhibitor is dispersed as homogeneously as possible throughout the molten mixture.

As indicated above, the temperature of the molten mixture should be about 10°C or more above the melting point of the lowest melting point excipient in the composition. Generally, the processing temperature may vary from 50°C up to about 200°C or higher, depending on the melting point of the CETP inhibitor and polymer, which is a function of the polymer grade selected. However, the processing temperature should not be so high that degradation of the drug or polymer occurs. In some cases, the molten mixture should be formed under an inert atmosphere to prevent degradation of the drug and/or polymer at the processing temperature.

The molten mixture may also comprise an excipient that will reduce the melting temperature of the composition (either the drug and/or the polymer), allowing processing at lower temperature. These excipients can comprise up to 30 wt% of the molten mixture. For example, a plasticizer may be added to the composition to reduce the melting temperature of the polymer. Examples of plasticizers include water, triethylcitrate, triacetin, and dibutyl sebacate. Swelling agents for the polymer, such as acetone, methanol, and ethyl acetate, may also be added in low quantities to reduce the melting point of the composition. Examples of other excipients that can be added to the composition to reduce the processing temperature include low molecular weight polymers or oligomers, such as polyethylene glycol, polyvinylpyrrolidone, and poloxamers; fats and oils, including mono-, di-, and triglycerides; natural and synthetic waxes, such as carnauba wax, beeswax, microcrystalline wax, castor wax, and paraffin wax; long-chain alcohols, such as cetyl alcohol and stearyl alcohol; and long-chain fatty acids, such as stearic acid. When the excipient added is volatile, it may be removed from the solid amorphous dispersion after solidification.

Virtually any process may be used to form the molten mixture. One method involves melting the concentration-enhancing polymer in a vessel and then adding the CETP inhibitor to the molten polymer. Another method involves melting the CETP inhibitor in a vessel and then adding the concentration-enhancing polymer. In yet another method, a solid blend of the CETP inhibitor and concentration-enhancing polymer may be added to a vessel and the blend heated to form the molten mixture.

Once the molten mixture is formed, it may be mixed to ensure the CETP inhibitor is homogeneously distributed throughout the molten mixture. Such mixing may be done using mechanical means, such as overhead mixers, magnetically driven mixers and stir bars, planetary mixers, and homogenizers. Optionally, when the molten mixture is formed in a vessel, the contents of the vessel can be pumped out of the vessel and through an in-line or static mixer and then returned to the vessel. The amount of shear used to mix the molten mixture should be sufficiently high to ensure uniform distribution of the drug in the molten mixture. The molten mixture can be mixed from a few minutes to several hours, the mixing time being dependent on the viscosity of the mixture and the solubility of the drug and any optional excipients in the concentration-enhancing polymer.

An alternative method of preparing the molten mixture is to use two vessels, melting the CETP inhibitor in the first vessel and the concentration-enhancing polymer in a second vessel. The two melts are then pumped through an in-line static mixer or extruder to produce the molten mixture that is then rapidly solidified.

Alternatively, the molten mixture can be generated using an extruder, such as a single-screw or twin-screw extruder, both well known in the art. In such devices, a solid feed of the composition is fed to the extruder whereby the combination of heat and shear forces produce a uniformly mixed molten mixture, which can then be rapidly solidified to form the solid amorphous dispersion. The solid feed can be prepared using methods well known in the art for obtaining solid mixtures with high content uniformity. Alternatively, the extruder may be equipped with two feeders, allowing the CETP inhibitor to be fed to the extruder through one feeder and the polymer through the other. Other excipients to reduce the processing temperature as described above may be included in the solid feed, or in the case of liquid excipients, such as water, may be injected into the extruder using methods well-known in the art.

The extruder should be designed such that it produces a molten mixture with the drug uniformly distributed throughout the composition. The various zones in the extruder should be heated to appropriate temperatures to obtain the desired extrudate temperature as well as the desired degree of mixing or shear, using procedures well known in the art.

When the drug has a high solubility in the concentration-enhancing polymer, a lower amount of mechanical energy will be required to form the dispersion. In such cases, the processing temperature may be below the melting temperature of the undispersed CETP inhibitor but greater than the melting point of the polymer, since the CETP inhibitor will dissolve into the molten polymer.

When the CETP inhibitor has a low solubility in the polymer, a higher amount of mechanical energy may be required to form the dispersion. Here, the processing temperature may need to be above the melting point of the CETP inhibitor and the polymer. A high amount of mechanical energy may be needed to mix the molten CETP inhibitor with the polymer to form a dispersion. Typically, the lowest processing temperature and an extruder design that imparts the lowest amount of mechanical energy (e.g., shear) that produces a satisfactory dispersion (substantially amorphous and substantially homogeneous) is chosen in order to minimize the exposure of the CETP inhibitor to harsh conditions. Once the molten mixture of CETP inhibitor and concentration-enhancing polymer is formed, the mixture should be rapidly solidified to form the solid amorphous dispersion. By "rapidly solidified" is meant that the molten mixture is cooled sufficiently fast such that substantial phase separation of the drug and polymer does not occur. Typically, this means that the mixture should be solidified in less than about 10 minutes, preferably less than about 5 minutes, more preferably less than about 1 minute. If the mixture is not rapidly solidified, phase separation can occur, resulting in the formation of CETP inhibitor-rich phases and polymer-rich phases. Over time, the drug in the CETP inhibitor-rich phases can crystallize. Such compositions are therefore not substantially amorphous or substantially homogeneous and tend not to perform as well as those compositions that are rapidly solidified and are substantially amorphous and substantially homogeneous.

Another method for forming substantially amorphous and substantially homogeneous dispersions is by "solvent processing," which consists of dissolution of the CETPI and one or more polymers in a common solvent. "Common" here means that the solvent, which can be a mixture of compounds, will simultaneously dissolve the drug and the polymer(s). After both the CETP inhibitor and the polymer have been dissolved, the solvent is rapidly removed by evaporation or by mixing with a non-solvent. Exemplary processes are spray-drying, spray-coating (pan-coating, fluidized bed coating, etc.), and precipitation by rapid mixing of the polymer and drug solution with CO₂, water, or some other non-solvent. Preferably, removal of the solvent results in a solid dispersion which is substantially homogeneous. As described previously, in such substantially homogeneous dispersions, the CETP inhibitor is dispersed as homogeneously as possible throughout the polymer and can be thought of as a solid solution of CETP inhibitor dispersed in the polymer(s). When the resulting dispersion constitutes a solid solution of CETP inhibitor in polymer, the dispersion may be thermodynamically stable, meaning that the concentration of CETP inhibitor in the polymer is at or below its equilibrium value, or it may be considered a supersaturated solid solution where the CETP inhibitor concentration in the dispersion polymer(s) is above its equilibrium value.

The solvent may be removed through the process of spray-drying. The term spray-drying is used conventionally and broadly refers to processes involving breaking up liquid mixtures into small droplets (atomization) and rapidly removing solvent from the mixture in a container (spray-drying apparatus) where there is a strong driving force for evaporation of solvent from the droplets. The strong driving force for solvent evaporation is generally provided by maintaining the partial pressure of solvent in the spray-drying apparatus well below the vapor pressure of the solvent at the temperature of the drying droplets. This is accomplished by either (1) maintaining the pressure in the spray-drying apparatus at a partial vacuum (e.g., 0.01 to 0.50 atm); (2) mixing the liquid droplets with a warm drying gas; or (3) both. In addition, at least a portion of the heat required for evaporation of solvent may be provided by heating the spray solution.

Solvents suitable for spray-drying can be any organic compound in which the CETPI and polymer are mutually soluble. Preferably, the solvent is also volatile with a boiling point of 150°C or less. In addition, the solvent should have relatively low toxicity and be removed from the dispersion to a level that is acceptable according to The International Committee on Harmonization (ICH) guidelines. Removal of solvent to this level may require a processing step such as tray-drying subsequent to the spray-drying or spray-coating process. Preferred solvents include alcohols such as methanol, ethanol, n-propanol, iso-propanol, and butanol; ketones such as acetone, methyl ethyl ketone and methyl iso-butyl ketone; esters such as ethyl acetate and propylacetate; and various other solvents such as acetonitrile, methylene chloride, toluene, and 1,1,1-trichloroethane. Lower volatility solvents such as dimethyl acetamide or dimethylsulfoxide can also be used. Mixtures of solvents, such as 50% methanol and 50% acetone, can also be used, as can mixtures with water as long as the polymer and CETPI are sufficiently soluble to make the spray-drying process practicable. Generally, due to the hydrophobic nature of CETPIs, non-aqueous solvents are preferred meaning that the solvent comprises less than about 10 wt% water, and preferably less than 1 wt% water.

Generally, the temperature and flow rate of the drying gas is chosen so that the polymer/drug-solution droplets are dry enough by the time they reach the wall of the apparatus that they are essentially solid, and so that they form a fine powder and do not stick to the apparatus wall. The actual length of time to achieve this level of dryness depends on the size of the droplets. Droplet sizes generally range from 1 µm to 500 µm in diameter, with 5 µm to 100 µm being more typical. The large surface-to-volume ratio of the droplets and the large driving force for evaporation of solvent leads to actual drying times of a few seconds or less, and more typically less than 0.1 second. This rapid drying is often critical to the particles maintaining a uniform, homogeneous dispersion instead of separating into drug-rich and polymer-rich phases. As above, to get large enhancements in concentration and bioavailability it is often necessary to obtain as homogeneous of a dispersion as possible. Solidification times should be less than 100 seconds, preferably less than a few seconds, and more preferably less than 1 second. In general, to achieve this rapid solidification of the CETP inhibitor/polymer solution, it is preferred that the size of droplets formed during the spray-drying process are less than about 100 µm in diameter. The resultant solid particles thus formed are generally less than about 100 µm in diameter.

Following solidification, the solid powder typically stays in the spray-drying chamber for about 5 to 60 seconds, further evaporating solvent from the solid powder. The final solvent content of the solid dispersion as it exits the dryer should be low, since this reduces the mobility of CETP inhibitor molecules in the dispersion, thereby improving its stability. Generally, the solvent content of the dispersion as it leaves the spray-drying chamber should be less than 10 wt% and preferably less than 2 wt%. In some cases, it may be preferable to spray a solvent or a solution of a polymer or other excipient into the spray-drying chamber to form granules, so long as the dispersion is not adversely affected.

Spray-drying processes and spray-drying equipment are described generally in Perry's Chemical Engineers' Handbook, Sixth Edition (R. H. Perry, D. W. Green, J. O. Maloney, eds.) McGraw-Hill Book Co. 1984, pages 20-54 to 20-57. More details on spray-drying processes and equipment are reviewed by Marshall "Atomization and Spray-Drying," 50 Chem. Eng. Prog. Monogr. Series 2 (1954).

For CETPIs, spray-drying is a preferred method for forming a dispersion in a CEP. For CETPIs, a preferred CEP is HPMCAS and a preferred solvent for spray-drying is acetone.

The amount of concentration-enhancing polymer relative to the amount of CETP inhibitor present in the dispersions of the present invention depends on the CETP inhibitor and polymer and may vary widely from a CETP inhibitor-to-polymer weight ratio of from 0.01 to about 4 (e.g.. 1 wt% CETP inhibitor to 80 wt% CETP inhibitor). However,in most cases it is preferred that the CETP inhibitor-to-polymer ratio is greater than about 0.05 (4.8 wt% CETP inhibitor) and less than about 2.5 (71 wt% CETP inhibitor). Often the enhancement in CETP inhibitor concentration or relative bioavailability that is observed increases as the CETP inhibitor-to-polymer ratio decreases from a value of about 1 (50 wt% CETP inhibitor) to a value of about 0.11 (10 wt% CETP inhibitor). In some cases it has been found that the bioavailability of dispersions with a CETP-inhibitor-to-polymer ratio of about 0.33 (25 wt% CETP inhibitor) have higher bioavailability when dosed orally than dispersions with a CETP-inhibitor-to-polymer ratio of 0.11 (10 wt% CETP inhibitor). The CETP inhibitor:polymer ratio that yields optimum results varies from CETP inhibitor to CETP inhibitor and is best determined in *in vitro* dissolution tests and/or *in vivo* bioavailability tests.

For CETPIs, it is preferred that the CETPI-to-polymer ratio is greater than about 0.05 (4.8 wt% CETPI) and less than about 2.5 (71 wt% CETPI).

In addition, the amount of concentration-enhancing polymer that can be used in a dosage form is often limited by the total mass requirements of the dosage form. For example, when oral dosing to a human is desired, at low CETP inhibitor-to-polymer ratios the total mass of drug and polymer may be unacceptably large for delivery of the desired dose in a single tablet or capsule. Thus, it is often necessary to use CETP inhibitor-to-polymer ratios that are less than optimum in specific dosage forms to provide a sufficient CETP inhibitor dose in a dosage form that is small enough to be easily delivered to a use environment.

### MEANS TO PREVENT PRECIPITATION

The optional "means to prevent precipitation" refers to any material which is added to the composition which slows the precipitation of the CETPI in an environment of use, after the CETPI has supersaturated that environment of use relative to an equilibrium concentration of crystalline CETPI.

The concentration-enhancing polymers (CEPs) of the dispersions described above may serve two purposes. First, in a dispersion with a CETPI, they provide a CETPI form which is capable of supersaturating an environment of use. Second, they inhibit precipitation of the CETPI once the CETPI has supersaturated an environment of use. In other words, the concentration-enhancing polymers described and enumerated in detail above may also serve as precipitation-inhibiting polymers, once the CETPI/polymer dispersion supersaturates the environment of use with CETPI.

These same precipitation-inhibiting polymers may be physically mixed with a solubility-enhanced form of the CETPI. This solubility-enhanced form may be a dispersion of the CETPI in a concentration-enhancing polymer or low molecular weight material (MW<2000 daltons), which may then be blended with a precipitation-inhibiting polymer. The solubility-enhanced form may be substantially pure amorphous CETPI, which may be blended with a precipitation-inhibiting polymer. The solubility-enhanced form may comprise nanoparticles, i.e. solid drug particles of diameter less than approximately 900 nm, optionally stabilized by small quantities of surfactants or polymers, as described in US Patent 5,145,684 (incorporated herein by reference). The solubility-enhanced form may comprise adsorbates of the drug in a crosslinked polymer, as described in US Patent 5,225,192 (incorporated herein by reference). The nanoparticles or adsorbates of these disclosures are physically blended with a PIP. The solubility-enhanced form to be mixed with a PIP may also comprise forms of the type disclosed in commonly assigned copending US provisional Patent Application 60/300.314. filed June 22, 2001. The solubility enhanced form may also comprise high energy crystalline forms mixed with the precipitation inhibiting polymer, as described more fully in commonly assigned US patent application serial number 09/742,785, filed December 20, 2000.

The preferred precipitation-inhibiting polymers of this invention are the same as the preferred concentration-enhancing polymers of this invention.

More than one precipitation-inhibiting polymer may be incorporated in the formulations of this invention.

### CONTROLLED RELEASE MEANS

### MATRIX TABLETS

Compositions of this invention are administered in a controlled release dosage form. In one such dosage form, the composition of the CETP inhibitor and polymer is incorporated into an erodible polymeric matrix device. By an erodible matrix is meant aqueous-erodible or water-swellable or aqueous-soluble in the sense of being either erodible or swellable or dissolvable in pure water or requiring the presence of an acid or base to ionize the polymeric matrix sufficiently to cause erosion or dissolution. When contacted with the aqueous environment of use, the erodible polymeric matrix imbibes water and forms an aqueous-swollen gel or "matrix" that entraps the solubility-enhanced form, such as a solid amorphous dispersion of CETP inhibitor and polymer. The aqueous-swollen matrix gradually erodes, swells, disintegrates or dissolves in the environment of use, thereby controlling the release of the dispersion to the environment of use. Examples of such dosage forms are disclosed more fully in commonly assigned pending U.S. Patent Application Serial No. 09/495,059 filed January 31, 2000 which claimed the benefit of priority of provisional patent application Serial No. 60/119,400 filed February 10,1999.

The erodible polymeric matrix into which the solubility-enhanced form, such as a solid dispersion, is incorporated may generally be described as a set of excipients that are mixed with the dispersion following its formation that, when contacted with the aqueous environment of use imbibes water and forms a water-swollen gel or "matrix" that entraps the dispersion. Drug release may occur by a variety of mechanisms: the matrix may disintegrate or dissolve from around the dispersion particles or granules; or the drug may dissolve in the imbibed aqueous solution and diffuse from the tablet, beads or granules of the dosage form. A key ingredient of this water-swollen matrix is the water-swellable, erodible, or soluble polymer, which may generally be described as a osmopolymer, hydrogel or water-swellable polymer. Such polymers may be linear, branched, or crosslinked. They may be homopolymers or copolymers. Although they may be synthetic polymers derived from vinyl, acrylate, methacrylate, urethane, ester and oxide monomers, they are most preferably derivatives of naturally occurring polymers such as polysaccharides or proteins.

Such materials include naturally occurring polysaccharides such as chitin, chitosan, dextran and pullulan; gum agar, gum arabic, gum karaya, locust bean gum, gum tragacanth, carrageenans, gum ghatti, guar gum, xanthan gum and scleroglucan; starches such as dextrin and maltodextrin; hydrophilic colloids such as pectin; phosphatides such as lecithin; alginates such as ammonia alginate, sodium, potassium or calcium alginate, propylene glycol alginate; gelatin; collagen; and cellulosics.

A preferred class of cellulosics for the erodible matrix comprises aqueous-soluble and aqueous-erodible cellulosics such as ethyl cellulose (EC), methylethyl cellulose (MEC), carboxymethyl cellulose (CMC), CMEC, hydroxyethyl cellulose (HEC), hydroxypropyl cellulose (HPC), cellulose acetate (CA), cellulose propionate (CP), cellulose butyrate (CB), cellulose acetate butyrate (CAB), CAP, CAT, hydroxypropyl methyl cellulose (HPMC), HPMCP, HPMCAS, hydroxypropyl methyl cellulose acetate trimellitate (HPMCAT), and ethylhydroxy ethylcellulose (EHEC). A particularly preferred class of such cellulosics comprises various grades of low viscosity (MW less than or equal to 50,000 daltons) and high viscosity (MW greater than 50,000 daltons) HPMC. Commercially available low viscosity HPMC polymers include the Dow METHOCEL series E5, E15LV, E50LV and K100LY, while high viscosity HPMC polymers include E4MCR, E10MCR, K4M, K15M and K100M; especially preferred in this group are the METHOCEL (Trademark) K series. Other commercially available types of HPMC include the Shin Etsu METOLOSE 90SH series.

Although the primary role of the erodible matrix material is to control the rate of release of drug to the environment of use, the inventors have found that the choice of matrix material can have a large effect on the maximum drug concentration attained by the controlled-release dosage form as well as the maintenance of a high drug concentration. The proper choice of polymer in turn affects the bioavailability of the drug. It has been found that water-soluble cellulosics such as certain grades of methyl cellulose (MC) or HPMC, when used as the primary rate-controlling matrix material, can result in higher maximum drug concentrations *in vitro* relative to other conventional matrix polymers such as polyoxamers (e.g., PEO or PEG) or carboxylic acid polymers such as CMC or calcium CMC or polyacrylic acids such as Carbopol. Thus, an especially preferred embodiment of the invention comprises a solid substantially amorphous dispersion of drug in a cellulosic polymer incorporated into controlled-release beads, granules, or tablets wherein the matrix polymer comprises an aqueous-soluble cellulosic. Exemplary of such cellulosics are MC, HEC, HPC, hydroxyethyl methyl cellulose, HPMC, and other closely related water-soluble polymers. Preferably, the matrix material comprises MC or HPMC.

Other materials useful as the erodible matrix material include, but are not limited to, pullulan, polyvinyl pyrrolidone, polyvinyl alcohol, polyvinyl acetate, glycerol fatty acid esters, polyacrylamide, polyacrylic acid, copolymers of ethacrylic acid or methacrylic acid (EUDRAGIT®, Rohm America, Inc., Piscataway, New Jersey) and other acrylic acid derivatives such as homopolymers and copolymers of butylmethacrylate, methylmethacrylate, ethylmethacrylate, ethylacrylate, (2-dimethylaminoethyl)methacrylate, and (trimethylaminoethyl) methacrylate chloride.

The erodible matrix polymer may contain concentration-enhancing dispersion polymers and precipitation-inhibiting polymers of the type discussed above. In addition, the erodible matrix polymer may contain a wide variety of the same types of additives and excipients known in the pharmaceutical arts and discussed above, including osmopolymers, osmagens, solubllity-enhancing or -retarding agents and excipients that promote stability or processing of the dosage form.

Alternatively, the compositions of the present invention may be administered by or incorporated into a non-erodible matrix device.

### OSMOTIC TABLETS

Alternatively, the compositions of the invention may be delivered using a coated osmotic controlled release dosage form. This dosage form has two components: (a) the core which contains an osmotic agent and the solubtiliy-enhanced form of the CETPI, such as a solid amorphous dispersion of CETP inhibitor and concentration-enhancing polymer, or amorphous CETPI blended with a PIP; and (b) a non-dissolving and non-eroding coating surrounding the core, the coating controlling the influx of water to the core from an aqueous environment of use so as to cause drug release by extrusion of some or all of the core to the environment of use. The osmotic agent contained in the core of this device may be an aqueous-swellable hydrophilic polymer, osmogen, or osmagent. The coating is preferably polymeric, aqueous-permeable, and has at least one delivery port. Examples of such dosage forms are disclosed more fully in commonly assigned pending U.S. Patent Application Serial No. 09/495,061 filed January 31, 2000 which claimed the benefit of priority of provisional Patent Application Serial No. 60/119,406 filed February 10, 1999.

### OSMOTIC TABLETS - THE OSMOTIC AGENT

In addition to the solubility-enhanced form, such as solid amorphous drug dispersions, the core of the osmotic tablet of the present invention includes an "osmotic agent." By "osmotic agent" is meant any agent which creates a driving force for transport of water from the environment of use into the core of the device. Exemplary osmotic agents are water-swellable hydrophilic polymers, and osmogens (or osmagens). Thus, the core may include water-swellable hydrophilic polymers, both ionic and nonionic, often referred to as "osmopolymers" and "hydrogels." The amount of water-swellable hydrophilic polymers present in the core may range from about 5 to about 80 wt%, preferably 10 to 50 wt%. Exemplary materials include hydrophilic vinyl and acrylic polymers, polysaccharides such as calcium alginate, polyethylene oxide (PEO), polyethylene glycol (PEG), polypropylene glycol (PPG), poly(2-hydroxyethyl methacrylate), poly(acrylic) acid, poly(methacrylic) acid, polyvinylpyrrolidone (PVP) and crosslinked PVP, polyvinyl alcohol (PVA), PVA/PVP copolymers and PVA/PVP copolymers with hydrophobic monomers such as methyl methacrylate, vinyl acetate, and the like, hydrophilic polyurethanes containing large PEO blocks, sodium croscarmellose, carrageenan, hydroxyethyl cellulose (HEC), hydroxypropyl cellulose (HPC), hydroxypropyl methyl cellulose (HPMC), carboxymethyl cellulose (CMC) and carboxyethyl cellulose (CEC), sodium alginate, polycarbophil, gelatin, xanthan gum, and sodium starch glycolate. Other materials include hydrogels comprising interpenetrating networks of polymers which may be formed by addition or by condensation polymerization, the components of which may comprise hydrophilic and hydrophobic monomers such as those just mentioned. Preferred polymers for use as the water-swellable hydrophilic polymers include PEO, PEG, PVP, sodium croscarmellose, HPMC, sodium starch glycolate, polyacrylic acid and crosslinked versions or mixtures thereof. In one embodiment of the invention the osmotic agent and the concentration-enhancing polymer can comprise the same polymeric material.

The core may also include an osmogen or osmagent. The amount of osmogen present in the core may range from about 2 to about 70 wt%, preferably 10 to 50 wt%. Typical classes of suitable osmogens are water-soluble organic acids, salts and sugars that are capable of imbibing water to thereby effect an osmotic pressure gradient across the barrier of the surrounding coating. Typical useful osmogens include magnesium sulfate, magnesium chloride, calcium chloride, sodium chloride, lithium chloride, potassium sulfate, sodium carbonate, sodium sulfite, lithium sulfate, potassium chloride, sodium sulfate, mannitol, xylitol, urea, sorbitol, inositol, raffinose, sucrose, glucose, fructose, lactose, citric acid, succinic acid, tartaric acid, and mixtures thereof. Particularly preferred osmogens are glucose, lactose, sucrose, mannitol, xylitol and sodium chloride.

### OSMOTIC TABLETS - OTHER CORE COMPONENTS

The core may include a wide variety of additives and excipients that enhance drug solubility or that promote stability, tableting or processing. Such additives and excipients include tableting aids, surfactants, water-soluble polymers, pH modifiers, fillers, binders, pigments, disintegrants, antioxidants, lubricants and flavorants. Exemplary of such components are microcrystalline cellulose; metallic salts of acids such as aluminum stearate, calcium stearate, magnesium stearate, sodium stearate, and zinc stearate; fatty acids, hydrocarbons and fatty alcohols such as stearic acid, palmitic acid, liquid paraffin, stearyl alcohol, and palmitol; fatty acid esters such as glyceryl (mono- and di-) stearates, triglycerides, glyceryl (palmiticstearic) ester, sorbitan monostearate, saccharose monostearate, saccharose monopalmitate, and sodium stearyl fumarate; alkyl sulfates such as sodium lauryl sulfate and magnesium lauryl sulfate; polymers such as polyethylene glycols, polyoxethylene glycols, and polytetrafluoroethylene; and inorganic materials such as talc and dicalcium phosphate; sugars such as lactose and xylitol; and sodium starch glycolate. Examples of disintegrants are sodium starch glycolate (e.g., Explotab^{™}), microcrystalline cellulose (e.g., Avicel^{™}), microcrystalline silicified cellulose (e.g., ProSolv^{™}) and croscarmellose sodium (e.g., Ac-Di-Sol^{™}).

The core may also include solubility-enhancing agents that promote the water solubility of the drug, present in an amount ranging from about 5 to about 50 wt%. Examples of suitable solubility-enhancing agents include surfactants; pH control agents such as buffers, organic acids and organic acid salts and organic and inorganic bases; glycerides; partial glycerides; glyceride derivatives; polyoxyethylene and polyoxypropylene ethers and their copolymers; sorbitan esters; polyoxyethylene sorbitan esters; carbonate salts; alkyl sulfonates; and cyclodextrins.

When the solubility-enhanced form is a solid amorphous dispersion formed by a solvent process, such solubility-enhancing and other additives may be added directly to the spray-drying solution when forming the CETPI/CEP dispersion such that the additive is dissolved or suspended in the solution as a slurry. Alternatively, such additives may be added following the spray-drying process to aid in forming the final dosage form.

### OSMOTIC TABLETS - THE COATING

The essential constraints on the coating for the osmotic tablet embodiment are that it be water-permeable, have at least one port for the delivery of drug, and be non-dissolving and non-eroding during release of the drug formulation, such that drug is substantially entirely delivered through the delivery port(s) or pores as opposed to delivery primarily via permeation through the coating material itself. By "delivery port" is meant any passageway, opening or pore whether made mechanically, by laser drilling, by pore formation either during the coating process or *in situ* during use or by rupture during use. The coating should be present in an amount ranging from about 5 to 30 wt%, preferably 10 to 20 wt% relative to the core weight.

A preferred form of coating is a semipermeable polymeric membrane that has the port(s) formed therein either prior to or during use. Thickness of such a polymeric membrane may vary between about 20 and 800 µm, and is preferably in the range of 100 to 500 µm. The delivery port(s) should generally range in size from 0.1 to 3000 µm or greater, preferably on the order of 50 to 3000 µm in diameter. Such port(s) may be formed post-coating by mechanical or laser drilling or may be formed *in situ* by rupture of the coatings; such rupture may be controlled by intentionally incorporating a relatively small weak portion into the coating. Delivery ports may also be formed *in situ* by erosion of a plug of water-soluble material or by rupture of a thinner portion of the coating over an indentation in the core. In addition, delivery ports may be formed during coating, as in the case of asymmetric membrane coatings of the type disclosed in U.S. Patent Nos. 5,612,059 and 5,698,220, the disclosures of which are incorporated by reference.

When the delivery port is formed *in situ* by rupture of the coating, a particularly preferred embodiment is a collection of beads that may be of essentially identical or of a variable composition. Drug is primarily released from such beads following rupture of the coating and, following rupture, such release may be gradual or relatively sudden. When the collection of beads has a variable composition, the composition may be chosen such that the beads rupture at various times following administration, resulting in the overall release of drug being sustained for a desired duration.

Coatings may be dense, microporous or "asymmetric," having a dense region supported by a thick porous region such as those disclosed in U.S. Patent Nos. 5,612,059 and 5,698,220. When the coating is dense the coating is composed of a water-permeable material. When the coating is porous, it may be composed of either a water-permeable or a water-impermeable material. When the coating is composed of a porous water-impermeable material, water permeates through the pores of the coating as either a liquid or a vapor.

Examples of osmotic devices that utilize such dense coatings include U.S. Patent Nos. 3,995,631 and 3,845,770. Such dense coatings are permeable to the external fluid such as water and may be composed of any of the materials mentioned in these patents as well as other water-permeable polymers known in the art.

The membranes may also be porous as disclosed in U.S. Patent Nos. 5,654,005 and 5,458,887 or even be formed from water-resistant polymers. U.S. Patent No. 5,120,548 describes another suitable process for forming coatings from a mixture of a water-insoluble polymer and a leachable water-soluble additive. The porous membranes may also be formed by the addition of pore-formers as disclosed in U.S. Patent No. 4,612,008.

In addition, vapor-permeable coatings may even be formed from extremely hydrophobic materials such as polyethylene or polyvinylidene difluoride that, when dense, are essentially water-impermeable, as long as such coatings are porous.

Materials useful in forming the coating include various grades of acrylics, vinyls, ethers, polyamides, polyesters and cellulosic derivatives that are water-permeable and water-insoluble at physiologically relevant pHs, or are susceptible to being rendered water-insoluble by chemical alteration such as by crosslinking.

Specific examples of suitable polymers (or crosslinked versions) useful in forming the coating include plasticized, unplasticized and reinforced cellulose acetate (CA), cellulose diacetate, cellulose triacetate, CA propionate, cellulose nitrate, cellulose acetate butyrate (CAB), CA ethyl carbamate, CAP, CA methyl carbamate, CA succinate, cellulose acetate trimellitate (CAT), CA dimethylaminoacetate, CA ethyl carbonate, CA chloroacetate, CA ethyl oxalate, CA methyl sulfonate, CA butyl sulfonate, CA p-toluene sulfonate, agar acetate, amylose triacetate, beta glucan acetate, beta glucan triacetate, acetaldehyde dimethyl acetate, triacetate of locust bean gum, hydroxlated ethylenevinylacetate, EC, PEG, PPG, PEG/PPG copolymers, PVP, HEC, HPC, CMC, CMEC, HPMC, HPMCP, HPMCAS, HPMCAT, poly(acrylic) acids and esters and poly-(methacrylic) adds and esters and copolymers thereof, starch, dextran, dextrin, chitosan, collagen, gelatin, polyalkenes, polyethers, polysulfones, polyethersulfones, polystyrenes, polyvinyl halides, polyvinyl esters and ethers, natural waxes and synthetic waxes.

A preferred coating composition comprises a cellulosic polymer, in particular cellulose ethers, cellulose esters and cellulose ester-ethers, i.e., cellulosic derivatives having a mixture of ester and ether substituents.

Another preferred class of coating materials are poly(acrylic) acids and esters, poly(methacrylic) adds and esters, and copolymers thereof.

A more preferred coating composition comprises cellulose acetate. An even more preferred coating comprises a cellulosic polymer and PEG. A most preferred coating comprises cellulose acetate and PEG.

Coating is conducted in conventional fashion, typically by dissolving the coating material in a solvent and then coating by dipping, spray-coating or preferably by pan-coating. A preferred coating solution contains 5 to 15 wt% polymer. Typical solvents useful with the cellulosic polymers mentioned above include acetone, methyl acetate, ethyl acetate, isopropyl acetate, n-butyl acetate, methyl isobutyl ketone, methyl propyl ketone, ethylene glycol monoethyl ether, ethylene glycol monoethyl acetate, methylene dichloride, ethylene dichloride, propylene dichloride, nitroethane, nitropropane, tetrachloroethane, 1,4-dioxane, tetrahydrofuran, diglyme, and mixtures thereof. Pore-formers and non-solvents (such as water, glycerol and ethanol) or plasticizers (such as diethyl phthalate) may also be added in any amount as long as the polymer remains soluble at the spray temperature. Pore-formers and their use in fabricating coatings are described in U.S. Patent No. 5,612,059.

Coatings may also be hydrophobic microporous layers wherein the pores are substantially filled with a gas and are not wetted by the aqueous medium but are permeable to water vapor, as disclosed in U.S. Patent No. 5,798,119. Such hydrophobic but water-vapor permeable coatings are typically composed of hydrophobic polymers such as polyalkenes, polyacrylic acid derivatives, polyethers, polysulfones, polyethersulfones, polystyrenes, polyvinyl halides, polyvinyl esters and ethers, natural waxes and synthetic waxes. Especially preferred hydrophobic microporous coating materials include polystyrene, polysulfones, polyethersulfones, polyethylene, polypropylene, polyvinyl chloride, polyvinylidene fluoride and polytetrafluoroethylene. Such hydrophobic coatings can be made by known phase inversion methods using any of vapor-quench, liquid quench, thermal processes, leaching soluble material from the coating or by sintering coating particles. In thermal processes, a solution of polymer in a latent solvent is brought to liquid-liquid phase separation in a cooling step. When evaporation of the solvent is not prevented, the resulting membrane will typically be porous. Such coating processes may be conducted by the processes disclosed in U.S. Patent Nos. 4,247,498; 4,490,431 and 4,744,906.

Another embodiment of sustained release osmotic dosage forms of this invention comprises an osmotic drug-containing tablet which is surrounded by an asymmetric membrane, where said asymmetric membrane possesses one or more thin dense regions in addition to less dense porous regions. This type of membrane, similar to those used in the reverse-osmosis industry, generally allows higher osmotic fluxes of water than can be obtained with a dense membrane. When applied to a drug formulation, e.g. a tablet, such an asymmetric membrane allows high drug fluxes and well-controlled sustained drug release. This asymmetric membrane comprises a semipermeable polymeric material, that is, a material which is permeable to water, and substantially impermeable to salts and organic solutes such as drugs.

Materials useful for forming such an asymmetric semipermeable membrane include polyamides, polyesters and cellulose derivatives. Preferred are cellulose ethers and esters. Especially preferred are CA, CAB and EC. Especially useful materials include those which spontaneously form one or more exit passageways, either during manufacturing or when placed in an environment of use. These preferred materials comprise porous polymers, the pores of which are formed by phase inversion during manufacturing, as described above, or by dissolution of a water-soluble component present in the membrane.

The asymmetric membrane is formed by a phase-inversion process. The coating polymer, e.g., EC or CA, is dissolved in a mixed solvent system comprising a mixture of solvents (e.g., acetone) and non-solvents (e.g., water) for the polymer. The components of the mixed solvent are chosen such that the solvent (e.g. acetone) is more volatile than the non-solvent (e.g. water). When a tablet is contacted with such a solution and dried, the solvent component of the solvent mixture evaporates more quickly than the non-solvent. This change in solvent composition during drying causes the solution to separate into two phases so that, when solid, the polymer on the tablet is a porous solid with a thin dense outer region. This outer region possesses multiple pores through which drug can be delivered as a solution or a suspension of drug particles, which particles may be crystalline, amorphous or a drug/polymer dispersion.

In a preferred embodiment of an asymmetric membrane-coated tablet, the polymer/solvent/non-solvent mixture is sprayed onto a bed of tablets in a tablet-coating apparatus such as a Freund HCT-60 tablet coater. In this process, the tablet is coated with thick porous regions, and with a final outer thin dense region.

In the environment of use such as the GI tract, water is imbibed through the semipermeable asymmetric membrane into the tablet core. As soluble material in the tablet core dissolves, an osmotic pressure gradient across the membrane builds. When the hydrostatic pressure within the membrane enclosed core exceeds the pressure of the environment of use, the drug-containing solution is "pumped" out of the dosage form via the delivery port(s) through the semipermeable membrane. In addition the hydrostatic pressure may cause the formation of pores or even large delivery port(s) by rupture of a portion of the coating. The relatively constant osmotic pressure difference across the membrane results in a constant, well-controlled delivery of drug to the use environment. A portion of the drug dissolved in the tablet also exits via diffusion.

In this asymmetric-membrane-coated tablet embodiment, the drug may be incorporated into the dispersion in its neutral form or as a salt. It is often desirable to include one or more solubilizing excipients, such as ascorbic acid, erythorbic acid, citric acid, tartaric acid, glutamic acid, aspartic acid, glycerides, partial glycerides, glyceride derivatives, PEG, PEG esters, PPG esters, polyhydric alcohol esters, polyoxyethylene ethers, sorbitan esters, polyoxyethylene sorbitan esters, saccharide esters, phospholipids, polyethylene oxide-polypropylene oxide block co-polymers. Most preferred are the solubilizing excipients ascorbic acid, aspartic acid, citric acid, tartaric acid, glyceryl monocaprylate, glyceryl monostearate, glycerol monolaurate, and C8-C10 partial glycerides.

### OSMOTIC TABLETS - USE AND FABRICATION

In use, the core imbibes water through the coating from the environment of use such as the GI tract so as to increase the pressure within the core. The pressure difference between the core and the device's exterior drives the delivery of the core's contents. Because the coating remains intact, the drug formulation is extruded out of the core through the delivery port(s) into the environment of use, either primarily as a solution of drug or as a suspension of drug; when delivered as a suspension, the drug formulation subsequently dissolves in the GI tract.

### BILAYER OSMOTIC TABLETS

A preferred embodiment of osmotic delivery devices consists of a drug layer containing the solubility-enhanced form of the CETPI, such as a solid amorphous drug/polymer dispersion, and a sweller layer that comprises a water-swellable polymer, with a coating surrounding the drug layer and sweller layer. Each layer may contain other excipients such as tableting aids, osmagents, surfactants, water-soluble polymers and water-swellable polymers.

Such osmotic delivery devices may be fabricated in various geometries including bilayer, wherein the core comprises a drug layer and a sweller layer adjacent to each other; trilayer, wherein the core comprises a sweller layer "sandwiched" between two drug layers; and concentric, wherein the core comprises a central sweller composition surrounded by the drug layer.

The coating of such a tablet comprises a membrane permeable to water but substantially impermeable to drug and excipients contained within. The coating contains one or more exit passageways or ports in communication with the drug-containing layer(s) for delivering the drug composition. The drug-containing layer(s) of the core contains the drug composition (including optional osmagents and hydrophilic water-soluble polymers), while the sweller layer consists of an expandable hydrogel, with or without additional osmotic agents.

When placed in an aqueous medium, the tablet imbibes water through the membrane, causing the composition to form a dispensable aqueous composition, and causing the hydrogel layer to expand and push against the drug-containing composition, forcing the composition out of the exit passageway. The composition can swell, aiding in forcing the drug out of the passageway. Drug can be delivered from this type of delivery system either dissolved or dispersed in the composition that is expelled from the exit passageway.

The rate of drug delivery is controlled by such factors as the permeability and thickness of the coating, the osmotic pressure of the drug-containing layer, the degree of hydrophilicity of the hydrogel layer, and the surface area of the device. Those skilled in the art will appreciate that increasing the thickness of the coating will reduce the release rate, while any of the following will increase the release rate: increasing the permeability of the coating; increasing the hydrophilicity of the hydrogel layer, increasing the osmotic pressure of the drug-containing layer; or increasing the device's surface area.

Exemplary materials useful in forming the drug -containing composition, in addition to the solubility-enhanced form of the drug itself (such as a solid amorphous dispersion), include HPMC, PEO and PVP and other pharmaceutically acceptable carriers. In addition, osmagents such as sugars or salts, especially sucrose, lactose, xylitol, mannitol, or sodium chloride, may be added. Materials which are useful for forming the hydrogel layer include sodium CMC, PEO, poly (acrylic acid), sodium (polyacrylate), sodium croscarmellose, sodium starch glycolate, PVP, crosslinked PVP, and other high molecular weight hydrophilic materials. Particularly useful are PEO polymers having an average molecular weight from about 5,000,000 to about 7,500,000 daltons.

In the case of a bilayer geometry, the delivery port(s) or exit passageway(s) may be located on the side of the tablet containing the drug composition or may be on both sides of the tablet or even on the edge of the tablet so as to connect both the drug layer and the sweller layer with the exterior of the device. The exit passageway(s) may be produced by mechanical means or by laser drilling, or by creating a difficult-to-coat region on the tablet by use of special tooling during tablet compression or by other means. The rate of drug delivery from the device may be optimized so as to provide a method of delivering drug to a mammal for optimum therapeutic effect.

### MONOLAYER OSMOTIC TABLETS

Osmotic systems can also be made with a homogeneous core surrounded by a semipermeable membrane coating, as in U.S. patent 3,845,770. The solubility-enhance form of the drug, such as a solid amorphous dispersion, can be incorporated into a tablet core that also contains other excipients that provide sufficient osmotic driving force and optionally solubilizing excipients such as acids or surfactant-type compounds. A semipermeable membrane coating can be applied via conventional tablet-coating techniques such as using a pan coater. A drug delivery passageway can then be formed in this coating by drilling a hole in the coating, either by use of a laser or mechanical means. Alternatively, the passageway may be formed by rupturing a portion of the coating or by creating a region on the tablet that is difficult to coat, as described above.

A particularly useful embodiment of a monolayer osmotic tablet is an osmotic tablet, which comprises:(a) a single-layer compressed core comprising: (i) the solubility-enhanced form of the CETPI, (ii) a hydroxyethylcellulose having a weight-average, molecular weight from about 300,000 to about 1,500,000, and (iii) an osmagent, wherein the hydroxyethylcellulose is present in the core from about 2.0% to about 35% by weight (preferably from about 3% to about 20%, more preferably from about 3% to about 15%, most preferably from about 3% to about 10%) and the osmagent is present from about 15% to about 70% by weight (preferably from about 30% to about 65%, more preferably from about 40% to about 60%, most preferably from about 40% to about 55%); (b) a water permeable layer surrounding the core; and at least one passageway within the layer (b) for delivering the drug to a fluid environment surrounding the tablet. In a preferred embodiment, the combination of the solubility-enhanced form of the drug and the osmagent have an average ductility from about 100 to about 200 Mpa, an average tensile strength from about 0.8 to about 2.0 Mpa, and an average brittle fracture index less than about 0.2. The single-layer core may optionally include a disintegrant (preferably, a non-swelling, non-gelling disintegrant, more preferably, an ion exchange resin, most preferably, a polacrin potassium resin, such as Amberlite^{™} IRP-88), a bioavailability enhancing additive, and/or a pharmaceutically acceptable excipient, carrier or diluent.

Entrainment of particles of the solubility-enhanced form of the drug in the extruding fluid during operation of the monolayer osmotic tablet device is highly desirable. For the particles to be well entrained, the drug form is preferably well dispersed in the fluid before the particles have an opportunity to settle in the tablet core. One means of accomplishing this is by adding a disintegrant that serves to break up the compressed core into its particulate components. Examples of standard disintegrants included materials such as sodium starch glycolate (e.g., Explotab^{™} CLV), microcrystalline cellulose (e.g., Avicel^{™}), microcrystalline silicified cellulose (e.g., ProSolv^{™}) and croscarmellose sodium (e.g., Ac-Di-Sol^{™}), and other disintegrants known to those skilled in the art. Depending upon the particular formulation, some disintegrants work better than others. Several disintegrants tend to form gels as they swell with water, thus hindering drug delivery from the device. Non-gelling, non-swelling disintegrants provide a more rapid dispersion of the drug particles within the core as water enters the core. Preferred non-gelling, non-swelling disintegrants are resins, preferably ion-exchange resins. A preferred resin is Amberlite^{™} IRP 88 (available from Rohm and Haas, Philadelphia, PA). When used, the disintegrant is present in amounts ranging from about 1-25% of the core composition; more preferably from about 1-15%; still more preferably from about 1-10%.

Water-soluble polymers are added to keep particles of the solubility-enhanced drug form suspended inside the dosage form before they can be delivered through the passageway(s) (e.g., an orifice). High viscosity polymers are useful in preventing settling. However, the polymer in combination with the drug is extruded through the passageway(s) under relatively low pressures. At a given extrusion pressure, the extrusion rate typically slows with increased viscosity. Certain polymers in combination with particles of the solubility-enhanced drug form high viscosity solutions with water but are still capable of being extruded from the tablets with a relatively low force. In contrast, polymers having a low weight-average, molecular weight (< about 300,000) do not form sufficiently viscous solutions inside the tablet core to allow complete delivery due to particle settling. Settling of the particles is a problem when monolayer osmotic tablets are prepared with no polymer added, which leads to poor drug delivery unless the tablet is constantly agitated to keep the particles from settling inside the core. Settling is also problematic when the particles are large and/or of high density such that the rate of settling increases.

Preferred water-soluble polymers for monolayer osmotic tablets do not interact with the drug. Non-ionic polymers are preferred. An example of a non-ionic polymer forming solutions having a high viscosity yet still extrudable at low pressures is Natrosol^{™} 250H (high molecular weight hydroxyethylcellulose, available from Hercules Incorporated, Aqualon Division, Wilmington, DE; MW equal to about 1M and a degree of polymerization equal to about 3,700). Natrosol^{™} 250H provides effective drug delivery at concentrations as low as about 3% by weight of the core when combined with an osmagent. Natrosol^{™} 250H NF is a high-viscosity grade nonionic cellulose ether which is soluble in hot or cold water. The viscosity of a 1% solution of Natrosol^{™} 250H using a Brookfield LVT (30 rpm) at 25°C is between about 1,500 and about 2,500 cps.

Preferred hydroxyethylcellulose polymers for use in these monolayer osmotic tablets have a weight-average, molecular weight from about 300,000 to about 1.5 million.

The hydroxyethylcellulose polymer is typically present in the core in an amount from about 2.0% to about 35% by weight, preferably from about 3% to about 20%, more preferably from about 3% to about 15%, most preferably from about 3% to about 10%.

### OSMOTIC MULTIPARTICULATES

Another embodiment of sustained release osmotic dosage forms of the invention includes multiparticulates comprising the solubility-enhanced form of the CETPI (such as a solid amorphous dispersion), coated with a water-permeable membrane; the coating polymer may be dense, porous or asymmetric as described above. Such multiparticulates are prepared by, for example, melt-congealing from a spinning disk, extrusion/spheronization or fluid bed granulation, or by coating seed cores with a mixture of drug and a water-soluble polymer, as described above. Drug-containing multiparticulates may be homogeneous or layered with a drug-containing solid amorphous dispersion surrounding the seed core. Following formation, such multiparticulates are then spray-coated with a substantially water-permeable coating comprising a solution or suspension of a polymer in a mixture of a solvent and, depending on the coating type desired, a non-solvent, as described above. This spray-coating operation is preferably carried out in a fluid bed coating apparatus, for example, a Glatt GPCG-5 fluid bed coater (Glatt Air, Ramsey, New Jersey). The polymer used for forming the semipermeable membrane is chosen as described above.

### OSMOTIC CAPSULES

Osmotic capsules can be made using the same or similar components to those described above for osmotic tablets and multiparticulates. The capsule shell or portion of the capsule shell can be semipermeable and made of materials described above. The capsule can then be filled either by a powder or liquid consisting of drug dispersion, excipients that imbibe water to provide osmotic potential, and/or a water-swellable polymer, or optionally solubilizing excipients. The capsule core can also be made such that it has a bilayer or multilayer composition analogous to the bilayer, trilayer or concentric geometries described above.

### COATED SWELLABLE TABLETS

Another class of sustained release dosage forms useful in this invention comprises coated swellable tablets, as described in EP 378 404. Coated swellable tablets comprise a tablet core comprising the solubility-enhance form of the drug, such as a solid amorphous dispersion, and a swelling material, preferably a hydrophilic polymer, coated with a membrane, which contains holes, or pores through which, in the aqueous use environment, the hydrophilic polymer can extrude and carry out the drug composition. Alternatively, the membrane may contain polymeric or low molecular weight water-soluble "porosigens". Porosigens dissolve in the aqueous use environment, providing pores through which the hydrophilic polymer and drug may extrude. Examples of porosigens are water-soluble polymers such as HPMC, PEG, and low molecular weight compounds such as glycerol, sucrose, glucose, and sodium chloride. In addition, pores may be formed in the coating by drilling holes in the coating using a laser or other mechanical means. In this class of sustained release dosage forms, the membrane material may comprise any film-forming polymer, including polymers which are water permeable or impermeable, providing that the membrane deposited on the tablet core is porous or contains water-soluble porosigens or possesses a macroscopic hole for water ingress and drug release. Embodiments of this class of sustained release dosage forms may also be multilayered, as described in EP 378 404 A2.

### MULTIPARTICULATES

Alternatively, the compositions may be administered as multiparticulates. Multiparticulates generally refer to dosage forms that comprise a multiplicity of particles that may range in size from about 10 µm to about 2 mm, more typically about 100 µm to 1 mm in diameter. Such multiparticulates may be packaged, for example, in a capsule such as a gelatin capsule or a capsule formed from an aqueous-soluble polymer such as HPMCAS, HPMC or starch or they may be dosed as a suspension or slurry in a liquid.

Such multiparticulates may be made by any known process, such as wet- and dry-granulation processes, extrusion/spheronization, roller-compaction, or by spray-coating seed cores. For example, in wet- and dry-granulation processes, the composition of the solubility-enhanced form of the CETP inhibitor and optional precipitation inhibiting polymer is prepared as described above. This composition is then granulated to form multiparticulates of the desired size. Other excipients, such as a binder (e.g., microcrystalline cellulose), may be blended with the composition to aid in processing and forming the multiparticulates. In the case of wet granulation, a binder such as microcrystalline cellulose may be included in the granulation fluid to aid in forming a suitable multiparticulate.

In any case, the resulting particles may themselves constitute the multiparticulate dosage form or they may be coated by various film-forming materials such as enteric polymers or water-swellable or water-soluble polymers, or they may be combined with other excipients or vehicles to aid in dosing to patients.

For any of the controlled or sustained-release dosage forms mentioned above, the dosage form may additionally comprise an immediate-release layer of the same or a different drug in crystalline, amorphous or dispersion form.

### DETERMINATION OF RELEASE RATES

The CR dosage forms of the present invention may be evaluated in an *in vitro* test to determine whether a dosage form provides a release profile within the scope of the present invention. *In vitro* tests are well known in the art. An example is a "residual test," which is performed as follows. The dosage form is first placed into a stirred USP type 2 dissoette flask containing 900 mL of a buffer solution at 37°C simulating the contents of the small intestine. Preferably the buffer consists of 50 mM KH₂PO₄ at pH 6.8. The dosage form is placed in a wire support to keep the dosage form off of the bottom of the flask, so that all surfaces are exposed to the moving release solution and the solutions are stirred using paddles that rotate at a rate of 75 rpm. At each time interval, a single dosage form is removed from the solution, released material is removed from the surface, and the dosage form cut in half and placed in 100 mL of a recovery solution as follows. For the first two hours, the dosage form is stirred in 25 mL acetone or other solvent suitable to dissolve any coating on the dosage form. Next, 125 mL of methanol is added and stirring continued overnight at ambient temperature to dissolve the drug remaining in the dosage form. Approximately 2 mL of the recovery solution is removed and centrifuged, and 250 mL of supernatant added to an HPLC vial and diluted with 750 mL methanol. Residual drug is then analyzed by HPLC. The amount remaining in the tablets is subtracted from the total drug initially present in the tablet to obtain the amount released at each time interval.

An alternative *in vitro* test is a direct test, in which samples of the dosage form are placed into a stirred USP type 2 dissoette flask containing 900 mL of a receptor solution at 37°C simulating the contents of the small intestine but with a surfactant added to provide a sink for the drug. Preferably the receptor solution consists of 6 mM KH₂PO₄, 30 mM NaCl, 60 mM KCl, and 27 mL polyoxyethylene sorbitan monooleate (Tween 80) at pH 6.8. Dosage forms are placed in a wire support as above, and the receptor solution stirred 50 rpm. Samples of the receptor solution are taken at periodic intervals and the drug concentration is analyzed by HPLC.

Alternatively, an *in vivo* test may be used to determine whether a dosage form provides a drug release profile within the scope of the present invention. However, due to the inherent difficulties and complexity of the *in vivo* procedure, it is preferred that *in vitro* procedures be used to evaluate dosage forms even though the ultimate use environment is often the human GI tract. Dosage forms are dosed to a group of test subjects, such as humans, and drug release and drug absorption is monitored either by (1) periodically withdrawing blood and measuring the serum or plasma concentration of drug or (2) measuring the amount of drug remaining in the dosage form following its exit from the anus (residual drug) or (3) both (1) and (2). In the second method, residual drug is measured by recovering the tablet upon exit from the anus of the test subject and measuring the amount of drug remaining in the dosage form using the same procedure described above for the *in vitro* residual test. The difference between the amount of drug in the original dosage form and the amount of residual drug is a measure of the amount of drug released during the mouth-to-anus transit time. This test has limited utility since it provides only a single drug release time point but is useful in demonstrating the correlation between *in vitro* and *in vivo* release.

In one *in vivo* method of monitoring drug release and absorption, the serum or plasma drug concentration is plotted along the ordinate (y-axis) against the blood sample time along the abscissa (x-axis). The data may then be analyzed to determine drug release rates using any conventional analysis, such as the Wagner-Nelson or Loo-Riegelman analysis. See also Welling, "Pharmacokinetics: Processes and Mathematics" (ACS Monograph 185, Amer. Chem. Soc., Washington, D.C., 1986). Treatment of the data in this manner yields an apparent *in vivo* drug release profile.

The dosage forms of the present invention release the CETPI slowly to the use environment. As used here and in the claims, the average rate of release of CETPI per hour for a time period is defined as the wt% of CETPI present in the dosage form released during the time period divided by the duration (in hours) of the time period. For example, if the dosage form releases 80 wt% of the CETPI initially present in the dosage form in 16 hours, the average rate of release of CETPI is 5 wt%/hr (80 wt%/16 hours). Average rates of release of CETPI from a dosage form may be determined using the *in vitro* or *in vivo* tests described above.

The CR dosage forms of the present invention release CETPI at a rate that is slower than the release rate provided by an immediate release (IR) dosage form. By "immediate release dosage form" is meant a dosage form that releases 80 wt% of the CETPI initially present in the dosage form within 1 hour or less following introduction to an environment of use. Thus, an IR dosage form releases CETPI at an average rate of 80 wt%/hr or more.

Preferably, the CR dosage forms of the present invention release CETPI at an average rate that is about 40 wt%/hr or less, more preferably about 30 wt%/hr or less, and even more preferably about 25 wt%/hr or less. However, the release of CETPI from the dosage form should not be too slow. Thus, it is also preferred that the CR dosage forms of the present invention release CETPI at an average rate that is about 2.5 wt%/hr or more, preferably about 3 wt%/hr or more, more preferably about 3.5 wt%/hr or more, most preferably about 4 wt%/hr or more.

In one separate aspect, the CR dosage form provides controlled release relative to an immediate release control dosage form consisting of an equivalent amount of the CETPI in the same solubility-enhanced form dosed as an oral powder for constitution. In one embodiment, the CR dosage form has a time to reach maximum drug concentration (Tₘₐₓ) in the *in vivo* use environment following administration that is least 1.25-fold longer than the immediate release control dosage form, preferably at least 2-fold longer, and more preferably at least 3-fold longer. In addition, the maximum concentration of drug in the *in vivo* use environment (Cₘₐₓ) is less than or equal to 80%, and may be less than or equal to 65%, or even less than or equal to 50% of the Cₘₐₓ provided by the immediate release control dosage form. Both Tₘₐₓ and Cₘₐₓ may be compared in either the fed or fasted state, and the CR dosage form meets the above criteria for at least one of, and preferably both, the fed and fasted state.

In another separate aspect, the CR dosage form provides an improvement in bioavailability compared with the immediate release dosage form. The CR dosage forms, when dosed orally to a human or other animal, provide an area under the curve (AUC) in drug concentration in the blood that is at least about 1.25-fold, preferably at least about 2-fold, and more preferably at least about 3-fold, that observed when an immediate release control composition is dosed. It is noted that such compositions can also be said to have a relative bioavailability of from about 1.25-fold to about 3-fold that of the control composition. Relative bioavailability may be compared in either the fed or fasted state, and meets the above criteria in at least one of, and preferably both, the fed and fasted state.

Relative bioavailability of the drug in the dosage forms can be tested *in vivo* in animals or humans using conventional methods for making such a determination. An *in vivo* test, such as a crossover study, may be used to determine whether a composition provides an enhanced relative bioavailability compared with a control composition as described above. In an *in vivo* crossover study a test composition is dosed to half a group of test subjects and, after an appropriate washout period (e.g., one week) the same subjects are dosed with a control composition. The other half of the group is dosed with the control composition first, followed by the test composition. The relative bioavailability is measured as the concentration in the blood (serum or plasma) versus time area under the curve (AUC) determined for the test group divided by the AUC in the blood provided by the control composition. Preferably, this test/control ratio is determined for each subject, and then the ratios are averaged over all subjects in the study. *In vivo* determinations of AUC can be made by plotting the serum or plasma concentration of drug along the ordinate (y-axis) against time along the abscissa (x-axis). To facilitate dosing, a dosing vehicle may be used to administer the dose. The dosing vehicle is preferably water, but may also contain materials for suspending the test or control composition, provided these materials do not dissolve the composition or change the drug solubility *in vivo.*

Compositions of the present invention may be used to treat any condition, which is subject to treatment by administering a CETP inhibitor.

One aspect of this invention is directed to a composition of the present invention for use in the treatment of atherosclerosis in a mammal (including a human being).

Yet another aspect of this invention is directed to a composition of the present invention for use in treating peripheral vascular disease in a mammal (including a human being).

Yet another aspect of this invention is directed to a composition of the present invention for use in treating dyslipidemia in a mammal (including a human being).

Yet another aspect of this invention is directed to a composition of the present invention for use in the treatment of hyperbetalipoproteinemia in a mammal (including a human being).

Yet another aspect of this invention is directed to a composition of the present invention for use in the treatment of hypoalphalipoproteinemia in a mammal (including a human being).

Yet another aspect of this invention is directed to a composition of the present invention for use in the treatment of hypercholesterolemia in a mammal (including a human being).

Yet another aspect of this invention is directed to a composition of the present invention for use in the treatment of hypertriglyceridemia in a mammal (including a human being).

Yet another aspect of this invention is directed to a composition of the present invention for use in the treatment of familial-hypercholesterolemia in a mammal (including a human being).

Yet another aspect of this invention is directed to a composition of the present invention for use in the treatment of cardiovascular disorders in a mammal (including a human being).

Yet another aspect of this invention is directed to a composition of the present invention for use in the treatment of angina in a mammal (including a human being).

Yet another aspect of this invention is directed to a composition of the present invention for use in the treatment of ischemia in a mammal (including a human being).

Yet another aspect of this invention is directed to a composition of the present invention for use in the treatment of cardiac ischemia in a mammal (including a human being).

Yet another aspect of this invention is directed to a composition of the present invention for use in the treatment of stroke in a mammal (including a human being).

Yet another aspect of this invention is directed to a composition of the present invention for use in the treatment of a myocardial infarction in a mammal (including a human being).

Yet another aspect of this invention is directed to a composition of the present invention for use in the treatment of reperfusion injury in a mammal (including a human being).

Yet another aspect of this invention is directed to a composition of the present invention for use in the treatment of angioplastic restenosis in a mammal (including a human being).

Yet another aspect of this invention is directed to a composition of the present invention for use in the treatment of hypertension in a mammal (including a human being).

Yet another aspect of this invention is directed to a composition of the present invention for use in the treatment of the vascular complications of diabetes in a mammal (including a human being).

Yet another aspect of this invention is directed to a composition of the present invention for use in the treatment of obesity in a mammal (including a human being).

Yet another aspect of this invention is directed to a composition of the present invention for use in the treatment of endotoxemia in a mammal (including a human being).

### EXAMPLES

### Examples 1-2

Examples 1-2 demonstrate the utility of the amorphous dispersions of the present invention with a CETP inhibitor, [2R,4S] 4-[(3,5-bis-trifluoromethyl-benzyl)-methoxycarbonyl-amino]-2-ethyl-6-trifluoromethyl-3,4-dihydro-2H-quinoline-1-carboxylic acid ethyl ester ("Drug A"), which has a solubility in water of <1 µg/ml, and a Clog P value of 7.5. (ClogP is the log of the calculated octanol-water partition coefficient.) To prepare Example 1, an amorphous solid dispersion of 25 wt% Drug A and 75 wt% polymer was made by mixing Drug A in the solvent acetone together with a "medium fine" (AQUOT-MF) grade of the cellulosic ester polymer HPMCAS (manufactured by Shin Etsu) to form a solution. The solution comprised 2.5 wt% Drug A, 7.5 wt% HPMCAS, and 90 wt% acetone. This solution was then spray-dried by directing an atomizing spray using a two-fluid external-mix spray nozzle at 2.7 bar (37 psig) at a feed rate of 150 g/min into the stainless-steel chamber of a Niro PSD-1 spray-dryer, maintained at a temperature of 155°C at the inlet and 70°C at the outlet. The preparation parameters are summarized in Table 1. The resulting amorphous solid spray-dried dispersion was collected via a cyclone and then dried in a Gruenberg solvent tray-dryer by spreading the spray-dried particles onto polyethylene-lined trays to a depth of not more than 1 cm and then drying them at 40°C for 24 hours.

Example 2 was prepared following the general procedure described in Example 1 except that the dispersion contained 10 wt% Drug A and the spray solution comprised 1.0 wt% Drug A, 9.0 wt% HPMCAS-MF, and 90 wt% acetone. The preparation parameters are summarized in Table 1.

**Table 1**

| Example | Drug A Mass (g) | Aqueous-Soluble Polymer | Polymer Mass (g) | Solvent | Solvent Mass (g) | Spray Apparatus |
|---|---|---|---|---|---|---|
| 1 | 100 | HPMCAS-MF | 300 | acetone | 3600 | PSD-1 |
| 2 | 100 | HPMCAS-MF | 900 | acetone | 9000 | PSD-1 |
| Control 1 | 0.0018 | none | - | - | - | - |

Comparative composition Control 1 consisted of 1.8 mg of the crystalline form of Drug A alone.

### Example 3

The spray-dried solid amorphous dispersions of Examples 1 and 2 were evaluated in an *in vitro* dissolution test using a microcentrifuge method. In this test, the spray-dried solid amorphous dispersion was added to a microcentrifuge tube for a Drug A dose of about 1000 µg/mL (7.2 mg for Example 1, 18 mg for Example 2). The tube was placed in a 37°C sonicating bath, and 1.8 mL phosphate buffered saline (PBS) at pH 6.5 and 290 mOsm/kg was added. The samples were quickly mixed using a vortex mixer for about 60 seconds. The samples were centrifuged at 13,000 G at 37°C for 1 minute. The resulting supernatant solution was then sampled and diluted 1:6 (by volume) with methanol and then analyzed by high-performance liquid chromatography (HPLC). The contents of the tubes were mixed on the vortex mixer and allowed to stand undisturbed at 37°C until the next sample was taken. Samples were collected at 4, 10, 20, 40, 90, and 1200 minutes. The concentrations of drug obtained in these samples are shown in Table 2.

For Control 1, an *in vitro* dissolution test was performed using the procedures described above except that 1.8 mg of crystalline Drug A was used. The concentrations of drug obtained in *in vitro* dissolution tests are shown in Table 2.

**Table 2**

| Example | Time (min) | Drug A Concentration (µg/mL) | AUC (min-µg/mL) |
|---|---|---|---|
| 1 | 0 | 0 | 0 |
| | 4 | 328 | 660 |
| | 10 | 701 | 3,700 |
| | 20 | 781 | 11,200 |
| | 40 | 805 | 27,000 |
| | 90 | 780 | 66,600 |
| | 1200 | 439 | 743,200 |
| 2 | 0 | 0 | 0 |
| | 4 | 925 | 1,900 |
| | 10 | 923 | 7,400 |
| | 20 | 910 | 16,600 |
| | 40 | 890 | 34,600 |
| | 90 | 858 | 78,300 |
| | 1200 | 623 | 900,200 |
| Control 1 | 0 | 0 | 0 |
| | 4 | <1 | <2 |
| | 10 | <1 | <8 |
| | 20 | <1 | <18 |
| | 40 | <1 | <38 |
| | 90 | <1 | <88 |
| | 1200 | <1 | <1,200 |

The results of dissolution tests for Examples 1 and 2, and Control 1 are summarized in Table 3, which shows the maximum concentration of Drug A in solution during the first 90 minutes of the test (C_{max,90}), the area under the aqueous concentration versus time curve after 90 minutes (AUC₉₀), and the concentration at 1200 minutes (C₁₂₀₀).

**Table 3**

| Example | Aqueous-Soluble Polymer | Drug A Conc. in the Dispersion (wt%) | Receptor Solution | C_{max,90} (µg/mL) | AUC₉₀ (min-µg/mL) | C₁₂₀₀ (µg/mL) |
|---|---|---|---|---|---|---|
| 1 | HPMCAS-MF | 25 | PBS | 805 | 66,600 | 439 |
| 2 | HPMCAS-MF | 10 | PBS | 925 | 78,300 | 623 |
| Control 1 | None (crystalline drug) | NA | PBS | <1 | <88 | <1 |

The results summarized in Table 3 show that the dissolution results for the compositions of Examples 1 and 2 were much better than that of the crystalline drug alone, providing C_{max,90} values that were greater than 805-fold and 925-fold that of the crystalline drug (Control 1), respectively, and AUC₉₀ values that were greater than 756-fold and 889-fold that of the crystalline drug (Control 1), respectively. Accurate measurements of the solubility of crystalline Drug A yield a value of about 0.01 µg/ml. Thus, the actual C_{max,90} for Drug A in Control 1 is believed to be about 0.01 µg/ml. Using this value, the compositions of Examples 1 and 2 provided C_{max,90} values that were about 80,000-fold to 92,500-fold that of the crystalline drug, and AUC₉₀ values that were about 70,000- to 80,000-fold that of the crystalline drug, respectively.

### Example 4

The following process was used to form a spray-dried solid amorphous dispersion containing 25 wt% Drug A and 75 wt% HPMCAS-MG. First, a 10,000 g spray solution was formed containing 2.5 wt% Drug A, 7.5 wt% HPMCAS-MG, and 90% acetone as follows. The HPMCAS-MG and acetone were combined in a container and mixed for at least 2 hours, allowing the HPMCAS to dissolve. The resulting mixture had a slight haze after the entire amount of polymer had been added. Next, Drug A was added directly to this mixture, and the mixture stirred for an additional 2 hours. This mixture was then filtered by passing it through a filter with a screen size of 250 µm to remove any large insoluble material from the mixture, thus forming the spray solution.

The spray-dried solid amorphous dispersion was then formed using the following procedure. The spray solution was pumped using a high-pressure pump (a Zenith Z-Drive 2000 High-Pressure Gear Pump), to a spray drier (a Niro type XP Portable Spray-Dryer with a Liquid-Feed Process Vessel) ("PSD-1"), equipped with a pressure nozzle (Spraying Systems Pressure Nozzle and Body) (SK 71-16). The PSD-1 was equipped with a 9-inch chamber extension. The 9-inch chamber extension was added to the spray dryer to increase the vertical length of the dryer. The added length increased the residence time within the dryer, which allowed the product to dry before reaching the angled section of the spray dryer. The spray drier was also equipped with a gas-dispersing means for introduction of the drying gas to the spray drying chamber. The gas-dispersing means consisted of a plate coextensive with the interior of the drying chamber (about 0.8 m diameter) and bearing a multiplicity of 1.7 mm perforations occupying about 1% of the surface area of the plate. The perforations were uniformly distributed across the plate, except that the density of perforations at the center 0.2 m of the diffuser plate was about 40% of the density of perforations in the outer part of the diffuser plate. The use of the diffuser plate resulted in organized plug flow of drying gas through the drying chamber and dramatically decreased product re-circulation within the spray dryer. The nozzle sat flush with the diffuser plate during operation. The spray solution was pumped to the spray drier at about 195 gm/min at a pressure of about 100 psig. Drying gas (e.g., nitrogen) was delivered to the diffuser plate at an inlet temperature of about 106°C. The evaporated solvent and drying gas exited the spray drier at a temperature of 45±4°C. The spray-dried dispersion formed by this process was collected in a cyclone, and had have a bulk specific volume of about 5 cm³/gm, with a mean particle size of about 80 µm. The concentration of residual acetone in the dispersion was 3 wt%.

The solid amorphous dispersion formed using the above procedure was post-dried using a Gruenberg single-pass convection tray dryer operating at 40°C for 25 hours. Following drying, the dispersion was equilibrated with ambient air and humidity (e.g., 20°C/50% RH).

The properties of the dispersion after secondary drying were as follows:

**Table 4**

| Bulk Properties (After Secondary Drying) | Tray Dried @ 40°C |
|---|---|
| Bulk Specific Volume (cc/g) | 5.0 |
| Tapped Specific Volume (cc/g) | 3.2 |
| Mean Particle Diameter (µm) | 80 |
| D₁₀, D₅₀, D₉₀ (µm) | 25, 73, 143 |
| Span (D₉₀-D₁₀)/D₅₀ | 1.60 |
| * 10 vol% of the particles had a diameter that was smaller than D₁₀; 50 vol% of the particles had a diameter that was smaller than D₅₀, and 90 vol% of the particles had a diameter that was smaller than D₉₀. | |

### Example 5

This example demonstrates the controlled release of a solid amorphous dispersion of Drug A using a bi-layer osmotic tablet controlled-release dosage form of the present invention.

### Preparation of the Dispersion

A solid amorphous dispersion of Drug A in HPMCAS was prepared using the procedures outlined in Example 4 with the following exceptions. The spray solution consisted of 2.3 wt% Drug A, 6.8 wt% HPMCAS-MG, and 90.9 wt% acetone. The spray solution was pumped to the spray drier at 185 gm/min at a pressure of about 225 psig. Drying gas (nitrogen) was circulated through the diffuser plate at an inlet temperature of about 96°C. The evaporated solvent and wet drying gas exited the spray drier at a temperature of 33°C. The spray-dried solid amorphous dispersion formed by this process was collected in a cyclone.

The solid amorphous dispersion formed using the above procedure was post-dried using a Gruenberg single-pass convection tray dryer operating at 40°C for about 3 hours. Following drying, the dispersion was then equilibrated with ambient air and humidity (e.g., 20°C/50% RH).

### Preparation of the Drug-Containing Composition

To form the drug-containing composition, the following materials were blended: 48 wt% Drug A dispersion (25 wt% Drug A/75 wt% HPMCAS), 23 wt% polyethylene oxide (PEO) having an average molecular weight of 600,000, 23 wt% xylitol (trade name XYLITAB 200), 5 wt% sodium starch glycolate (trade name EXPLOTAB), and 1 wt% magnesium stearate. The drug-containing composition ingredients were first combined without the magnesium stearate and blended for 20 minutes in a TURBULA mixer. This blend was pushed through a screen (screen size of 0.065 inch), then blended again for 20 minutes in the same mixer. Next, magnesium stearate was added and the drug-containing composition was blended again for 4 minutes in the same mixer.

### Preparation of the Water-Swellable Composition

To form the water-swellable composition, the following materials were blended: 75 wt% sodium croscarmellose (trade name AcDiSol), 24.4 wt% of the tableting aid silicified microcrystalline cellulose (trade name PROSOLV 90), 0.5 wt% magnesium stearate, and 0.1 wt% Red Lake #40. The AcDiSol and PROSOLV were combined and blended for 20 minutes in aTURBULA mixer. Next, the magnesium stearate and Red Lake dye were mixed together. All ingredients were pushed through a screen (screen size of 0.033 inch), then blended again for 20 minutes in the same mixer.

### Preparation of Tablet Cores

Tablet cores were formed by placing 375 mg of the drug-containing composition in a standard 13/32-inch standard round concave (SRC) die and gently leveling with the press. Then, 125 mg of the water-swellable composition was placed in the die on top of the drug-containing composition. The tablet core was then compressed to a hardness of about 14 kiloponds (Kp). The resulting bi-layer tablet core had a total weight of 500 mg and contained a total of 9.0 wt% Drug A (45 mg), 27.0 wt% HPMCAS-MG, 17.25 wt% XYLITAB 200, 17.25 wt% PEO 600,000, 3.75 wt% EXPLOTAB, 18.75 wt% AcDiSol, 6.1 wt% PROSOLV 90, 0.87 wt% magnesium stearate, and 0.03 wt% Red Lake dye.

### Application of the Coating

Coatings were applied in a Vector LDCS-20 pan coater. The coating solution contained cellulose acetate (CA 398-10 from Eastman Fine Chemical, Kingsport, Tennessee), polyethylene glycol (PEG 3350, Union Carbide), water, and acetone in a weight ratio of 3.5/1.5/3/92 (wt%). The flow rate of the inlet heated drying gas of the pan coater was set at 40 ft³/min with the outlet temperature set at 25°C. Nitrogen at 20 psi was used to atomize the coating solution from the spray nozzle, with a nozzle-to-bed distance of 2 inches. The pan rotation was set to 20 rpm. The so-coated tablets were dried at 50°C in a convection oven. The final dry coating weight amounted to about 15 wt% of the tablet core. One 900 µm diameter hole was then mechanically drilled in the coating on the drug-containing composition side of the tablet to provide one delivery port per tablet.

### Example 5A: In Vitro Dissolution Tests

*In vitro* tests were performed using residual drug analysis as follows. A sample of the dosage form was first placed into a stirred USP type 2 dissoette flask containing 1000 mL of a buffer solution simulating the contents of the small intestine (50 mM KH₂PO₄, pH 6.8, 37°C). In the flasks, the dosage form was placed in a wire support to keep the dosage form off of the bottom of the flask, so that all surfaces were exposed to the moving release solution and the solutions were stirred using paddles at a rate of 75 rpm. At each time interval, a single dosage form was removed from the solution, released material was rinsed from the surface, the dosage form cut in half and placed in a recovery solution as follows. For the first two hours, the tablet was stirred in 25 mL acetone to dissolve the tablet coating. Next, 125 mL of methanol was added and stirring continued overnight at ambient temperature to dissolve the drug remaining in the dosage form. Approximately 2 mL of the solution was removed and centrifuged, and 250 µL of supernatant added to an HPLC vial and diluted with 750 µL methanol. Residual drug was analyzed by HPLC at a UV absorbance of 256 nm using a Waters Symmetry C8 column and a mobile phase consisting of 15% (0.2% H₃PO₄)/85% methanol. Drug concentration was calculated by comparing UV absorbance of samples to the absorbance of drug standards. The amount of drug remaining in the tablets was subtracted from the total drug initially present in the tablet to obtain the amount released at each time interval. Results are shown in Table 5.

**Table 5**

| Time (hours) | Residual Analysis Drug A (mg released) | Residual Analysis Drug A (wt% released) |
|---|---|---|
| 0 | 0 | 0 |
| 6 | 19 | 43 |
| 12 | 36 | 80 |
| 20 | 41 | 92 |

The data demonstrate that the dosage form provided controlled release of Drug A, releasing 80 wt% of the drug over a 12 hour period.

### Example 5B: In Vivo Tests

The controlled release dosage forms were tested in *in vivo* tests using male beagle dogs. Each dog was dosed with two of the tablets of Example 5, resulting in a total amount dosed of 90 mgA Drug A. For one set of 6 dogs, the dogs were fasted for at least 12 hours prior to dosing. For another set of 6 dogs, the dogs were fed a meal prior to dosing. Whole blood samples of 6 ml were taken from the jugular vein using a plasma serum separator tube containing sodium heparin with a 20 gauge needle at 0, ½, 1, 2, 3, 4, 6, 8, 12, 24, and 28 hours post dosing. Samples were spun in a refrigerated (5°C) centrifuge at 3000 rpm for 5 minutes. The resultant plasma samples were poured into 2 ml cryogenic plastic tubes and stored in a freezer (-20°C) within ½ hour post sampling time. Samples were then analyzed for Drug A using an HPLC method.

For Control 1, 90 mgA of crystalline Drug A was dosed as an oral powder for constitution (OPC) as follows. The OPC was dosed as a suspension in a solution containing 0.5 wt% Methocel® (Dow Chemical Co.), and was prepared as follows. First, 7.5 g of Methocel® was weighed out and added slowly to approximately 490 ml of water at 90° to 100°C to form a Methocel® suspension. After all the Methocel® was added, the suspension was placed in a beaker of ice water. Next, 1000 ml of chilled water was added with stirring. When all of the Methocel® had been suspended, 2.55 g Tween 80 was added and the mixture stirred until all had dissolved. A 400 mL sample of this solution was then placed in a 500 mL container. Next, 90 mgA of crystalline Drug A was added in a mortar. About 20 mL of the Methocel® suspension was added to the mortar and the drug mixture was ground with a pestle until a uniform suspension had formed. Additional Methocel® suspension was added gradually with grinding until all of the 400 mL sample of the Methocel® suspension solution was in the mortar. The suspension was then transferred back to the 500 mL container.

As Control 2, an OPC was prepared as above using 90 mgA of the dispersion described in Example 4.

Table 6 summarizes the results of these tests, which show that the dosage forms of Example 5 provided controlled-release of Drug A. The dosage forms of Example 5 provided a Tₘₐₓ that was significantly longer than that provided by either of the immediate release controls. In addition, the Cₘₐₓ values obtained with the controlled-release dosage forms of Example 5 were lower than the corresponding Cₘₐₓ values obtained with the Example 4 OPC. Additionally, the dosage forms of Example 5 provided concentration enhancement of Drug A over the crystalline control.

**Table 6**

| Dosage Form | Condition | AUC₀₋₂₄ₕᵣ (ng-hr/mL) | Cₘₐₓ (ng/mL) | Tₘₐₓ (hr) |
|---|---|---|---|---|
| Example 5 | Fasted (N = 6) | 4209 ± 3088 | 376 ± 225 | 15.3 ± 8.5 |
| | Fed (N = 6) | 5623 ± 1774 | 535 ± 85 | 9.3 ± 3.0 |
| Control 1 (crystalline Drug A) | Fasted (N = 6) | < LOQ* | < LOQ | <LOQ |
| | Fed (N = 6) | 928 ± 641 | 191 ± 55 | 2.3 ± 1.3 |
| Control 2 (Example 4 dispersion as an OPC) | Fasted (N = 24) | 1725 ± 780 | 481 ± 179 | 1.4 ± 1.1 |
| | Fed (N = 6) | 6673 ± 1255 | 1281 ± 610 | 1.0 ± 0 |
| * LOQ = level of quantification | | | | |

### Examples 6-8

These examples demonstrate controlled release of an amorphous dispersion of Drug A in a concentration-enhancing polymer from an erodible matrix dosage form of the present invention.

Matrix tablets containing 90 mgA of Drug A were formed by combining an amorphous solid dispersion of 25 wt% Drug A in 75 wt% HPMCAS-MG (prepared using a process similar to the processes described for Example 4), hydroxypropyl methylcellulose (METHOCEL K100LV), xylitol (XYLITAB 200), and magnesium stearate using the following process. First, 7.5 g 25 wt% Drug A/HPMCAS-MG, 3.375 g METHOCEL, and 3.975 g XYLITAB were combined and blended for 20 minutes in a TURBULA mixer. This blend was pushed through a 20 mesh screen, then blended again for 20 minutes in the same mixer. Next, 0.15 g magnesium stearate was added and the composition was blended again for 4 minutes in the same mixer. Tablet cores were then formed by placing 720 mg of this blend into a caplet die (0.3300 x 0.6585 inch) and compressing to a hardness of about 8 Kp. Table 7 summarizes the composition of the tablets of Example 6. The tablets of Examples 7 and 8 where made using the same process but with the compositions shown in Table 7. Each of the dosage forms of Examples 6, 7, and 8 contained 90 mgA Drug A.

**Table 7**

| Example | Composition (wt%) | | | |
|---|---|---|---|---|
| | Dispersion (25% Drug A/ 75% HPMCAS-MG) | METHOCEL | XYLITAB | Magnesium Stearate |
| Example 6 | 50.0 | 22.5 | 26.5 | 1.0 |
| Example 7 | 50.0 | 15.0 | 34.0 | 1.0 |
| Example 8 | 50.0 | 12.0 | 37.0 | 1.0 |

*In vitro* tests were performed by placing a single dosage form into a dissoette flask stirred at 50 rpm with 900 mL of receptor solution containing 6 mM KH₂PO₄, 30 mM NaCl, 60 mM KCl, and 27 mL polyoxyethylene sorbitan monooleate (Tween 80) (pH 6.8, 37°C). The autosampler dissoette device removed a sample of the receptor solution at 1, 2, 3, 4, 5, 6, 7, 8, 10, and 12 hours. Samples were centrifuged for 1 minute, and 0.5 mL of supernatant was removed and placed in an HPLC vial with 0.5 mL methanol. The concentration of Drug A released was analyzed by HPLC. The results are shown in Table 8.

**Table 8**

| Time (hours) | Example 6 Drug A (wt% released) | Example 7 Drug A (wt% released) | Example 8 Drug A (wt% released) |
|---|---|---|---|
| 0 | 0 | 0 | 0 |
| 1 | 19.1 | 26.4 | 27.7 |
| 2 | 27.3 | 39.0 | 45.5 |
| 3 | 34.3 | 46.4 | 65.3 |
| 4 | 40.0 | 55.8 | 88.7 |
| 5 | 47.0 | 63.1 | 99.4 |
| 6 | 51.8 | 70.4 | 101.0 |
| 7 | 58.1 | 77.9 | 102.2 |
| 8 | 63.9 | 84.6 | 101.3 |
| 10 | 71.8 | 93.0 | 102.2 |
| 12 | 80.5 | 102.4 | 101.4 |

The data demonstrate controlled delivery of a dispersion of Drug A from dosage forms of this invention. Increasing the amount of XYLITAB and decreasing the amount of METHOCEL in the tablets resulted in faster release of Drug A. The dosage form of Example 6 released 80% of the drug in about 12 hours, while the dosage form of Example 7 released 80% of the drug in about 7.5 hours, and the dosage form of Example 8 released 80% of the drug in about 3.5 hours.

### Example 9.

Human subjects were dosed 120 mg Drug A, plasma was collected at multiple times post-dose, plasma Drug A concentrations were determined, and a single-dose plasma Drug A concentration vs. time curve was prepared. Pharmacokinetic (PK) constants were derived by fitting this curve to a 2-compartment PK model, assuming a small intestinal transit time of 4 hr, and a gastric emptying time of 15 min. The fitted derived constants were:
Absorption Ka = 2.27 (1/hr)
K12 = 0.017 (1/hr)
K21 = 0.002 (1/hr)
Clearance (CI) = 13.05 (liters/hr)
Volume of distribution (Vd) = 173.7 (liters)
Kel (Elimination Rate Constant) was calculated from Cl/Vd = 0.042 (1/hr)

This mathematical model was used to explore Drug A input rates (Drug A release rates from controlled release Drug A dosage forms) which would meet certain *in vivo* criteria. Drug A input into the body was modeled as a zero order (constant rate) input and, in the tables below is described as time to reach 80% release of Drug A from the controlled release dosage form. Drug A plasma concentrations were computed for each 0.5 hr after dosing for 500 hr post-dose. These computed data were superimposed for 14 days of dosing to give the predicted plasma Drug A vs. time curve for day 14 (at steady state). The model was run for Drug A input rates and doses.

Drug A release rates are described in terms of the time duration between dosing the dosage form to an environment of use and the time at which 80% of the Drug A has left the dosage form.

For once daily (QD) dosing, the model was run utilizing three assumptions about colonic bioavailability: colonic bioavailability is 0%, 30%, or 80% relative to oral bioavailability. It is likely that colonic absorption of Drug A will be poor, due to its low solubility and the small quantity of water available in the colon for drug dissolution. This was approximated in the model by varying the colonic absorption rate constant. *In vivo* in humans, CR dosage forms reach the colon about 4-6 hr after dosing when the subject is fasted, and about 6-8 hr after dosing when the subject is fed, depending on the size of the meal. The model was run assuming a 4 hr small intestinal transit time and a 15 min gastric emptying time, which models fasted state dosing. The model assumes that the dosage form itself exits the stomach at 1 hr after dosing. The model may also be run with a longer gastric emptying time, e.g. 2-4 hr, to simulate dosing in the fed state. The modeled data below are for fasted state dosing of controlled release Drug A dosage forms, unless otherwise indicated.

The model was used to model twice daily (BID) dosing of doses from 5-60 mg Drug A, at various drug release rates from the CR dosage form. Table 9 shows which doses and release rates would achieve plasma Drug A concentrations above 70 ng/ml for at least about 12 hr. Table 9 also shows which doses and release rates would achieve 50% or greater inhibition of plasma CETP for at least 12 hr.

**Table 9.**

| Determination of which Drug A doses and release rates, when dosed BID, will achieve plasma Drug A concentrations above 70 ng/ml and 50% or greater CETP inhibition for at least about 12 hr, on the 14^{th} day of dosing. It is assumed that no colonic absorption occurs. | | | |
|---|---|---|---|
| Dose (mg) | Time to Reach 80% Release (hr) | Plasma Drug A Conc. greater than 70 ng/ml for 12 hr? | Plasma CETP inhibition greater than 50% for 12 hr? |
| 5 | 2 | No | No |
| 10 | 2 | Yes | Yes |
| 10 | 4 | Yes | Yes |
| 10 | 6 | No | No |
| 10 | 8 | No | No |
| 30 | 2 | Yes | Yes |
| 30 | 4 | Yes | Yes |
| 30 | 6 | Yes | Yes |
| 30 | 8 | Yes | Yes |
| 40 | 2 | Yes | Yes |
| 40 | 4 | Yes | Yes |
| 40 | 6 | Yes | Yes |
| 40 | 8 | Yes | Yes |
| 60 | 2 | Yes | Yes |
| 60 | 4 | Yes | Yes |
| 60 | 6 | Yes | Yes |
| 60 | 8 | Yes | Yes |

The model was used to model once daily dosing of doses from 10-60 mg Drug A, at various drug release rates from the CR dosage form. Table 10 shows which doses and release rates would achieve plasma Drug A concentrations above 70 ng/ml for at least about 16 hr. Table 10 also shows which doses and release rates would achieve 50% or greater inhibition of plasma CETP for at least about 16 hr. It is assumed that the colonic bioavailability is about 30%.

**Table 10**

| Determination of which Drug A doses and release rates, when dosed QD, will achieve plasma Drug A concentrations above 70 ng/ml and 50% or greater CETP inhibition for at least about 16 hr, on the 14^{th} day of dosing. It is assumed that the colonic bioavailability constant is about 30%. | | | |
|---|---|---|---|
| Dose (mg) | Time to Reach 80% Release (hr) | Plasma Drug A Conc. greater than 70 ng/ml for 16 hr? | Plasma CETP inhibition greater than 50% for 16 hr? |
| 10 | 2 | No | No |
| 10 | 4 | No | No |
| 10 | 6 | No | No |
| 10 | 8 | No | No |
| 30 | 2 | Yes | Yes |
| 30 | 4 | Yes | Yes |
| 30 | 6 | Yes | Yes |
| 30 | 8 | Yes | Yes |
| 30 | 12 | No | No |
| 40 | 2 | Yes - | Yes |
| 40 | 4 | Yes | Yes |
| 40 | 6 | Yes | Yes |
| 40 | 8 | Yes | Yes |
| 40 | 12 | Yes | Yes |
| 60 | 2 | Yes | Yes |
| 60 | 4 | Yes | Yes |
| 60 | 6 | Yes | Yes |
| 60 | 8 | Yes | Yes |
| 60 | 12 | Yes | Yes |
| 60 | 18 | Yes | Yes |

Although the modeling in Table 10 was only carried out up to 60 mg, it follows that doses higher than 60 mg, dosed QD at 80% released in 2-18 hr, will achieve the stated plasma Drug A concentration and CETP inhibition targets of Table 10.

The model was used to model once daily dosing of doses from 10-60 mg Drug A, at various drug release rates from the CR dosage form. Table 11 shows which doses and release rates would achieve plasma Drug A concentrations above 70 ng/ml for at least about 16 hr. Table 11 also shows which doses and release rates would achieve 50% or greater inhibition of plasma CETP for at least about 16 hr. It is assumed that the colonic bioavailability constant is about 80%.

**Table 11**

| Determination of which Drug A doses and release rates, when dosed QD, will achieve plasma Drug A concentrations above 70 ng/ml and 50% or greater CETP inhibition for at least about 16 hr; on the 14^{th} day of dosing. It is assumed that the colonic bioavailability constant is about 80%. | | | |
|---|---|---|---|
| Dose (mg) | Time to Reach 80% Release (hr) | Plasma Drug A Conc. greater than 70 ng/ml for 16 hr? | Plasma CETP inhibition greater than 50% for 16 hr? |
| 10 | 2 | No | No |
| 10 | 4 | No | No |
| 10 | 6 | No | No |
| 10 | 8 | No | No |
| 30 | 2 | Yes | Yes |
| 30 | 4 | Yes | Yes |
| 30 | 6 | Yes | Yes |
| 30 | 8 | Yes | Yes |
| 30 | 12 | Yes | Yes |
| 30 | 16 | Yes | Yes |
| 40 | 2 | Yes | Yes |
| 40 | 4 | Yes | Yes |
| 40 | 6 | Yes | Yes |
| 40 | 8 | Yes | Yes |
| 60 | 2 | Yes | Yes |
| 60 | 4 | Yes | Yes |
| 60 | 6 | Yes | Yes |
| 60 | 8 | Yes | Yes |

The model was used to model once daily dosing of doses from 10-60 mg Drug A, at various drug release rates from the CR dosage form. Table 12 shows which doses and release rates would achieve plasma Drug A concentrations above 70 ng/ml for at least about 16 hr. Table 12 also shows which doses and release rates would achieve 50% or greater inhibition of plasma CETP for at least about 16 hr. It is assumed that the colonic bioavailability constant is zero.

**Table 12**

| Determination of which Drug A doses and release rates, when dosed QD, will achieve plasma Drug A concentrations above 70 ng/ml and 50% or greater CETP inhibition for at least about 16 hr, on the 14^{th} day of dosing. It is assumed that the colonic bioavailability constant is zero. | | | |
|---|---|---|---|
| Dose (mg) | Time to Reach 80% Release (hr) | Plasma Drug A Conc. greater than 70 ng/ml for 16 hr? | Plasma CETP inhibition greater than 50% for 16 hr? |
| 10 | 2 | No | No |
| 10 | 4 | No | No |
| 10 | 6 | No | No |
| 10 | 8 | No | No |
| 30 | 2 | Yes | Yes |
| 30 | 4 | Yes | Yes |
| 30 | 6 | No | No |
| 30 | 8 | No | No |
| 40 | 2 | Yes | Yes |
| 40 | 4 | Yes | Yes |
| 40 | 6 | Yes | Yes |
| 40 | 8 | No | No |
| 40 | 12 | No | No |
| 60 | 2 | Yes | Yes |
| 60 | 4 | Yes | Yes |
| 60 | 6 | Yes | Yes |
| 60 | 8 | Yes | Yes |
| 60 | 12 | No | No |
| 60 | 18 | No | No |

The model was used to model BID dosing of doses from 10-60 mg Drug A, at various drug release rates from the CR dosage form. Table 13 shows which doses and release rates would achieve plasma Drug A concentrations above 160 ng/ml for at least about 12 hr. Table 13 also shows which doses and release rates would achieve 80% or greater inhibition of plasma CETP for at least about 12 hr. It is assumed that the colonic bioavailability constant is zero.

**Table 13**

| Determination of which Drug A doses and release rates, when dosed BID, will achieve plasma Drug A concentrations above 160 ng/ml and 80% or greater CETP inhibition for at least about 12 hr, on the 14^{th} day of dosing. It is assumed that the colonic bioavailability constant is zero. | | | |
|---|---|---|---|
| Dose (mg) | Time to Reach 80% Release (hr) | Plasma Drug A Conc. greater than 160 ng/ml for 12 hr? | Plasma CETP inhibition greater than 80% for 12 hr? |
| 10 | 2 | No | No |
| 10 | 4 | No | No |
| 10 | 6 | No | No |
| 10 | 8 | No | No |
| 30 | 2 | Yes | Yes |
| 30 | 4 | Yes | Yes |
| 30 | 6 | No | No |
| 30 | 8 | No | No |
| 40 | 2 | Yes | Yes |
| 40 | 4 | Yes | Yes |
| 40 | 6 | Yes | Yes |
| 40 | 8 | No | No |
| 60 | 2 | Yes | Yes |
| 60 | 4 | Yes | Yes |
| 60 | 6 | Yes | Yes |
| 60 | 8 | Yes | Yes |

The model was used to model QD dosing of doses from 10-120 mg Drug A, at various drug release rates from the CR dosage form. Table 14 shows which doses and release rates would achieve plasma Drug A concentrations above 160 ng/ml for at least about 16 hr. Table 14 also shows which doses and release rates would achieve 80% or greater inhibition of plasma CETP for at least about 16 hr. It is assumed that the colonic bioavailability constant is 30%.

**Table 14**

| Determination of which Drug A doses and release rates, when dosed QD, will achieve plasma Drug A concentrations above 160 ng/ml and 80% or greater CETP inhibition for at least about 16 hr, on the 14^{th} day of dosing. It is assumed that the colonic bioavailability constant is 30%. | | | |
|---|---|---|---|
| Dose (mg) | Time to Reach 80% Release (hr) | Plasma Drug A Conc. greater than 160 ng/ml for 16 hr? | Plasma CETP inhibition greater than 80% for 16 hr? |
| 10 | 2 | No | No |
| 10 | 4 | No | No |
| 10 | 6 | No | No |
| 10 | 8 | No | No |
| 30 | 2 | No | No |
| 30 | 4 | No | No |
| 30 | 6 | No | No |
| 30 | 8 | No | No |
| 30 | 12 | No | No |
| 40 | 2 | No | No |
| 40 | 4 | No | No |
| 40 | 6 | No | No |
| 40 | 8 | No | No |
| 40 | 12 | No | No |
| 60 | 2 | Yes | Yes |
| 60 | 4 | Yes | Yes |
| 60 | 6 | Yes | Yes |
| 60 | 8 | No | No |
| 60 | 12 | No | No |
| 60 | 18 | No | No |
| 80 | 2 | Yes | Yes |
| 80 | 12 | No | No |
| 100 | 12 | Yes | Yes |
| 100 | 18 | No | No |
| 120 | 6 | Yes | Yes |
| 120 | 8 | Yes | Yes |
| 120 | 12 | Yes | Yes |
| 120 | 18 | Yes | Yes |

The model was used to model QD dosing of doses from 10-120 mg Drug A, at various drug release rates from the CR dosage form. Table 15 shows which doses and release rates would achieve plasma Drug A concentrations above 160 ng/ml for at least about 16 hr. Table 15 also shows which doses and release rates would achieve 80% or greater inhibition of plasma CETP for at least about 16 hr. It is assumed that the colonic bioavailability constant is 80%.

**Table 15**

| Determination of which Drug A doses and release rates, when dosed QD, will achieve plasma Drug A concentrations above 160 ng/ml and 80% or greater CETP inhibition for at least about 16 hr, on the 14^{th} day of dosing. It is assumed that the colonic bioavailability constant is 80%. | | | |
|---|---|---|---|
| Dose (mg) | Time to Reach 80% Release (hr) | Plasma Drug A Conc. greater than 160 ng/ml for 16 hr? | Plasma CETP inhibition greater than 80% for 16 hr? |
| 10 | 2 | No | No |
| 10 | 4 | No | No |
| 10 | 6 | No | No |
| 10 | 8 | No | No |
| 30 | 2 | No | No |
| 30 | 4 | No | No |
| 30 | 6 | No | No |
| 30 | 8 | No | No |
| 40 | 2 | No | No |
| 40 | 4 | No | No |
| 40 | 6 | No | No |
| 40 | 8 | Yes | Yes |
| 60 | 2 | Yes | Yes |
| 60 | 4 | Yes | Yes |
| 60 | 6 | Yes | Yes |
| 60 | 8 | Yes | Yes |
| 120 | 6 | Yes | Yes |
| 120 | 8 | Yes | Yes |

The model was used to model QD dosing of doses from 10-120 mg Drug A, at various drug release rates from the CR dosage form. Table 16 shows which doses and release rates would achieve plasma Drug A concentrations above 160 ng/ml for at least about 16 hr. Table 16 also shows which doses and release rates would achieve 80% or greater inhibition of plasma CETP for at least about 16 hr. It is assumed that the colonic bioavailability constant is zero.

**Table 16**

| Determination of which Drug A doses and release rates, when dosed QD, will achieve plasma Drug A concentrations above 160 ng/ml and 80% or greater CETP inhibition for at least about 16 hr, on the 14^{th} day of dosing. It is assumed that the colonic bioavailability constant is zero. | | | |
|---|---|---|---|
| Dose (mg) | Time to Reach 80% Release (hr) | Plasma Drug A Conc. greater than 160 ng/ml for 16 hr? | Plasma CETP inhibition greater than 80% for 16 hr? |
| 10 | 2 | No | No |
| 10 | 4 | No | No |
| 10 | 6 | No | No |
| 10 | 8 | No | No |
| 30 | 2 | No | No |
| 30 | 4 | No | No |
| 30 | 6 | No | No |
| 30 | 8 | No | No |
| 40 | 2 | No | No |
| 40 | 4 | No | No |
| 40 | 6 | No | No |
| 40 | 8 | No | No |
| 60 | 2 | Yes | Yes |
| 60 | 4 | Yes | Yes |
| 60 | 6 | No | No |
| 60 | 8 | No | No |
| 80 | 2 | Yes | Yes |
| 80 | 4 | Yes | Yes |
| 80 | 6 | Yes | Yes |
| 80 | 8 | No | No |
| 120 | 2 | Yes | Yes |
| 120 | 4 | Yes | Yes |
| 120 | 6 | Yes | Yes |
| 120 | 8 | Yes | Yes |

The model was used to model BID dosing of doses from 5-60 mg Drug A, at various drug release rates from the CR dosage form. Table 17 shows which doses and release rates would achieve plasma Drug A concentrations above 325 ng/ml for at least about 12 hr. Table 17 also shows which doses and release rates would achieve 90% or greater inhibition of plasma CETP for at least about 12 hr. It is assumed that the colonic bioavailability constant is zero.

**Table 17**

| Determination of which Drug A doses and release rates, when dosed BID, will achieve plasma Drug A concentrations above 325 ng/ml and 90% or greater CETP inhibition for at least about 12 hr, on the 14^{th} day of dosing. It is assumed that the colonic bioavailability is zero. | | | |
|---|---|---|---|
| Dose (mg) | Time to Reach 80% Release (hr) | Plasma Drug A Conc. greater than 325 ng/ml for 12 hr? | Plasma CETP inhibition greater than 90% for 12 hr? |
| 5 | 2 | No | No |
| 10 | 2 | No | No |
| 30 | 2 | No | No |
| 40 | 2 | No | No |
| 60 | 2 | Yes | Yes |
| 60 | 4 | Yes | Yes |
| 60 | 6 | No | No |
| 60 | 8 | No | No |
| 120 | 4 | Yes | Yes |
| 120 | 6 | Yes | Yes |
| 120 | 8 | Yes | Yes |

### Example 10.

The modeling methodology of Example 9 was used to identify controlled release Drug A release rates for CR Drug A dosage forms which, when dosed QD in the fed state, result in plasma Drug A concentrations above 70 ng/ml and 50% or greater CETP inhibition for at least about 16 hr, on the 14^{th} day of dosing. It is assumed that the colonic bioavailability constant is 30%. To model fed state dosing, it is assumed that the CR dosage form exits the stomach (enters the duodenum) at 4 hr post dosing. Table 18 presents the results.

**Table 18.**

| Determination of which Drug A doses and release rates, when dosed QD to a fed human, will achieve plasma Drug A concentrations above 70 ng/ml and 50% or greater CETP inhibition for at least about 16 hr, on the 14^{th} day of dosing. It is assumed that the colonic bioavailability constant is 30%. | | | |
|---|---|---|---|
| Dose (mg) | Time to Reach 80% Release (hr) | Plasma Drug A Conc greater than 70 ng/ml for 16 hr? | Plasma CETP inhibition greater than 50% for 16 hr? |
| 10 | 2 | No | No |
| 10 | 4 | No | No |
| 10 | 6 | No | No |
| 10 | 8 | No | No |
| 30 | 2 | Yes | Yes |
| 30 | 4 | Yes | Yes |
| 30 | 6 | Yes | Yes |
| 30 | 8 | Yes | Yes |
| 40 | 2 | Yes | Yes |
| 40 | 4 | Yes | Yes |
| 40 | 6 | Yes | Yes |
| 40 | 8 | Yes | Yes |
| 40 | 12 | Yes | Yes |
| 60 | 2 | Yes | Yes |
| 60 | 4 | Yes | Yes |
| 60 | 6 | Yes | Yes |
| 60 | 8 | Yes | Yes |
| 60 | 12 | Yes | Yes |
| 60 | 18 | Yes | Yes |

### Example 11.

The modeling methodology of Example 9 was used to identify controlled release Drug A release rates for CR Drug A dosage forms which, when dosed QD or BID in the fasted state, result in plasma Drug A concentrations above 70 ng/ml and 50% or greater CETP inhibition for greater than about 30 minutes longer than that predicted for an immediate release Drug A dosage form at the same dose, on the 14^{th} day of dosing. It is assumed that the colonic bioavailability constant is 30%. Table 19 presents the results.

**Table 19.**

| Determination of which Drug A doses and release rates, when dosed QD or BID to a fasted human, will achieve plasma Drug A concentrations above 70 ng/ml and 50% or greater CETP inhibition for greater than about 30 minutes longer than that predicted for an immediate release Drug A dosage form at the same dose, on the 14^{th} day of dosing. It is assumed that the colonic bioavailability constant is 30%. | | | | |
|---|---|---|---|---|
| Dose (mg) | Formulation | Dosing Frequency | Time that Plasma Drug A concentration is above 70 ng/ml (and greater than 50% CETP inhibition) | Meets Criterion |
| 10 | IR | QD | Never | --- |
| 10 | 2 hr CR* | QD | 2 hr | Yes |
| 10 | IR | BID | 5 hr | --- |
| 10 | 2 hr CR | BID | 12 hr | Yes |
| 10 | 4 hr CR | BID | 12 hr | Yes |
| 10 | 6 hr CR | BID | 4 hr | Yes |
| 10 | 8 hr CR | BID | never | No |
| 30 | IR | QD | 20 hr | --- |
| 30 | 2 hr CR | QD | 24 hr | Yes |
| 30 | 4 hr CR | QD | 24 hr | Yes |
| 30 | 6 hr CR | QD | 21.5 hr | Yes |
| 30 | 8 hr CR | QD | 17.5 hr | No |

| | | | | |
|---|---|---|---|---|
| * 2 hr CR means 80% Drug A release in 2 hr. | | | | |

## Claims

1. A controlled release dosage form comprising:
(a) a solubility-enhanced form of a cholesteryl ester transfer protein inhibitor (CETPI) wherein CETPI is selected from the group consisting of the compounds of Formula XVI: 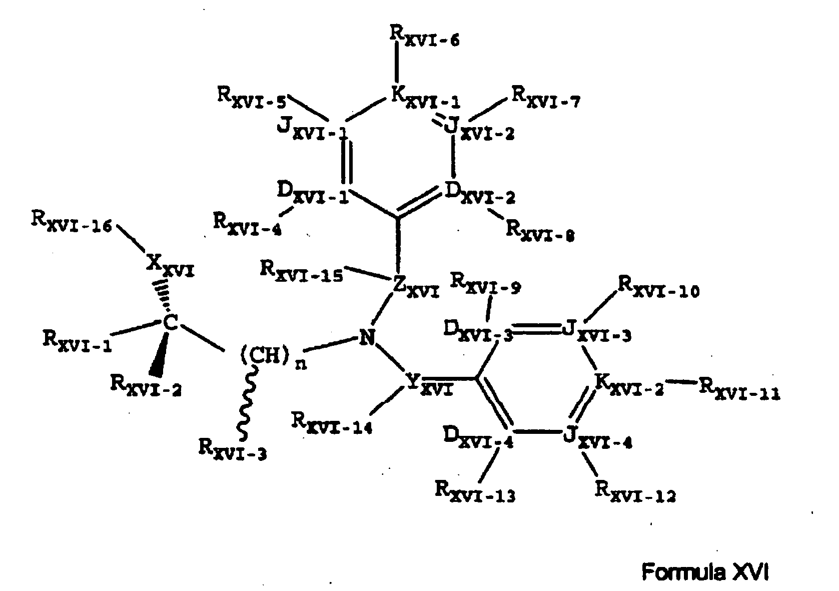 and pharmaceutically acceptable forms thereof, wherein:
n_{XVI} is an integer selected from 1 through 4;
X_{XVI} is oxy;
R_{XVI-1} is selected from the group consisting of haloalkyl, haloalkenyl, haloalkoxymethyl, and haloalkenyloxymethyl with the proviso that R_{XVI-1} has a higher Cahn-Ingold-Prelog stereochemical system ranking than both R_{XVI-2} and (CHR_{XVI-3})ₙ-N(A_{XVI})Q_{XVI} wherein A_{XVI} is Formula XVI-(II) and Q is Formula XVI-(III); 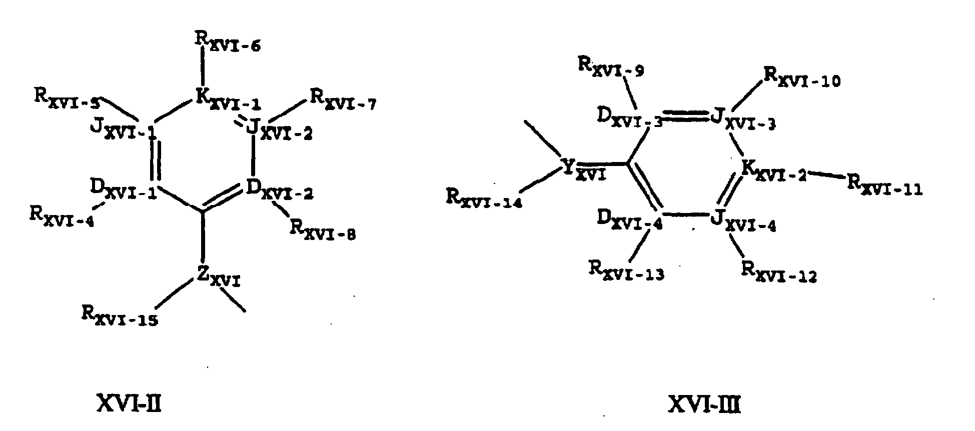
R_{XVI-16} is selected from the group consisting of hydrido, alkyl, acyl, aroyl, heteroaroyl, trialkylsilyl, and a spacer selected from the group consisting of a covalent single bond and a linear spacer moiety having a chain length of 1 to 4 atoms linked to the point of bonding of any aromatic substituent selected from the group consisting of R_{XVI-4}, R_{XVI-8}, R_{XVI-9}, and R_{XVI-13} to form a heterocyclyl ring having from 5 through 10 contiguous members;
D_{XVI-1}, D_{XVI-2}, J_{XVI-1}, J_{XVI-2} and K_{XVI-1} are independently selected from the group consisting of C, N, O, S and covalent bond with the provisos that no more than one of D_{XYI-1}, D_{XVI-2}, J_{XVI-1}, J_{XVI-2} and K_{XVI-1} is a covalent bond, no more than one D_{XVI-1}, D_{XVI-2}, J_{XVI-1}, J_{XVI-2} and K_{XVI-1} is be O, no more than one of D_{XVI-1}, D_{XVI-2}, J_{XVI-1}, J_{XVI-2} and K_{XVI-1} is S, one of D_{XVI-1}, D_{XVI-2}, J_{XVI-1}, J_{XVI-2} and K_{XVI-1} must be a covalent bond when two of D_{XVI-1}, D_{XVI-2}, J_{XVI-1}, J_{XVI-2} and K_{XVI-1} are O and S, and no more than four of D_{XVI-1}, D_{XVI-2}, J_{XVI-1}, J_{XVI-2} and K_{XVI-1} is N;
D_{XVI-3}, D_{XVI-4}, J_{XVI-3}, J_{XVI-4} and K_{XVI-2} are independently selected from the group consisting of C, N, O, S and covalent bond with the provisos that no more than one is a covalent bond, no more than one of D_{XVI-3}, D_{XVI-4}, J_{XVI-3}, J_{XVI-4} and K_{XVI-2} is O, no more than one of D_{XVI-3}, D_{XVI-4}, J_{XVI-3}, J_{XVI-4} and K_{XVI-2} is S, no more than two of D_{XVI-3}, D_{XVI-4}, J_{XVI-3}, J_{XVI-4} and K_{XVI-2} is 0 and S, one of D_{XVI-3}, D_{XVI-4}, J_{XVI-3}, J_{XVI-4} and K_{XVI-2} must be a covalent bond when two of D_{XVI-3}, D_{XVI-4}, J_{XVI-3}, J_{XVI-4} and K_{XVI-2} are O and S, and no more than four of D_{XVI-3}, D_{XVI-4}, J_{XVI-3}, J_{XVI-4} and K_{XVI-2} are N;
R_{XVI-2} is selected from the group consisting of hydrido, aryl, aralkyl, alkyl, alkenyl, alkenyloxyalkyl, haloalkyl, haloalkenyl, halocycloalkyl, haloalkoxy, haloalkoxyalkyl, haloalkenyloxyalkyl, halocycloalkoxy, halocycloalkoxyalkyl, perhaloaryl, perhaloaralkyl, perhaloaryloxyalkyl, heteroaryl, dicyanoalkyl, and carboalkoxycyanoalkyl, with the proviso that R_{XVI-2} has a lower Cahn-Ingold-Prelog system ranking than both R_{XVI-1} and (CHR_{XVI-3})ₙ-N(A_{XVI})Q_{XVI};
R_{XVI-3} is selected from the group consisting of hydrido, hydroxy, cyano, aryl, aralkyl, acyl, alkoxy, alkyl, alkenyl, alkoxyalkyl, heteroaryl, alkenyloxyalkyl, haloalkyl, haloalkenyl, haloalkoxy, haloalkoxyalkyl, haloalkenyloxyalkyl, monocyanoalkyl, dicyanoalkyl, carboxamide, and carboxamidoalkyl, with the provisos that (CHR_{XVI-3})ₙ-N(A_{XVI})Q_{XVI} has a lower Cahn-Ingold-Prolog stereochemical system ranking than R_{XVI-1} and a higher Cahn-Ingold-Prelog stereochemical system ranking than R_{XVI-2};
Y_{XVI} is selected from a group consisting of a covalent single bond, (C(R_{XVI-14})₂)_{q} wherein q is an integer selected from 1 and 2 and (CH(R_{XV1-14}))_{g}-W_{XVI}-(CH(R_{XVI-14}))ₚ wherein g and p are integers independently selected from 0 and 1;
R_{XVI-14} is selected from the group consisting of hydrido, hydroxy, cyano, hydroxyalkyl, acyl, alkoxy, alkyl, alkenyl, alkynyl, alkoxyalkyl, haloalkyl, haloalkenyl, haloalkoxy, haloalkoxyalkyl, haloalkenyloxyalkyl, monocarboalkoxyalkyl, monocyanoalkyl, dicyanoalkyl, carboalkoxycyanoalkyl, carboalkoxy, carboxamide, and carboxamidoalkyl;
Z_{XVI} is selected from a group consisting of a covalent single bond, (C(R_{XVI-16})₂)_{q}, wherein q is an integer selected from 1 and 2, and (CH(R_{XVI-15}))-W_{XVI}-(CH(R_{XVI-15}))ₖ wherein j and k are integers independently selected from 0 and 1;
W_{XVI} is selected from the group consisting of O, C(O), C(S),C(O)N(R_{XVI-14}), C(S)N(R_{XVI-14}),(R_{XVI-14})NC(O), (R_{XVI-14})NC(S), S, S(O), S(O)₂, S(O)₂N(R_{XVI-14}), (R_{XVI-14})NS(O)₂, and N(R_{XVI-14}) with the proviso that R_{XVI-14} is other than cyano;
R_{XVI-15} is selected, from the group consisting of hydrido, cyano, hydroxyalkyl, acyl, alkoxy, alkyl, alkenyl, alkynyl, alkoxyalkyl, haloalkyl, haloalkenyl, haloalkoxy, haloalkoxyalkyl, haloalkenyloxyalkyl, monocarboalkoxyalkyl, monocyanoalkyl, dicyanoalkyl, carboalkoxycyanoalkyl, carboalkoxy, carboxamide, and carboxamidoalkyl;
R_{XVI-4}, R_{XVI-5}, R_{XVI-6}, R_{XVI-7}, R_{XVI-8}, R_{XVI-9}, R_{XVI-10}, R_{XVI-11}, R_{XVI-12}, and R_{XVI-13} are independently selected from the group consisting of hydrido, carboxy, heteroaralkylthio, heteroaralkoxy, cycloalkylamino, acylalkyl, acylalkoxy, aroylalkoxy, heterocyclyloxy, aralkylaryl, aralkyl, aralkenyl, aralkynyl, heterocyclyl, perhaloaralkyl, aralkylsulfonyl, aralkylsulfonylalkyl, aralkylsulfinyl, aralkylsulfinylalkyl, halocycloalkyl, halocycloalkenyl, cycloalkylsulfinyl, cycloalkylsulfinylalkyl, cycloalkylsulfonyl, cycloalkylsulfonylalkyl, heteroarylamino, N-heteroarylamino-N-alkylamino, heteroaralkyl, heteroarylaminoalkyl, haloalkylthio, alkanoyloxy, alkoxy, alkoxyalkyl, haloalkoxylalkyl, heteroaralkoxy, cycloalkoxy, cycloalkenyloxy, cycloalkoxyalkyl, cycloalkylalkoxy, cycloalkenyloxyalkyl, cycloalkylenedioxy, halocycloalkoxy, halocycloalkoxyalkyl, halocycloalkenyloxy, halocycloalkanyloxyalkyl, hydroxy, amino, thio, nitro, lower alkylamino, alkylthio, alkylthioalkyl, arylamino, aralkylamino, arylthio, arylthioalkyl, heteroaralkoxyalkyl, alkylsulfinyl, alkylsulfinylalkyl, arylsulfinylalkyl, aryisulfonylalkyl, heteroarylsulfinylalkyl, heteroarylsulfonylalkyl, alkylsulfonyl, alkylsulfonylalkyl, haloalkylsulfinylalkyl, haloalkylsulfonylalkyl, alkylsulfonamido, alkylaminosulfonyl, amidosulfonyl, monoalkyl amidosulfonyl, dialkyl, amidosulfonyl, monoarylamidosulfonyl, arylsulfonamido, diarylamidosulfonyl, monoalkyl monoaryl amidosulfonyl, arylsulfinyl, arylsulfonyl, heteroarylthio, heteroarylsulfinyl, heteroarylsulfonyl, heterocyclylsulfonyl, heterocyclylthio, alkanoyl, alkenoyl, aroyl, heteroaroyl, aralkanoyl, heteroaralkanoyl, haloalkanoyl, alkyl, alkenyl, alkynyl, alkenyloxy, alkenyloxyalky, alkylenedioxy, haloalkylenedioxy, cycloalkyl, cycloalkylalkanoyl, cycloalkenyl, lower cycloalkylalkyl, lower cycloalkenylalkyl, halo, haloalkyl, haloalkenyl, haloalkoxy, hydroxyhaloalkyl, hydroxyaralkyl, hydroxyalkyl, hydoxyheteroaralkyl, haloalkoxyalkyl, aryl, heteroaralkynyl, aryloxy, aralkoxy, aryloxyalkyl, saturated heterocyclyl, partially saturated heterocyclyl, heteroaryl, heteroaryloxy, heteroaryloxyalkyl, arylalkenyl, heteroarylalkenyl, carboxyalkyl, carboakoxy, alkoxycarboxamido, alkylamidocarbonylamido, arylamidocarbonylamido, carboalkoxyalkyl, carboalkoxyalkenyl, carboaralkoxy, carboxamido, carboxamidoalkyl, cyano, carbohaloalkoxy, phosphono, phosphonoalkyl, dlaralkoxyphosphono, and diaralkoxyphosphonoalkyl with the proviso that R_{XVI-4}, R_{XVI-5}, R_{XVI-6}, R_{XVI-7}, R_{XVI-8}, R_{XVI-9}, R_{XVI-10}, R_{XVI-11}, R_{XVI-12}, and R_{XVI-13} are each independently selected to maintain the tetravalent nature of carbon, trivalent nature of nitrogen, the divalent nature of sulfur, and the divalent nature of oxygen;
R_{XVI-4} and R_{XVI-5}, R_{XVI-5} and R_{XVI-6}, R_{XVI-6} and R_{XVI-7}, R_{XVI-7} and R_{XVI-8}, R_{XVI-9} and R_{XVI-10}, R_{XVI-10} and R_{XVI-11}, R_{XVI-11} and R_{XVI-12}, and R_{XVI-12} and R_{XIV-13} are independently selected to form spacer pairs wherein a spacer pair is taken together to form a linear moiety having from 3 through 6 contiguous atoms connecting the points of bonding of said spacer pair members to form a ring selected from the group consisting of a cycloalkenyl ring having 5 through 8 contiguous members, a partially saturated heterocyclyl ring having 5 through 8 contiguous members, a heteroaryl ring having 5 through 6 contiguous members, and an aryl with the provisos that no more than one of the group consisting of spacer pairs R_{XVI-4} and R_{XVI-5}, R_{XVI-5} and R_{XVI-6}, R_{XVI-6} and R_{XVI-7}, and R_{XVI-7} and R_{XVI-8} is used at the same time and that no more than one of the group consisting of spacer pairs R_{XIV-9} and R_{XVI-10}, R_{XVI-10} and R_{XVI-11}, R_{XVI-11} and R_{XVI-12}, and R_{XVI-12} and R_{XVI-13} can be used at the same time; R_{XVI-4} and R_{XVI-9}, R_{XVI-4} and R_{XVI-13}, R_{XVI-8} and R_{XVI-9}, and R_{XVI-8} and R_{XVI-13} is independently selected to form a spacer pair wherein said spacer pair is taken together to form a linear moiety wherein said linear moiety forms a ring selected from the group consisting of a partially saturated heterocyclyl ring having from 5 through 8 contiguous members and a heteroaryl ring having from 5 through 6 contiguous members with the proviso that no more than one group consisting of spacer pairs R_{XVI-4} and R_{XVI-9}, R_{XVI-4} and R_{XVI-13}, R_{XVI-8} and R_{XVI-9}, and R_{XVI-8} and R_{XVI-13} is used at the same time; and
(b) a controlled release means for delivering said CETPI;
wherein following administration to an *in vivo* use environment, said controlled release dosage form provides at least one of:
(i) at least 50% inhibition of plasma cholesteryl ester transfer protein for at least 12 hours;
(ii) a maximum drug concentration in the blood that is less than or equal to 80% of the maximum drug concentration in the blood provided by an immediate release dosage form consisting of the same amount of the solubility-enhanced form of said CETPI;
(iii) a mean HDL cholesterol level after dosing for 8 weeks that is at least about 1.2-fold that obtained prior to dosing; and
(iv) a mean LDL cholesterol level after dosing for 8 weeks that is less than or equal to 90% that obtained prior to dosing.

2. A controlled release dosage form comprising:
(a) a solubility-enhanced form of a cholesteryl ester transfer protein inhibitor (CETPI)
wherein CETPI is selected from the group consisting of the compounds of Formula XVI as defined in claim 1 ; and
(b) a controlled release means for delivering said CETPI;
wherein following administration to a use environment, said controlled release dosage form releases at least 80 wt% of said CETPI in greater than about 2 hours.

3. The controlled release dosage form of claim 1 or 2 wherein said CETPI is (2R)-3-[[3-(4-chloro-3-ethylphenoxy)phenyl][[3-(1,1,2,2-tetrafluoroethoxy)phenyl]-methyl]amino]-1,1,1-trifluoro-2-propanol.

4. The controlled release dosage form of claim 1, 2 or 3 further comprising a concentration-enhancing polymer.

5. The controlled release dosage form of claim 4 wherein said solubility-enhanced form is a solid amorphous dispersion of said CETPI in said concentration-enhancing polymer.

6. The controlled release dosage form of claim 1, 2 or 3 further comprising a means to prevent precipitation of said CETPI in said use environment.

7. The controlled release dosage form of claim 1, 2 or 3 wherein said solubility-enhanced form provides, when administered alone to a use environment,
a) a maximum drug concentration that is at least 2-fold that provided by a control composition consisting of an equivalent amount of CEPTI in crystalline form alone; or
b) a concentration versus time area under the curve (AUC), for any period of at least 90 minutes between the time of introduction into the use environment and about 270 minutes following introduction to the use environment that is at least about 1.25-fold that of a control composition consisting of an equivalent quantity of CETPI in crystalline form alone.

8. The controlled release dosage form of claim 1, 2 or 3 wherein said dosage form is selected from the group consisting of a tablet, a capsule, and a multiparticulate.

9. The controlled release dosage form of claim 1, 2 or 3 wherein said dosage form, when administered to a human, exhibits at least one of :
a) a rate of release into the gastrointestinal tract of said human that results in at least about 50% inhibition of plasma CETP in said human for a time period of at least about 12 hours from the time of administration; or
b) 50% inhibition at a dose that is lower than an immediate release dose that results in equivalent inhibition.

10. The controlled release dosage form of claim 1, 2 or 3 wherein said dosage form, when administered to a human, provides a Tₘₐₓ in the blood that is at least 1.25-fold that provided by an immediate release dosage form consisting of an equivalent amount of said CETPI in the same solubility-enhanced form.

11. The controlled release dosage form of claim 1, 2 or 3 wherein said dosage form, when administered to a human, provides a Cₘₐₓ in the blood that is less than or equal to 80% that provided by an immediate release dosage form consisting of an equivalent amount of said CETPI in the same solubility-enhanced form.

12. The controlled release dosage form of claim 1, 2 or 3 wherein said dosage form, when administered to a human, provides a relative bioavailability of at least 1.25 relative to an immediate release dosage form consisting of an equivalent amount of said CETPI in the same solubility-enhanced form.

13. The controlled release dosage form of claim 1, 2 or 3 wherein said dosage form following administration to an *in vitro* use environment has a release rate of less than 40wt%/hour.

14. The controlled release dosage form of claim 1, 2 or 3 wherein said dosage form, when administered to a human, has a rate of release into the gastrointestinal tract of said human that results in a drug plasma concentration in said human in excess of about 70ng/ml for a time period of at least about 12 hours from administration.

## Patentansprüche

1. Dosierungsform zur kontrollierten Freisetzung, umfassend:
(a) eine bezüglich der Löslichkeit verstärkte Form eines Cholesterylestertransferprotein-Inhibitors (CETPI), worin CETPI ausgewählt ist aus der Gruppe, bestehend aus den Verbindungen der Formel XVI: 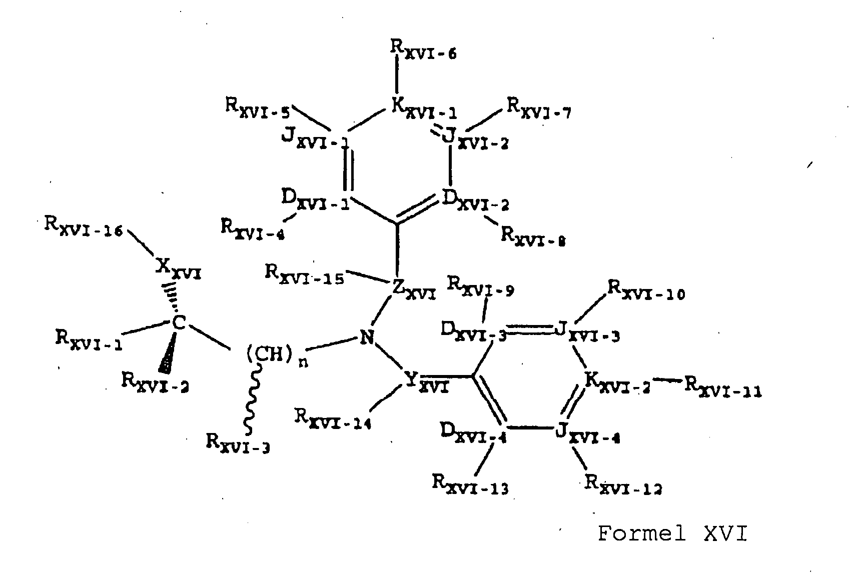 und pharmazeutisch verträglichen Formen davon, worin:
n_{XVI} eine ganze Zahl, ausgewählt aus 1 bis 4, ist;
X_{XVI} Oxy ist;
R_{XVI-1} ausgewählt ist aus der Gruppe, bestehend aus Halogenalkyl, Halogenalkenyl, Halogenalkoxymethyl und Halogenalkenyloxymethyl, mit der Maßgabe, dass R_{XVI-1} ein höheres Ranking im Cahn-Ingold-Prelog-Stereochemiesystem hat als sowohl R_{XVI-2} als auch (CHR_{XVI-3})ₙN(A_{XVI})Q_{XVI}, worin A_{XVI} die Formel XVI-(II) hat und Q die Formel XVI-(III) hat; 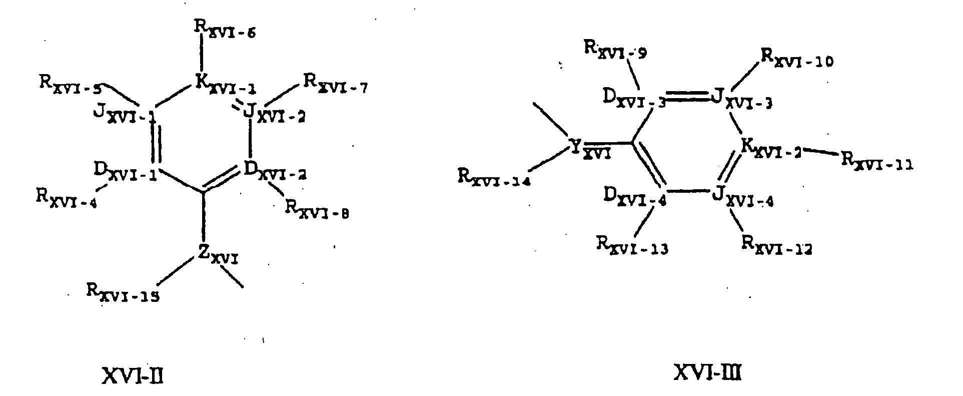
R_{XVI-16} ausgewählt ist aus der Gruppe, bestehend aus Hydrido, Alkyl, Acyl, Aroyl, Heteroaroyl, Trialkylsilyl und einem Spacer, ausgewählt aus der Gruppe, bestehend aus einer kovalenten Einfachbindung und einer linearen Spacer-Gruppierung mit einer Kettenlänge von 1 bis 4 Atomen, gebunden an den Bindungspunkt eines aromatischen Substituenten, ausgewählt aus der Gruppe, bestehend aus R_{XVI-4}, R_{XVI-5}, R_{XVI-9} und R_{XVI-13}, unter Bildung eines Heterocyclylrings mit 5 bis 10 fortlaufenden Gliedern;
D_{XVI-1}, D_{XVI-2}, J_{XVI-1}, J_{XVI-2} und K_{XVI-1} unabhängig ausgewählt sind aus der Gruppe, bestehend aus C, N, 0, S und kovalenter Bindung, mit den Maßgaben, dass nicht mehr als eines von D_{XVI-1}, D_{XVI-2}, J_{XVI-1}, J_{XVI-2} und K_{XVI-1} eine kovalente Bindung ist, nicht mehr als eines von D_{XVI-1}, D_{XVI-2}, J_{XVI-1}, J_{XVI-2} und K_{XVI-1} O ist, nicht mehr als eines von D_{XVI-1}, D_{XVI-2}, J_{XVI-1}, J_{XVI-2} und K_{XVI-1} S ist, eines von D_{XVI-1}, D_{XVI-2}, J_{XVI-1}, J_{XVI-2} und K_{XVI-1} eine kovalente Bindung sein muss, wenn zwei von D_{XVI-1}, D_{XVI-2}, J_{XVI-1}, J_{XVI-2} und K_{XVI-1} O und S sind und nicht mehr als vier von D_{XVI-1}, D_{XVI-2}, J_{XVI-1}, J_{XVI-2} und K_{XVI-1} N sind;
D_{XVI-3}, D_{XVI-4}, J_{XVI-3}, J_{XVI-4} und K_{XVI-2} unabhängig ausgewählt sind aus der Gruppe, bestehend aus C, N, 0, S und kovalenter Bindung, mit den Maßgaben, dass nicht mehr als eines eine kovalente Bindung ist, nicht mehr als eines von D_{XVI-3}, D_{XVI-4}, J_{XVI-3}, J_{XVI-4} und K_{XVI-2} 0 ist, nicht mehr als eines von D_{XVI-3}, D_{XVI-4}, J_{XVI-3}, J_{XVI-4} und K_{XVI-2} S ist, nicht mehr als zwei von D_{XVI-3}, D_{XVI-4}, J_{XVI-3}, J_{XVI-4} und K_{XVI-2} 0 und S sind, eines von D_{XVI-3}, D_{XVI-4}, J_{XVI-3}, J_{XVI-4} und K_{XVI-2} eine kovalente Bindung sein muss, wenn zwei von D_{XVI-3}, D_{XVI-4}, J_{XVI-3}, J_{XVI-4} und K_{XVI-2} O und S sind und nicht mehr als vier von D_{XVI-3}, D_{XVI-4}, J_{XVI-3}, J_{XVI-4} und K_{XVI-2} N sind;
R_{XVI-2} ausgewählt ist der Gruppe, bestehend aus Hydrido, Aryl, Aralkyl, Alkyl, Alkenyl, Alkenyloxyalkyl, Halogenalkyl, Halogenalkenyl, Halogencycloalkyl, Halogenalkoxy, Halogenalkoxyalkyl, Halogenalkenyloxyalkyl, Halogencycloalkoxy, Halogencycloalkoxyalkyl, Perhalogenaryl, Perhalogenaralkyl, Perhalogenaryloxyalkyl, Heteroaryl, Dicyanoalkyl und Carboalkoxycyanoalkyl, mit der Maßgabe, dass R_{XVI-2} ein niedrigeres Ranking im Cahn-Ingold-Prelog-System hat als sowohl R_{XVI-1} als auch (CHR_{XVI-3})ₙ-N(A_{XVI})Q_{XVI};
R_{XVI-3} ausgewählt ist aus der Gruppe, bestehend aus Hydrido, Hydroxy, Cyano, Aryl, Aralkyl, Acyl, Alkoxy, Alkyl, Alkenyl, Alkoxyalkyl, Heteroaryl, Alkenyloxyalkyl, Halogenalkyl, Halogenalkenyl, Halogenalkoxy, Halogenalkoxyalkyl, Halogenalkenyloxyalkyl, Monocyanoalkyl, Dicyanoalkyl, Carboxamid und Carboxamidoalkyl, mit den Maßgaben, dass (CHR_{XVI-3})ₙ-N(A_{XVI})Q_{XVI} ein niedrigeres Ranking im Cahn-Ingold-Prelog-Stereochemiesystem hat als R_{XVI-1} und ein höheres Ranking im Cahn-Ingold-Prelog-Stereochemiesystem als R_{XVI-2} hat;
Y_{XVI} ausgewählt ist aus einer Gruppe, bestehend aus einer kovalenten Einfachbindung, (C(R_{XVI-14})₂)_{q}, worin q eine ganze Zahl, ausgewählt aus 1 und 2, ist, und (CH(R_{XVI-14}))_{g}-W_{XVI}-(CH(R_{XVI-14}))ₚ, worin g und p ganze Zahlen sind, die unabhängig ausgewählt sind aus 0 und 1;
R_{XVI-14} ausgewählt ist aus der Gruppe, bestehend aus Hydrido, Hydroxy, Cyano, Hydroxyalkyl, Acyl, Alkoxy, Alkyl, Alkenyl, Alkinyl, Alkoxyalkyl, Halogenalkyl, Halogenalkenyl, Halogenalkoxy, Halogenalkoxyalkyl, Halogenalkenyloxyalkyl, Monocarboalkoxyalkyl, Monocyanoalkyl, Dicyanoalkyl, Carboalkoxycyanoalkyl, Carboalkoxy, Carboxamid und Carboxamidoalkyl;
Z_{XVI} ausgewählt ist aus einer Gruppe, bestehend aus einer kovalenten Einfachbindung, (C(R_{XVI-15})₂)_{q}, worin q eine ganze Zahl, ausgewählt aus 1 und 2, ist, und (CH(R_{XVI-15}))ⱼ-W_{XVI}-(CH(R_{XVI-15}))ₖ, worin j und k ganze Zahlen sind, die unabhängig ausgewählt sind aus 0 und 1;
W_{XVI} ausgewählt ist aus der Gruppe, bestehend aus 0, C(O), C(S), C(O)N(R_{XVI-14}), C(S)N(R_{XVI-14}), (R_{XVI-14})NC(O), (R_{XVI-14})NC(S), S, S(O), S(O)₂, S(O)₂N(R_{XVI-14}), (R_{XVI-14})NS(O)₂ und N(R_{XVI-14}) mit der Maßgabe, dass R_{XVI-14} eine andere Bedeutung als Cyano hat;
P_{XVI-15} ausgewählt ist aus der Gruppe, bestehend aus Hydrido, Cyano, Hydroxyalkyl, Acyl, Alkoxy, Alkyl, Alkenyl, Alkinyl, Alkoxyalkyl, Halogenalkyl, Halogenalkenyl, Halogenalkoxy, Halogenalkoxyalkyl, Halogenalkenyloxyalkyl, Monocarboalkoxyalkyl, Monocyanoalkyl, Dicyanoalkyl, Carboalkoxycyanoalkyl, Carboalkoxy, Carboxamid und Carboxamidoalkyl;
R_{XVI-4}, R_{XVI-5}, R_{XVI-6}, R_{XVI-7}, R_{XVI-8}, R_{XVI-9}, R_{XVI-10}, R_{XVI-11}, R_{XVI-12} und R_{XVI-13} unabhängig ausgewählt sind aus der Gruppe, bestehend aus Hydrido, Carboxy, Heteroaralkylthio, Heteroaralkoxy, Cycloalkylamino, Acylalkyl, Acylalkoxy, Aroylalkoxy, Heterocyclyloxy, Aralkylaryl, Aralkyl, Aralkenyl, Aralkinyl, Heterocyclyl, Perhalogenaralkyl, Aralkylsulfonyl, Aralkylsulfonylalkyl, Aralkylsulfinyl, Aralkylsulfinylalkyl, Halogencycloalkyl, Halogencycloalkenyl, Cycloalkylsulfinyl, Cycloalkylsulfinylalkyl, Cycloalkylsulfonyl, Cycloalkylsulfonylalkyl, Heteroarylamino, N-Heteroarylamino-N-alkylamino, Heteroaralkyl, Heteroarylaminoalkyl, Halogenalkylthio, Alkanoyloxy, Alkoxy, Alkoxyalkyl, Halogenalkoxylalkyl, Heteroaralkoxy, Cycloalkoxy, Cycloalkenyloxy, Cycloalkoxyalkyl, Cycloalkylalkoxy, Cycloalkenyloxyalkyl, Cycloalkylendioxy, Halogencycloalkoxy, Halogencycloalkoxyalkyl, Halogencycloalkenyloxy, Halogencycloalkenyloxyalkyl, Hydroxy, Amino, Thio, Nitro, Niederalkylamino, Alkylthio, Alkylthioalkyl, Arylamino, Aralkylamino, Arylthio, Arylthioalkyl, Heteroaralkoxyalkyl, Alkylsulfinyl, Alkylsulfinylalkyl, Arylsulfinylalkyl, Arylsulfonylalkyl, Heteroarylsulfinylalkyl, Heteroarylsulfonylalkyl, Alkylsulfonyl, Alkylsulfonylalkyl, Halogenalkylsulfinylalkyl, Halogenalkylsulfonylalkyl, Alkylsulfonamido, Alkylaminosulfonyl, Amidosulfonyl, Monoalkylamidosulfonyl, Dialkyl, Amidosulfonyl, Monoarylamidosulfonyl, Arylsulfonamido, Diarylamidosulfonyl, Monoalkylmonoarylamidosulfonyl, Arylsulfinyl, Arylsulfonyl, Heteroarylthio, Heteroarylsulfinyl, Heteroarylsulfonyl, Heterocyclylsulfonyl, Heterocyclylthio, Alkanoyl, Alkenoyl, Aroyl, Heteroaroyl, Aralkanoyl, Heteroaralkanoyl, Halogenalkanoyl, Alkyl, Alkenyl, Alkinyl, Alkenyloxy, Alkenyloxyalkyl, Alkylendioxy, Halogenalkylendioxy, Cycloalkyl, Cycloalkylalkanoyl, Cycloalkenyl, Niedercycloalkylalkyl, Niedercycloalkenylalkyl, Halogen, Halogenalkyl, Halogenalkenyl, Halogenalkoxy, Hydroxyhalogenalkyl, Hydroxyaralkyl, Hydroxyalkyl, Hydroxyheteroaralkyl, Halogenalkoxyalkyl, Aryl, Heteroaralkinyl, Aryloxy, Aralkoxy, Aryloxyalkyl, gesättigtem Heterocyclyl, teilweise gesättigtem Heterocyclyl, Heteroaryl, Heteroaryloxy, Heteroaryloxyalkyl, Arylalkenyl, Heteroarylalkenyl, Carboxyalkyl, Carboalkoxy, Alkoxycarboxamido, Alkylamidocarbonylamido, Arylamidocarbonylamido, Carboalkoxyalkyl, Carboalkoxyalkenyl, Carboaralkoxy, Carboxamido, Carboxamidoalkyl, Cyano, Carbohalogenalkoxy, Phosphono, Phosphonoalkyl, Diaralkoxyphosphono und Diaralkoxyphosphonoalkyl, mit der Maßgabe, dass R_{XVI-4}, R_{XVI-5}, R_{XVI-6}, R_{XVI-7}, R_{XVI-8}, R_{XVI-9}, R_{XVI-10}, R_{XVI-11}, R_{XVI-12} und R_{XVI-13} jeweils unabhängig ausgewählt sind, um die tetravalente Natur von Kohlenstoff, die trivalente Natur von Stickstoff, die divalente Natur von Schwefel und die divalente Natur von Sauerstoff aufrechtzuerhalten;
R_{XVI-4} und R_{XVI-5}, R_{XVI-5} und R_{XVI-6}, R_{XVI-6} und R_{XVI-7}, R_{XVI-7} und R_{XVI-8}, R_{XVI-9} und R_{XVI-10}, R_{XVI-10} und R_{XVI-11}, R_{XVI-11} und R_{XVI-12} und R_{XVI-12} und R_{XVI-13} unabhängig ausgewählt sind, um Spacer-Paare zu bilden, wobei ein Spacer-Paar zusammengenommen wird, um eine lineare Gruppierung zu bilden, die 3 bis 6 fortlaufende Atome hat, die die Bindungspunkte der Spacer-Paarglieder unter Bildung eines Rings verbinden, der ausgewählt ist aus der Gruppe, bestehend aus einem Cycloalkenylring mit 5 bis 8 fortlaufenden Gliedern, einem partiell gesättigten Heterocyclylring mit 5 bis 8 fortlaufenden Gliedern, einem Heteroarylring mit 5 bis 6 fortlaufenden Gliedern und einem Aryl, mit den Maßgaben, dass nicht mehr als eine der Gruppe, bestehend aus den Spacer-Paaren R_{XVI-4} und R_{XVI-5}, R_{XVI-5} und R_{XVI-6}, R_{XVI-6} und R_{XVI-7} und R_{XVI-7} und R_{XVI-8}, gleichzeitig verwendet wird und
dass nicht mehr als eines der Gruppe, bestehend aus den Spacer-Paaren R_{XVI-9} und R_{XVI-10}, R_{XVI-10} und R_{XVI-11}, R_{XVI-11} und R_{XVI-12} und R_{XVI-12} und R_{XVI-13}, gleichzeitig verwendet werden kann;
R_{XVI-4} und R_{XVI-9}, R_{XVI-4} und R_{XVI-13}, R_{XVI-8} und R_{XVI-9} und R_{XVI-8} und R_{XVI-13} unabhängig ausgewählt sind, um ein Spacer-Paar zu bilden, wobei das Spacer-Paar zusammengenommen wird, um eine lineare Gruppierung zu bilden, wobei die lineare Gruppierung einen Ring bildet, der ausgewählt ist aus der Gruppe, bestehend aus einem partiell gesättigten Heterocyclylring mit 5 bis 8 fortlaufenden Gliedern und einem Heteroarylring mit 5 bis 6 fortlaufenden Gliedern, mit der Maßgabe, dass nicht mehr als eine Gruppe, bestehend aus den Spacer-Paaren R_{XVI-4} und R_{XVI-9}, R_{XVI-4} und R_{XVI-13}, R_{XVI-8} und P_{XVI-9} und R_{XVI-8} und R_{XVI-13}, gleichzeitig verwendet wird; und
(b) ein Mittel zur kontrollierten Freisetzung zur Abgabe des CETPI;
wobei die Dosierungsform zur kontrollierten Freisetzung nach Verabreichung an eine In-vivo-Verwendungsumgebung wenigstens eines der folgenden bereitstellt:
(i) wenigstens 50% Inhibierung von Plasmacholesterylestertransferprotein für wenigstens 12 Stunden;
(ii) eine maximale Wirkstoffkonzentration in Blut, die kleiner als oder gleich 80% der maximalen Wirkstoffkonzentration in Blut, die durch eine Dosierungsform mit sofortiger Freisetzung bereitgestellt wird, welche aus derselben Menge der bezüglich der Löslichkeit verstärkten Form des CETPI besteht, ist;
(iii) eine mittlere HDL-Cholesterinkonzentration nach Dosierung für 8 Wochen, die wenigstens etwa das 1,2-Fache derjenigen ist, die vor Dosierung erhalten wird, und
(iv) eine mittlere LDL-Cholesterinkonzentratioh nach Dosierung für 8 Wochen, die geringer als oder gleich 90% derjenigen ist, die vor Dosierung erhalten wird.

2. Dosierungsform zur kontrollierten Freisetzung, umfassend:
(a) eine bezüglich der Löslichkeit verstärkte Form eines Cholesterylestertransferprotein-Inhibitors (CETPI), wobei CETPI ausgewählt ist aus der Gruppe, bestehend aus den Verbindungen der Formel XVI, wie sie in Anspruch 1 definiert ist, und
(b) ein Mittel zur kontrollierten Freisetzung zur Abgabe des CETPI;
wobei die Dosierungsform zur kontrollierten Freisetzung nach Verabreichung an eine Verwendungsumgebung wenigstens 80 Gew.-% des CETPI in mehr als etwa 2 Stunden freisetzt.

3. Dosierungsform zur kontrollierten Freisetzung nach Anspruch 1 oder 2, wobei das CETPI (2R)-3-[[3-(4-Chlor-3-ethylphenoxy)phenyl][[3-(1,1,2,2-tetrafluorethoxy)phenyl]-methyl]amino]-1,1,1-trifluor-2-propanol ist.

4. Dosierungsform zur kontrollierten Freisetzung nach Anspruch 1, 2 oder 3, die außerdem ein die Konzentration erhöhendes Polymer umfasst.

5. Dosierungsform zur kontrollierten Freisetzung nach Anspruch 4, wobei die bezüglich der Löslichkeit verstärkte Form eine feste amorphe Dispersion des CETPI in dem die Konzentration erhöhenden Polymer ist.

6. Dosierungsform zur kontrollierten Freisetzung nach Anspruch 1, 2 oder 3, die außerdem ein Mittel umfasst, um eine Präzipitation des CETPI in der Verwendungsumgebung zu verhindern.

7. Dosierungsform zur kontrollierten Freisetzung nach Anspruch 1, 2 oder 3, wobei die bezüglich der Löslichkeit verstärkte Form, wenn sie allein an eine Verwendungsumgebung verabreicht wird, bereitstellt:
a) eine maximale Wirkstoffkonzentration, die das wenigstens 2-Fache derjenigen ist, die durch eine Kontrollzusammensetzung bereitgestellt wird, die aus einer äquivalenten Menge an CETPI in kristalliner Form allein besteht, oder
b) eine Konzentration-versus-Zeit-Fläche unter der Kurve (AUC) für einen Zeitraum von wenigstens 90 Minuten zwischen der Einführungszeit in die Verwendungsumgebung und etwa 270 Minuten nach Einführung in die Verwendungsumgebung, die das wenigstens etwa 1,25-Fache derjenigen einer Kontrollzusammensetzung ist, die aus einer äquivalenten Menge von nur CETPI in kristalliner Form besteht.

8. Dosierungsform zur kontrollierten Freisetzung nach Anspruch 1, 2 oder 3, wobei die Dosierungsform aus der Gruppe, bestehend aus einer Tablette, einer Kapsel und einem Multiparticulate, ausgewählt ist.

9. Dosierungsform zur kontrollierten Freisetzung nach Anspruch 1, 2 oder 3, wobei die Dosierungsform, wenn sie an einen Menschen verabreicht wird, wenigstens eines der folgenden aufweist:
a) eine Freisetzungsrate in den Gastrointestinaltrakt des Menschens, die in einer wenigstens etwa 50%-igen Inhibierung von Plasma-CETPI in dem Menschen für einen Zeitraum von wenigstens etwa 12 Stunden ab der Verabreichungszeit resultiert, oder
b) 50% Inhibierung bei einer Dosis, die niedriger ist als eine Dosis mit unverzüglicher Freisetzung, die in einer äquivalenten Inhibierung resultiert.

10. Dosierungsform zur kontrollierten Freisetzung nach Anspruch 1, 2 oder 3, wobei die Dosierungsform, wenn sie an einen Menschen verabreicht wird, einen Tₘₐₓ in Blut bereitstellt, der wenigstens das 1,25-Fache desjenigen ist, der durch eine Dosierungsform mit unverzüglicher Freisetzung, bestehend aus einer äquivalenten Menge des CETPI in derselben bezüglich der Löslichkeit verstärkten Form, bereitgestellt wird.

11. Dosierungsform zur kontrollierten Freisetzung nach Anspruch 1, 2 oder 3, wobei die Dosierungsform, wenn sie an einen Menschen verabreicht wird, einen Cₘₐₓ-Wert im Blut bereitstellt, der kleiner als oder gleich 80% desjenigen ist, der durch eine Dosierungsform zur unverzüglichen Freisetzung, bestehend aus einer äquivalenten Menge des CETPI in derselben bezüglich der Löslichkeit verstärkten Form, bereitgestellt wird.

12. Dosierungsform zur kontrollierten Freisetzung nach Anspruch 1, 2 oder 3, wobei die Dosierungsform, wenn sie an einen Menschen verabreicht wird, eine relative Bioverfügbarkeit des wenigstens 1,25-Fachen verglichen mit einer Dosierungsform zur unverzüglichen Freisetzung, bestehend aus einer äquivalenten Menge des CETPI in derselben bezüglich der Löslichkeit verstärkten Form, bereitstellt.

13. Dosierungsform zur kontrollierten Freisetzung nach Anspruch 1, 2 oder 3, wobei die Dosierungsform nach Verabreichung an eine In-intro-Verwendungsumgebung eine Freisetzungsrate von weniger als 40 Gew.-%/Stunde hat.

14. Dosierungsform zur kontrollierten Freisetzung nach Anspruch 1, 2 oder 3, wobei die Dosierungsform, wenn sie an einen Menschen verabreicht wird, eine Freisetzungsrate in den Gastrointestinaltrakt des Menschen hat, die in einer Wirkstoff-Plasmakonzentration beim Menschen von über etwa 70 ng/ml für einen Zeitraum von wenigstens etwa 12 Stunden ab Verabreichung resultiert.

## Revendications

1. Forme posologique à libération contrôlée, comprenant :
(a) une forme, à solubilité accrue, d'un inhibiteur de la protéine de transfert d'ester de cholestéryle (CETPI), ledit CETPI étant choisi dans le groupe consistant en les composés de formule XVI : 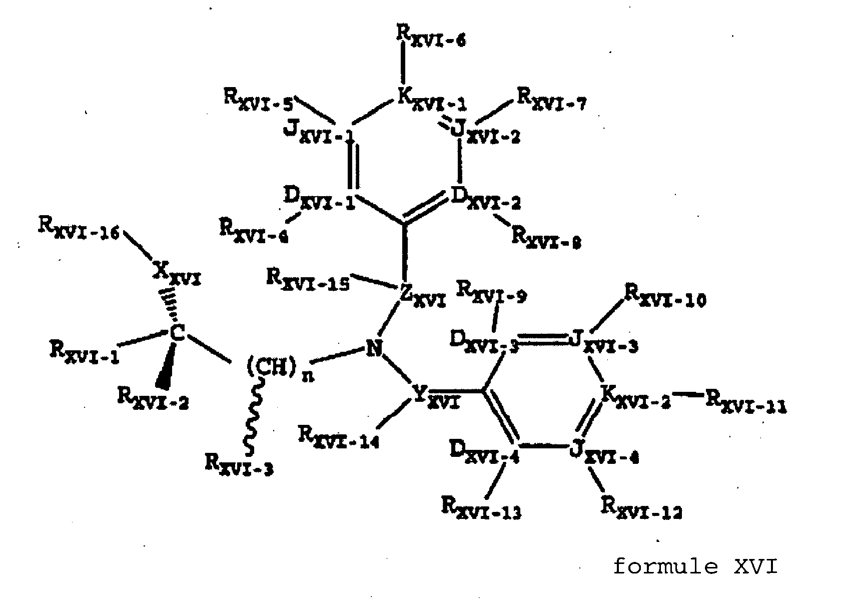 et leurs formes pharmaceutiquement acceptables, formule
dans laquelle :
n_{XVI} représente un nombre entier de 1 à 4 ;
X_{XVI} représente un groupe oxy ;
R_{XVI-1} est choisi dans le groupe consistant en des groupes halogénalkyle, halogénalcényle, halogénalkoxyméthyle et halogénalcényloxyméthyle, sous réserve que R_{XVI-1} ait un système stéréochimique de Cahn-Ingold-Prelog d'un rang supérieur à ceux de R_{XVI-2} et (CHR_{XVI-3})ₙ-N(A_{XVI})Q_{XVI} dans lequel A_{XVI} répond à la formule XVI-(II) et Q répond à la formule XVI-(III) : 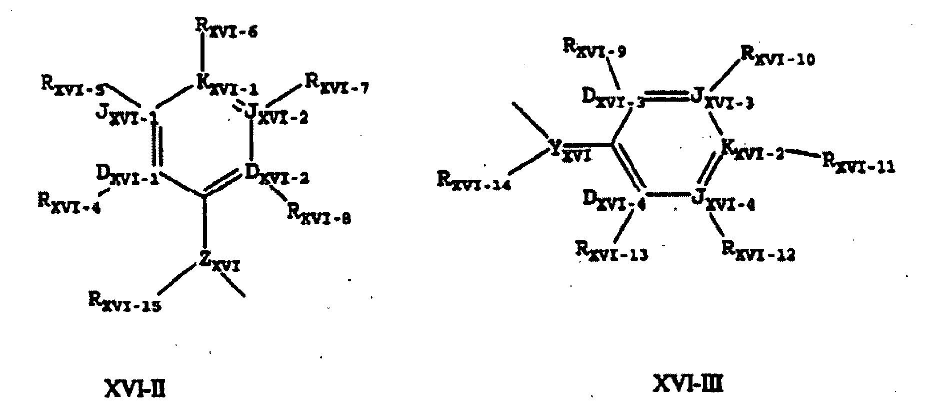
R_{XVI-16} est choisi dans le groupe consistant en des groupes hydrido, alkyle, acyle, aroyle, hétéroaroyle, trialkylsilyle, et un groupe intercalaire choisi dans le groupe consistant en une liaison simple covalente et un groupement intercalaire linéaire ayant une longueur de chaîne de 1 à 4 atomes lié au point de liaison de n'importe quel substituant aromatique choisi dans le groupe consistant en R_{XVI-4}, R_{XVI-8}, R_{XVI-9} et R_{XVI-13} pour former un noyau hétérocyclyle ayant 5 à 10 membres contigus ;
D_{XVI-1}, D_{XVI-2}, J_{XVI-1}, J_{XVI-2} et K_{XVI-1} sont choisis indépendamment dans le groupe consistant en C, N, O, S et une liaison covalente, sous réserve que pas plus d'un de D_{XVI-1}, D_{XVI-2}, J_{XVI-1}, J_{XVI-2} et K_{XVI-1} ne représente une liaison covalente, pas plus d'un de D_{XVI-1}, D_{XVI-2}, J_{XVI-1}, J_{XVI-2} et K_{XVI-1} ne représente 0, pas plus d'un de D_{XVI-1}, D_{XVI-2}, J_{XVI-1}, J_{XVI-2} et K_{XVI-1} ne représente S, un de D_{XVI-1}, D_{XVI-2}, J_{XVI-1}, J_{XVI-2} et K_{XVI-1} doit représenter une liaison covalente lorsque deux de D_{XVI-1}, D_{XVI-2}, J_{XVI-1}, J_{XVI-2} et K_{XVI-1} représentent O et S et pas plus de quatre de D_{XVI-1}, D_{XVI-2}, J_{XVI-1}, J_{XVI-2} et K_{XVI-1} ne représentent N ;
D_{XVI-3}, D_{XVI-4}, J_{XVI-3}, J_{XVI-4} et K_{XVI-2} sont choisis indépendamment dans le groupe consistant en C, N, 0, S et une liaison covalente, sous réserve que pas plus d'un ne représente une liaison covalente, pas plus d'un de D_{XVI-3}, D_{XVI-4}, J_{XVI-3}, J_{XVI-4} et K_{XVI-2} ne représente O, pas plus d'un de D_{XVI-3}, D_{XVI-4}, J_{XVI-3}, J_{XVI-4} et K_{XVI-2} ne représente S, pas plus de deux de D_{XVI-3}, D_{XVI-4}, J_{XVI-3}, J_{XVI-4} et K_{XVI-2} ne représentent O et S, un de D_{XVI-3}, D_{XVI-4}, J_{XVI-3}, J_{XVI-4} et K_{XVI-2} doit représenter une liaison covalente lorsque deux de D_{XVI-3}, D_{XVI-4}, J_{XVI-3}, J_{XVI-4} et K_{XVI-2} représentent 0 et S, et pas plus de quatre de D_{XVI-3}, D_{XVI-4}, J_{XVI-3}, J_{XVI-4} et K_{XVI-2} ne représentent N ;
R_{XVI-2} est choisi dans le groupe consistant en des groupes hydrido, aryle, aralkyle, alkyle, alcényle, alcényloxyalkyle, halogénalkyle, halogénalcényle, halogénocycloalkyle, halogénalkoxy, halogénalkoxyalkyle, halogénalcényloxyalkyle, halogénocycloalkoxy, halogénocycloalkoxyalkyle, perhalogénaryle, perhalogénaralkyle, perhalogénaryloxyalkyle, hétéroaryle, dicyanalkyle et carboalkoxycyanalkyle, sous réserve que R_{XVI-2} ait un système de Cahn-Ingold-Prelog de rang inférieur à ceux de R_{XVI-1} et (CHR_{XVI-3})ₙ-N(A_{XVI})Q_{XVI} ;
R_{XVI-3} est choisi dans le groupe consistant en des groupes hydrido, hydroxy, cyano, aryle, aralkyle, acyle, alkoxy, alkyle, alcényle, alkoxyalkyle, hétéroaryle, alcényloxyalkyle, halogénalkyle, halogénalcényle, halogénalkoxy, halogénalkoxyalkyle, halogénalcényloxyalkyle, monocyanalkyle, dicyanalkyle, carboxamide et carboxamidoalkyle, sous réserve que (CHR_{XVI-3})ₙ-N(A_{XVI})Q_{XVI} ait un système stéréochimique de Cahn-Ingold-Prelog de rang inférieur à celui de R_{XVI-1} et un système stéréochimique de Cahn-Ingold-Prelog de rang supérieur à celui de R_{XVI-2} ;
Y_{XVI} est choisi dans un groupe consistant en une liaison simple covalente, un groupe (C(R_{XVI-14})₂)_{q} dans lequel q représente un nombre entier choisi entre 1 et 2 et un groupe (CH(R_{XVI-14}))_{g}-W_{XVI}-(CH(R_{XVI-14}))ₚ dans lequel g et p représentent des nombres entiers choisis indépendamment entre 0 et 1 ;
R_{XVI-14} est choisi dans le groupe consistant en des groupes hydrido, hydroxy, cyano, hydroxyalkyle, acyle, alkoxy, alkyle, alcényle, alcynyle, alkoxyalkyle, halogénalkyle, halogénalcényle, halogénalkoxy, halogénalkoxyalkyle, halogénalcényloxyalkyle, monocarboalkoxyalkyle, monocyanalkyle, dicyanalkyle, carboalkoxycyanalkyle, carboalkoxy, carboxamide et carboxamidoalkyle ;
Z_{XVI} est choisi dans un groupe consistant en une liaison simple covalente, un groupe (C(R_{XVI-15})₂)_{q}, dans lequel q représente un nombre entier choisi entre 1 et 2 et un groupe (CH(R_{XVI-15}))ⱼ-W_{XVI}-(CH(R_{XVI-15}))ₖ dans lequel j et k représentent des nombres entiers choisis indépendamment entre 0 et 1 ;
W_{XVI} est choisi dans le groupe consistant en O, C (0), C(S), C(O)N(R_{XVI-14}), C(S)N(R_{XVI-14}), (R_{XVI-14})NC(O), (R_{XVI-14})NC(S), S, S(O), S(O)₂, S(O)₂N(R_{XVI-14}), (R_{XVI-14})NS(O)₂ et N(R_{XVI-14}), sous réserve que R_{XVI-14} soit autre qu'un groupe cyano ; R_{XVI-15} est choisi dans le groupe consistant en des groupes hydrido, cyano, hydroxyalkyle, acyle, alkoxy, alkyle, alcényle, alcynyle, alkoxyalkyle, halogénalkyle, halogénalcényle, halogénalkoxy, halogénalkoxyalkyle, halogénalcényloxyalkyle, mocarboalkoxyalkyle, monocyanalkyle, dicyanalkyle, carboalkoxycyanalkyle, carboalkoxy, carboxamide et carboxamidoalkyle ;
R_{XVI-4}, R_{XVI-5}, R_{XVI-6}, R_{XVI-7}, R_{XVI-8}, R_{XVI-9}, R_{XVI-10}, R_{XVI-11}, R_{XVI-12} et R_{XVI-13} sont choisis indépendamment dans le groupe consistant en des groupes hydrido, carboxy, hétéroaralkylthio, hétéroaralkoxy, cycloalkylamino, acylalkyle, acylalkoxy, aroylalkoxy, hétérocyclyloxy, aralkylaryle, aralkyle, aralcényle, aralcynyle, hétérocyclyle, perhalogénaralkyle, aralkylsulfonyle, aralkylsulfonylalkyle, aralkylsulfinyle, aralkylsulfinylalkyle, halogénocycloalkyle, halogénocycloalcényle, cycloalkylsulfinyle, cycloalkylsulfinylalkyle, cycloalkylsulfonyle, cycloalkylsulfonylalkyle, hétéroarylamino, N-hétéroarylamino-N-alkylamino, hétéroaralkyle, hétéroarylaminoalkyle, halogénalkylthio, alcanoyloxy, alkoxy, alkoxyalkyle, halogénalkoxyalkyle, hétéroaralkoxy, cycloalkoxy, cycloalcényloxy, cycloalkoxyalkyle, cycloalkylalkoxy, cycloalcényloxyalkyle, cycloalkylènedioxy, halogénocycloalkoxy, halogénocycloalkoxyalkyle, halogénocycloalcényloxy, halogénocycloalcényloxyalkyle, hydroxy, amino, thio, nitro, alkylamino inférieur, alkylthio, alkylthioalkyle, arylamino, aralkylamino, arylthio, arylthioalkyle, hétéroaralkoxyalkyle, alkylsulfinyle, alkylsulfinylalkyle, arylsulfinylalkyle, arylsulfonylalkyle, hétéroarylsulfinylalkyle, hétéroarylsulfonylalkyle, alkylsulfonyle, alkylsulfonylalkyle, halogénalkylsulfinylalkyle, halogénalkylsulfonylalkyle, alkylsulfonamido, alkylaminosulfonyle, amidosulfonyle, monoalkylamidosulfonyle, dialkyle, amidosulfonyle, monoarylamidosulfonyle, arylsulfonamido, diarylamidosulfonyle, monoalkylmonoarylamindosulfonyle, arylsulfinyle, arylsulfonyle, hétéroarylthio, hétéroarylsulfinyle, hétéroarylsulfonyle, hétérocyclylsulfonyle, hétérocyclylthio, alcanoyle, alcénoyle, aroyle, hétéroaroyle, aralcanoyle, hétéroaralcanoyle, halogénalcanoyle, alkyle, alcényle, alcynyle, alcényloxy, alcényloxyalkyle, alkylènedioxy, halogénalkylènedioxy, cycloalkyle, cycloalkylalcanoyle, cycloalcényle, cycloalkylalkyle inférieur, cycloalcénylalkyle inférieur, halogéno, halogénalkyle, halogénalcényle, halogénalkoxy, hydroxyhalogénalkyle, hydroxyaralkyle, hydroxyalkyle, hydroxyhétéroaralkyle, halogénalkoxyalkyle, aryle, hétéroaralcynyle, aryloxy, aralkoxy, aryloxyalkyle, hétérocyclyle saturé, hétérocyclyle partiellement saturé, hétéroaryle, hétéroaryloxy, hétéroaryloxyalkyle, arylalcényle, hétéroarylalcényle, carboxyalkyle, carboalkoxy, alkoxycarboxamido, alkylamidocarbonylamido, arylamidocarbonylamido, carboalkoxyalkyle, carboalkoxyalcényle, carboaralkoxy, carboxamido, carboxamidoalkyle, cyano, carbohalogénalkoxy, phosphono, phosphonoalkyle, diaralkoxyphosphono et diaralkoxyphosphonoalkyle, sous réserve que R_{XVI-4}, R_{XVI-5}, R_{XVI-6}, R_{XVI-7}, R_{XVI-8}, R_{XVI-9}, R_{XVI-10}, R_{XVI-11}, R_{XVI-12} et R_{XVI-13} soient choisis chacun indépendamment pour maintenir la nature tétravalente du carbone, la nature trivalente de l'azote, la nature divalente du soufre et la nature divalente de l'oxygène ;
R_{XVI-4} et R_{XVI-5}, R_{XVI-5} et R_{XVI-6}, R_{XVI-6} et R_{XVI-7}, R_{XVI-7} et R_{XVI-8}, R_{XVI-9} et R_{XVI-10}, R_{XVI-10} et R_{XVI-11}, R_{XVI-11} et R_{XVI-12} et R_{XVI-12} et A_{XIV-13} sont choisis indépendamment pour former des paires de groupes intercalaires, dans lesquelles une paire de groupes intercalaires est prise conjointement pour former un groupement linéaire ayant 3 à 6 atomes contigus connectant les points de liaison desdits membres de la paire de groupes intercalaires pour former un noyau choisi dans le groupe consistant en un noyau cycloalcényle ayant 5 à 8 membres contigus, un noyau hétérocyclyle partiellement saturé ayant 5 à 8 membres contigus, un noyau hétéroaryle ayant 5 ou 6 membres contigus et un groupe aryle, sous réserve que pas plus d'un du groupe consistant en les paires de groupes intercalaires R_{XVI-4} et R_{XVI-5}, R_{XVI-5} et R_{XVI-6}, R_{XVI-6} et R_{XVI-7} et R_{XVI-7} et R_{XVI-8}, ne soit utilisé en même temps et que pas plus d'un du groupe consistant en les paires de groupes intercalaires R_{XVI-9} et R_{XVI-10}, R_{XVI-10} et R_{XVI-11}, R_{XVI-11} et R_{XVI-12} et R_{XVI-12} et R_{XVI-13} ne puisse être utilisé en même temps ;
R_{XVI-4} et R_{XVI-9}, R_{XVI-4} et R_{XVI-13}, R_{XVI-8} et R_{XVI-9} et R_{XVI-8} et R_{XVI-13} sont choisis indépendamment pour former une paire de groupes intercalaires, ladite paire de groupes intercalaires étant prise conjointement pour former conjointement un groupement linéaire, ledit groupement linéaire formant un noyau choisi dans le groupe consistant en un noyau hétérocyclyle partiellement saturé ayant 5 à 8 membres contigus et un noyau hétéroaryle ayant 5 ou 6 membres contigus, sous réserve que pas plus d'un groupe consistant en les paires de groupes intercalaires R_{XVI-4} et R_{XVI-9}, R_{XVI-4} et R_{XVI-13}, R_{XVI-8} et R_{XVI-9} et R_{XVI-8} et R_{XVI-13} ne soit utilisé en même temps ; et
(b) un moyen de libération contrôlée pour délivrer ledit CETPI ;
dans laquelle, après administration à un environnement d'utilisation *in vivo*, ladite forme posologique à libération contrôlée provoque au moins les résultats suivants :
(i) une inhibition d'au moins 50 % de la protéine de transfert d'ester de cholestéryle plasmatique pendant au moins 12 heures ;
(ii) une concentration maximale en médicament dans le sang qui est inférieure ou égale à 80 % de la concentration maximale en médicament dans le sang provoquée par une forme posologique à libération immédiate consistant en la même quantité de la forme à solubilité accrue dudit CETPI :
(iii) un taux moyen de cholestérol-HDL après administration pendant 8 semaines qui est d'au moins environ 1,2 fois celui obtenu avant administration ; et
(iv) un taux moyen de cholestérol-LDL après administration pendant 8 semaines qui est inférieur ou égal à 90 % de celui obtenu avant administration.

2. Forme posologique à libération contrôlée, comprenant :
(a) une forme à solubilité accrue d'un inhibiteur de protéine de transfert d'ester de cholestéryle (CETPI), ledit CETPI étant choisi dans le groupe consistant en les composés de formule XVI répondant à la définition figurant dans la revendication 1 ; et
(b) un moyen de libération contrôlée pour délivrer ledit CETPI ;
dans laquelle, après administration à un environnement d'utilisation, ladite forme posologique à libération contrôlée libère au moins 80 % en poids dudit CETPI en une période de temps supérieure à environ 2 heures.

3. Forme posologique à libération contrôlée suivant la revendication 1 ou 2, dans laquelle ledit CETPI est le (2R)-3-[[3-(4-chloro-3-éthylphénoxy)phényl][[3-(1,1,2,2-tétra-fluoréthoxy)phényl]-méthyl]amino]-1,1,1-trifluoro-2-propanol.

4. Forme posologique à libération contrôlée suivant la revendication 1, 2 ou 3, comprenant en outre un polymère augmentant la concentration.

5. Forme posologique à libération contrôlée suivant la revendication 4, dans laquelle ladite forme à solubilité accrue est une dispersion amorphe solide dudit CETPI dans ledit polymère augmentant la concentration.

6. Forme posologique à libération contrôlée suivant la revendication 1, 2 ou 3, comprenant en outre un moyen pour éviter la précipitation dudit CETPI dans ledit environnement d'utilisation.

7. Forme posologique à libération contrôlée suivant la revendication 1, 2 ou 3, dans laquelle ladite forme à solubilité accrue provoque, lorsqu'elle est administrée seule à un environnement d'utilisation :
a) une concentration maximale en médicament qui est d'au moins 2 fois celle provoquée par une composition témoin consistant en une quantité équivalente de CETPI sous forme cristalline seule ; ou
b) une aire sous la courbe (AUC) de concentration en fonction du temps, pendant n'importe quelle période d'au moins 90 minutes entre le temps d'introduction dans l'environnement d'utilisation et environ 270 minutes après l'introduction dans l'environnement d'utilisation, qui est égale à au moins environ 1,25 fois celle d'une composition témoin consistant en une quantité équivalente de CETPI sous forme cristalline seule.

8. Forme posologique à libération contrôlée suivant la revendication 1, 2 ou 3, ladite forme posologique étant choisie dans le groupe consistant en un comprimé, une capsule et une forme multiparticules.

9. Forme posologique à libération contrôlée suivant la revendication 1, 2 ou 3, ladite forme posologique, lors de son administration à un être humain, présente au moins une des caractéristiques suivantes :
a) une vitesse de libération dans le tractus gastrointestinal dudit être humain qui entraîne une inhibition d'au moins environ 50 % de la CETP plasmatique chez ledit être humain pendant une période de temps d'au moins environ 12 heures à partir du moment de l'administration ; ou
b) une inhibition de 50 % à une dose qui est inférieure à une dose à libération immédiate qui entraîne une inhibition équivalente.

10. Forme posologique à libération contrôlée suivant la revendication 1, 2 ou 3, ladite forme posologique, lors de son administration à un être humain, engendre une Tₘₐₓ dans le sang qui est d'au moins 1,25 fois celle engendrée par une forme posologique à libération immédiate consistant en une quantité équivalente dudit CETPI dans la même forme à solubilité accrue.

11. Forme posologique à libération contrôlée suivant la revendication 1, 2 ou 3, ladite forme posologique, lors de son administration à un être humain, engendre une Cₘₐₓ dans le sang qui est inférieure ou égale à 80 % de celle engendrée par une forme posologique à libération immédiate consistant en une quantité équivalente dudit CETPI dans la même forme à solubilité accrue.

12. Forme posologique à libération contrôlée suivant la revendication 1, 2 ou 3, ladite forme posologique, lors de son administration à un être humain, présente une biodisponibilité relative d'au moins 1,25 par rapport à une forme posologique à libération immédiate consistant en une quantité équivalente dudit CETPI dans la même forme à solubilité accrue.

13. Forme posologique à libération contrôlée suivant la revendication 1, 2 ou 3, ladite forme posologique, après administration à un environnement d'utilisation *in vitro,* a une vitesse de libération inférieure à 40 % en poids/heure.

14. Forme posologique à libération contrôlée suivant la revendication 1, 2 ou 3, ladite forme posologique, lorsqu'elle est administrée à un être humain, a une vitesse de libération dans le tractus gastro-intestinal dudit être humain qui entraîne une concentration plasmatique en médicament chez ledit être humain supérieure à environ 70 ng/ml pendant une période de temps d'au moins environ 12 heures à partir du moment de l'administration.
